# EUROPEAN PATENT APPLICATION

(11) **EP 4 691 475 A1**
(43) Date of publication of application: **11.02.2026**
(21) Application number: 23929534.8
(22) Date of filing: 31.03.2023
(51) Int. Cl.: A61K 31/7088, C12N 15/113, A61P 25/16, C12N 15/11

(54) **SEQUENCE FOR FORMING EDITING SUBSTRATE TOGETHER WITH RNA EDITING TARGET SITE**

(71) Applicant: Recorna (Guangzhou) Biotechnology Co., Ltd., Guangzhou, Guangdong 510320 (CN)
(72) Inventor: YANG, Wenbing, Guangzhou, Guangdong 510320 (CN); ZHANG, Rui, Guangzhou, Guangdong 510320 (CN)
(74) Representative: J A Kemp LLP
(86) International application number: PCT/CN2023/085802
(87) International publication number: WO 2024/197951

(57) **Abstract**

A sequence for forming an editing substrate together with a target RNA editing site. The sequence and the upstream and downstream portions of the target RNA editing site form a specific double-chain secondary structure, so that efficient editing of the target RNA editing site is realized.

## Description

### FIELD

The present disclosure belongs to the field of biomedicine, and relates to a sequence for forming an editing substrate together with a target RNA editing site.

### BACKGROUND

As one of the most important technologies in life sciences in recent years, gene editing has unlimited potential and advantages in treatment of diseases, especially genetic diseases, various chronic diseases and tumors. It is foreseeable that the next 5 to 10 years will be the golden age of rapid development of the gene editing technology gradually pushed into the clinic. The traditional treatment of genetic diseases and rare diseases, as well as the treatment concepts of some chronic diseases, geriatric diseases and tumors that threaten human health, will be completely subverted. Medical treatment based on gene editing will really help countless patients in the near future.

Gene editing can be divided into DNA editing and RNA editing according to different targets. Compared with DNA editing, RNA editing is intended to repair mutations at the RNA level without changing the genome sequence, is reversible and controllable, has safer characteristics, and has huge advantages in certain disease scenarios.

Currently, RNA editing specifically refers to single-base editing technology. Its principle is to use deaminase to change specific bases by deaminating ribonucleic acid. There are currently two main technical paths for targeted RNA editing. A first technique is a dual-molecule editing system (containing one exogenously expressed deaminase and one guide RNA).

A second technique is a single-molecule editing system, which delivers only a sequence that can recruit both endogenous deaminase and target editing sites. The second technique is currently successful only in recruiting ADAR for A-to-G targeted RNA editing. Compared to the exogenous ADAR expression system, the system that recruits endogenous ADAR only needs to deliver a fragment of antisense strand RNA to recruit endogenous ADAR to edit a specific RNA sequence site. This antisense strand RNA-based technique has a series of advantages such as fast drug preparation speed, security, and relatively mature delivery technique, and also has a series of advantages in clinical transformation and drug preparation. For example, this technology will not induce immune response, and can be applied to a plurality of developmental stages and a plurality of tissue types. It is easy to achieve tissue-specific gene editing and can achieve different levels of editing of a plurality of genes. Therefore, it has incomparable advantages over DNA editing technology in terms of security.

In the second technique, there are currently two methods for designing a guide RNA. A first guide RNA is composed of two segments. One segment is a self-generated ADAR substrate sequence with secondary structure characteristics that can recruit ADAR, and the other segment is a customizable targeting sequence for targeting a specific site for editing, where the targeting sequence and the target site form a completely complementary double-stranded RNA structure, except that the target site is set to A: C mismatch for facilitating editing of the target site (RESTORE method). Moreover, there is an optimized version of this method to reduce off-target editing (CLUSTER method). The guide RNA designed by the second method contain no recruitment sequence, but is a targeting sequence with a length of about 100 nt to 150 nt that is only complementary to the target sequence (LEAPER method). In the two methods, the regions complementary to the target sites are almost completely paired dsRNA double-stranded structures. Some studies have shown that almost completely paired dsRNA double-stranded structures are not the best ADAR editing substrates, so the two design methods exhibit low editing efficiency in vivo.

There are three levels of descriptions of the molecular structure of an RNA, including a primary sequence, a secondary structure, and a tertiary spatial structure. Although the tertiary spatial structure of the RNA is a stable structure formed in space, it is produced by the interaction between, or deformation, folding, or the like of secondary structural units. Furthermore, the tertiary structure of the RNA is difficult to obtain without the secondary structure of the RNA. Moreover, the prediction of the secondary structure of the RNA needs to consider not only the sequence arrangement, but also stable pairing modes, including pseudoknots and hairpins. The secondary structure of the RNA has two important roles. First, it can help explain the function of the RNA. The function of RNA is often related to the structure of the RNA. The secondary structure is the most crucial of all structures (the primary structure, the secondary structure, and the tertiary structure) of the RNA. Once the RNA is formed, it will undergo changes to form a specific tertiary structure. The formation of the tertiary structure depends on the matching between base pairs in the secondary structure. Second, the understanding of the secondary structure can also be used to explore a new function of the RNA.

Therefore, finding the secondary structure of a suitable editing substrate in suitable RNA editing is of great significance for improving the RNA editing efficiency.

### SUMMARY

In one aspect, the present application provides a sequence for forming an editing substrate together with a target RNA editing site, where the sequence, in the form of a complementary strand, forms a double-stranded secondary structure with upstream and downstream portions of the target RNA editing site, where a structure of the complementary strand includes at least one of sets of features indicated by i to x:
i. 0 to 4 mismatches, 0 to 7 wobble base pairs, 0 to 3 internal loops, 0 to 5 bulges, and 0 to 12 deletions from -100 to -81 of the editing site;
ii. 0 to 5 mismatches, 0 to 7 wobble base pairs, 0 to 5 internal loops, 0 to 5 bulges, and 0 to 15 deletions from -80 to -61 of the editing site;
iii. 0 to 5 mismatches, 0 to 7 wobble base pairs, 0 to 3 internal loops, 0 to 5 bulges, and 0 to 18 deletions from -60 to -41 of the editing site;
iv. 0 to 4 mismatches, 0 to 7 wobble base pairs, 0 to 3 internal loops, 0 to 8 bulges, and 0 to 11 deletions from -40 to -21 of the editing site;
v. 0 to 5 mismatches, 0 to 6 wobble base pairs, 0 to 4 internal loops, 0 to 5 bulges, and 0 to 9 deletions from -20 to -1 of the editing site;
vi. 0 to 5 mismatches, 0 to 7 wobble base pairs, 0 to 3 internal loops, 0 to 3 bulges, and 0 to 5 deletions from 0 to 19 of the editing site;
vii. 0 to 4 mismatches, 0 to 7 wobble base pairs, 0 to 3 internal loops, 0 to 6 bulges, and 0 to 9 deletions from 20 to 39 of the editing site;
viii. 0 to 5 mismatches, 0 to 6 wobble base pairs, 0 to 3 internal loops, 0 to 5 bulges, and 0 to 17 deletions from 40 to 59 of the editing site;
ix. 0 to 5 mismatches, 0 to 8 wobble base pairs, 0 to 4 internal loops, 0 to 5 bulges, and 0 to 19 deletions from 60 to 79 of the editing site; and
x. 0 to 4 mismatches, 0 to 6 wobble base pairs, 0 to 3 internal loops, 0 to 5 bulges, and 0 to 19 deletions from 80 to 100 of the editing site. In some embodiments, a mismatch type on the complementary strand is one of A-A, A-G, A-C, U-U, U-C, G-G, G-A, C-A, C-C, and C-U.

In some embodiments, the wobble base pair on the complementary strand is a G-U pair.

In some embodiments, the bulge on the complementary strand refers to that one base upstream of and one base downstream of a bulge region are both complementarily paired with a target editing strand and corresponding two bases of the target editing strand are consecutive. In some embodiments, the deletion on the complementary strand is that bases upstream and downstream of a deletion region are consecutive, and the target editing strand in the deletion region has a corresponding number of bases. In some embodiments, the internal loop on the complementary strand refers to that the target editing strand is complementarily paired with bases of the complementary strand, one upstream of and one downstream of the internal loop, and the internal loop has a corresponding number of bases mismatched.

In some embodiments, the double-stranded secondary structure is one selected from structures Struc1 to Struc1196. In some embodiments, structural features of the double-stranded secondary structure are as shown in Table 1.

In some embodiments, the structural features of the double-stranded secondary structure include one or more of:
a mismatch formed between bases, denoted by M;
wobble base pairing, denoted by W;
an internal loop, denoted by Li;
a deletion, denoted by D;
a bulge, denoted by Ii; and
Watson-Crick pairing, denoted by P.

In some embodiments, the double-stranded secondary structure has a feature denoted by X, X = [n1, n2, n3 ...], with n1, n2, ... indicating positions of a structure X, where the editing site is at 0, -n indicates that the position is an n-th base upstream of the editing site, and n indicates that the position is an n-th base downstream of the editing site.

In some embodiments, when the double-stranded secondary structure X is M, M = [n1, n2, n3 ...] indicates that mismatches are present at positions of n1, n2, n3 ... from the editing site.

When the double-stranded secondary structure X is W, W = [n1, n2, n3 ...] indicates that wobble base pairs are present at positions of n1, n2, n3 ... from the editing site.

When the double-stranded secondary structure X is D, D = [n1, n2, n3...] indicates that deletions are present at positions of n1, n2, n3 ... from the editing site.

When X is Ii, Ii = [n1, n2], with i being any positive integer indicating that i bases are inserted between n1 and n2 from the editing site.

When the double-stranded secondary structure X is Li, Li = [n1 to n2, n3 to n4 ...], with i being any positive integer indicating that internal loops having a size of i nt are formed in position ranges of n1 to n2, n3 to n4 ... from the editing site.

In any double-stranded secondary structure, all positions are P except where X is one or more of M, W, D, Ii, and Li.

In some embodiments, a motif of the target RNA editing site, and one base upstream of and one base downstream of the RNA editing site is NAN. In some embodiments, the NAN is UAN, AAN, CAN, or GAN. In some embodiments, the UAN is UAG, UAU, UAC, or UAA. In some embodiments, the AAN is AAU, AAC, AAG, or AAA. In some embodiments, the CAN is CAU, CAG, CAC, or CAA. In some embodiments, the GAN is GAU, GAC, GAA, or GAG.

In some embodiments, when the motif of the target RNA editing site, and one base upstream of and one base downstream of the RNA editing site is NAN, the double-stranded secondary structure is selected from Struc1 to Struc1196

In some embodiments, when the motif of the target RNA editing site, and one base upstream of and one base downstream of the RNA editing site is UAA, the double-stranded secondary structure is selected from Struc1 to Struc143.

In some embodiments, when the motif of the target RNA editing site, and one base upstream of and one base downstream of the RNA editing site is UAG, the double-stranded secondary structure is selected from Struc144 to Struc260.

In some embodiments, when the motif of the target RNA editing site, and one base upstream of and one base downstream of the RNA editing site is UAU, the double-stranded secondary structure is selected from Struc261 to Struc320.

In some embodiments, when the motif of the target RNA editing site, and one base upstream of and one base downstream of the RNA editing site is UAC, the double-stranded secondary structure is selected from Struc321 to Struc395.

In some embodiments, when the motif of the target RNA editing site, and one base upstream of and one base downstream of the RNA editing site is CAA, the double-stranded secondary structure is selected from Struc396 to Struc515.

In some embodiments, when the motif of the target RNA editing site, and one base upstream of and one base downstream of the RNA editing site is CAC, the double-stranded secondary structure is selected from Struc516 to Struc541.

In some embodiments, when the motif of the target RNA editing site, and one base upstream of and one base downstream of the RNA editing site is AAG, the double-stranded secondary structure is selected from Struc542 to Struc684.

In some embodiments, when the motif of the target RNA editing site, and one base upstream of and one base downstream of the RNA editing site is AAC, the double-stranded secondary structure is selected from Struc685 to Struc767.

In some embodiments, when the motif of the target RNA editing site, and one base upstream of and one base downstream of the RNA editing site is GAA, the double-stranded secondary structure is selected from Struc768 to Struc809.

In some embodiments, when the motif of the target RNA editing site, and one base upstream of and one base downstream of the RNA editing site is AAG, the double-stranded secondary structure is selected from Struc810 to Struc875.

In some embodiments, when the motif of the target RNA editing site, and one base upstream of and one base downstream of the RNA editing site is AAU, the double-stranded secondary structure is selected from Struc876 to Struc982.

In some embodiments, when the motif of the target RNA editing site, and one base upstream of and one base downstream of the RNA editing site is CAG, the double-stranded secondary structure is selected from Struc983 to Struc1085.

In some embodiments, when the motif of the target RNA editing site, and one base upstream of and one base downstream of the RNA editing site is CAU, the double-stranded secondary structure is selected from Struc1086 to Struc1196.

In some embodiments, the complementary strand is complementarily base-paired at a non-mismatch or non-deletion or non-bulge or non-internal loop or non-wobble-base-pairing site.

In some embodiments, the sequence forming an editing substrate together with a target RNA editing site is 25 bp to 300 bp in length. In some embodiments, the sequence is 25 bp to 151 bp in length.

In some embodiments, the RNA is an RNA forming a specific secondary structure together with a target RNA to be edited.

In some embodiments, the target RNA to be edited is a messenger RNA (mRNA).

In some embodiments, the small RNA is miRNA, pri-miRNA, pre-miRNA, piRNA, siRNA, snoRNA, snRNA, exRNA, or scaRNA.

In one aspect, the present application provides an mcRNA, including a mimic domain, where a sequence of the mimic domain is the sequence for forming an editing substrate together with a target RNA editing site, and forms the double-stranded secondary structure together with a target RNA to be edited.

In some embodiments, the mcRNA further includes binding domains located at both ends of the mimic domain and complementarily paired with regions other than the double-stranded secondary structure of the target RNA to be edited.

In some embodiments, the binding domain is 0 to 55 bp in length on a single side.

In some embodiments, a sequence of the mcRNA is any one selected from sequences of SEQ ID NOs.: 1 to 16083.

In one aspect, the present application provides a design method for the mcRNA, including the following steps:
S1, obtaining a reference sequence: using a strand of a reference secondary structure where an editing site is located is an editing strand of the reference secondary structure and the other strand as a complementary strand of the reference secondary structure, and obtaining nucleotide sequences of both strands, respectively;
S2, obtaining a pre-mcRNA sequence of a target editing site: extending a preset length upstream and downstream from a site to be edited as a starting point to obtain a template sequence of a mimic domain, namely a target editing strand; and designing a sequence complementarily paired with the template sequence as the pre-mcRNA sequence;
S3, designing an mcRNA mimic domain: aligning the sequence of the target editing strand to a sequence of the editing strand of the reference secondary structure by extending from the editing site to both ends; aligning the pre-mcRNA sequence to a sequence of the complementary strand of the reference secondary structure; when an alignment result of the sequence of the target editing strand and the sequence of the editing strand of the reference secondary structure indicates consistency, setting a corresponding position of the mcRNA mimic domain to the corresponding sequence of the complementary strand of the reference secondary structure; and when the alignment result of the sequence of the target editing strand and the sequence of the editing strand of the reference secondary structure indicates inconsistency, setting and designing the corresponding position of the mcRNA mimic domain to a sequence forming a same structure as the corresponding reference secondary structure together with the target editing site; and
S4, designing mcRNA binding domains: on the basis of the mcRNA mimic domain designed in step S3, extending complementarily paired sequences a preset length from both ends of the mimic domain to form the binding domains.

In the present disclosure, a design method, i.e., mimic ADAR substrates structure approach (MASSA), is provided by simulating the structural features of the natural double-stranded RNA substrate with a high editing level in the human body, and an mcRNA with certain structural characteristics is formed with upstream and downstream sequences of a target site, thereby achieving an efficient target RNA editing effect.

In one aspect, the present application provides use of the sequence forming an editing substrate together with a target RNA editing site or the mcRNA in preparation of an RNA editing reagent or composition.

In one aspect, the present application provides an RNA editing reagent or composition including the sequence forming an editing substrate together with a target RNA editing site or the mcRNA.

In one aspect, the present application provides a method of editing a target RNA in a host cell, where an oligonucleotide including the sequence forming an editing substrate together with a target RNA editing site or the mcRNA is introduced into the host cell.

In some embodiments, the target RNA is ATP7B gene, FGFR gene, TP53 gene, APC gene, SERPINA1 gene, MECP2 gene, or SCN1A gene.

In one aspect, the present application provides use of the sequence forming an editing substrate together with a target RNA editing site or the mcRNA in preparation of a medicament for treating a disease or disorder, where the disease or disorder is an inherited genetic disease, or a disease or disorder associated with one or more acquired gene mutations.

In some embodiments, the disease or disorder is a monogenic disease or disorder.

In some embodiments, the disease or disorder is a polygenic disease or disorder.

In some embodiments, the gene is ATP7B, FGFR, TP53, APC, SERPINA1, MECP2, or SCN1A.

In some embodiments, the disease or disorder is selected from the group consisting of hepatolenticular degeneration, rett syndrome, Dravet syndrome, cystic fibrosis, Hurler syndrome, alpha-1-antitrypsin (A1AT) deficiency, Parkinson's disease, Alzheimer's disease, albinism, amyotrophic lateral sclerosis, asthma, beta-thalassemia, Cadasil syndrome, peroneal muscular atrophy, chronic obstructive pulmonary disease (COPD), distal spinal muscular atrophy (DSMA), Duchenne/Becker muscular dystrophy, dystrophic epidermolysis bullosa, epidermolysis bullosa, Fabry disease, Leiden factor V related disease, familial adenoma, polyposis, galactosemia, Gaucher's disease, glucose-6-phosphate dehydrogenase, hemophilia, hereditary hemochromatosis, Hunter syndrome, Huntington's disease, inflammatory bowel disease (IBD), hereditary polyagglutination syndrome, Leber congenital amaurosis, Lesch-Nyhan syndrome, Lynch syndrome, marfan syndrome, mucopolysaccharidosis, muscular dystrophy, myotonic dystrophy type 1 and type 2, neurofibroma, Niemann-Pick diseases type A, type B and type C, NY-esol-associated cancers, Peutz-Jeghers syndrome, phenylketonuria, Pompe's disease, primary eyelash disease, and prothrombin mutation-associated diseases such as prothrombin G20210A mutation, pulmonary hypertension, retinitis pigmentosa, Sandhoff's disease, severe complicated immunodeficiency syndrome (SCID), sickle cell anemia, spinal muscular atrophy, Steger's disease, Tay-Sahr's disease, Usher's syndrome, X-linked immunodeficiency, and cancers.

In some embodiments, the mcRNA may contain one or more modifications. In some embodiments, the mcRNA has one or more modified nucleotides, including nucleobase modifications and/or backbone modifications. Exemplary modifications to an RNA include, but are not limited to, phosphorothioate backbone modifications, and ribose-modified LNA.

The present disclosure serves as a basic platform capable of targeted editing of RNAs, such as a pre-messenger RNA, a messenger RNA, a ribosomal RNA, a transfer RNA, a long non-coding RNA, and a small RNA (e.g., miRNA). The methods of the present application can employ a number of delivery systems including, but not limited to, viruses, liposomes, electroporation, microinjection, and conjugation to achieve introduction of the mcRNAs as described herein or a construct into a host cell. Conventional viral- and non-viral-based gene transfer methods can be used to introduce nucleic acids into mammalian cells or target tissues. Such methods can be used to administer nucleic acids encoding the sequences of the present application to cultured cells or host organisms. Non-viral vector delivery systems include DNA plasmids, RNAs (e.g., transcripts of the constructs described herein), naked nucleic acids, and nucleic acids complexed with delivery vectors, e.g., liposomes. The viral vector delivery systems include DNA and RNA viruses having episomal or integrated genomes for delivery to the host cells.

The terms "oligonucleotide", "nucleotide sequence", and "nucleic acid" are used interchangeably. They refer to polymeric forms of nucleotides, deoxyribonucleotides, or ribonucleotides of any length, or analogs thereof.

In the context of the present application, "target RNA" refers to that the sequence of the present application is designed to be an RNA sequence completely complementary or substantially complementary thereto, and the hybridization between the target sequence and the sequence of the present application results in an RNA region containing the double-stranded secondary structure of the target adenosine, which recruits an adenosine deaminase (ADAR) acting on the RNA, which enzyme deaminates the target adenosine. In some embodiments, the ADAR is naturally present in a host cell, such as a eukaryotic cell (in some embodiments a mammalian cell, and in some embodiments a human cell). In some embodiments, the ADAR is introduced into a host cell.

The term "including" encompasses "including" as well as "consisting of". For example, a composition "including X" may exclusively consist of X, or may include some additional components, e.g., X + Y. The term "about" with respect to the numerical value x is optional and, for example, refers to x ± 10%.

The expression "substantially" does not exclude "completely". For example, a composition that is "substantially free of Y" may be completely free of Y. When relevant, the expression "substantially" may be omitted from the definitions of the present disclosure.

The term "complementary" as used herein refers to the fact that an oligonucleotide is hybridized with a target sequence under physiological conditions. The term does not mean that each nucleotide in the oligonucleotide is perfectly paired with its opposite nucleotide in the target sequence. In other words, while the oligonucleotide may be complementary to the target sequence, there may be mismatches, wobbles and/or bulges between the oligonucleotide and the target sequence. Meanwhile, the oligonucleotide is still hybridized with the target sequence under physiological conditions, so that the cellular RNA editing enzyme can edit the target adenosine. Accordingly, the term "substantially complementary" also means that the oligonucleotide has sufficient matching nucleotides between the oligonucleotide and the target sequence under the physiological condition of hybridization of the oligonucleotide with the target RNA, despite the presence of mismatches, wobbles and/or bulges. As shown herein, the oligonucleotide may be complementary, but may also include one or more mismatches, wobbles and/or bulges relative to the target sequence, as long as the oligonucleotide can be hybridized with its target under physiological conditions.

Regarding the sequence of a nucleic acid, the term "downstream" refers to continuing in the 3' direction along the sequence, while the term "upstream" means the opposite. Thus, on any sequence encoding a polypeptide, the start codon is upstream of the stop codon in the positive-sense strand but downstream of the stop codon in the antisense strand.

Reference to "hybridization" generally refers to specific hybridization and excludes nonspecific hybridization. Specific hybridization can be performed using techniques well known in the art under selected experimental conditions to ensure that most stable interactions between the probe and the target are at least 70%, In some embodiments at least 80%, and more In some embodiments at least 90% sequence identity between the probe and the target.

The term "mismatch" as used herein refers to opposing nucleotides in a double-stranded RNA complex do not form perfect base pairs according to the Watson-Crick base pairing rule. The mismatched nucleotides are A-A, A-G, A-C, U-U, U-C, G-G, G-A, C-A, C-C, C-U pairs.

### BRIEF DESCRIPTION OF THE DRAWINGS

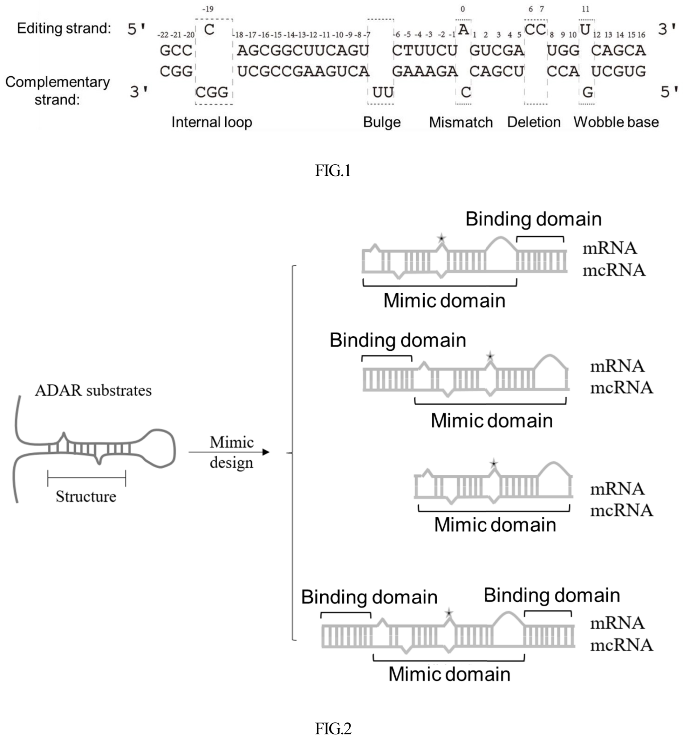

FIG. 1 illustrates an example of features of a secondary structure of a double-stranded RNA. FIG. 1 shows the example of the features of the secondary structure of a segment of double-stranded RNA, where an editing strand is a strand where an editing site is located, and a complementary strand is a strand where a sequence forms a specific structure together with the editing strand is located. In the figure, position 0 is the position of the A base of the editing site, positions-1 to -22 are the positions of the sequence upstream of the editing site, and positions 1 to 16 are the positions of the sequence downstream of the editing site. As shown in the figure, the structural features include a mismatch, a bulge, a deletion, a wobble base, and an internal loop, and other positions are all Watson-Crick base pairs.
FIG. 2 is a schematic diagram of the MASSA method of the present application. FIG. 2 shows the schematic diagram of the MASSA method used in the present application. The left side of the figure is a schematic diagram of the secondary structure of the ADAR substrate, and the right side shows the mcRNA designed by the MASSA method according to the structure on the left side. In the figure, the asterisk indicates the position of the editing site; the upstream and downstream regions with structural features are mimic domains, simulating the features of the secondary structure of the substrate; and there are binding domains at both ends, and the binding domains form Watson-Crick complementary base pairs together with the sequence of the target site.

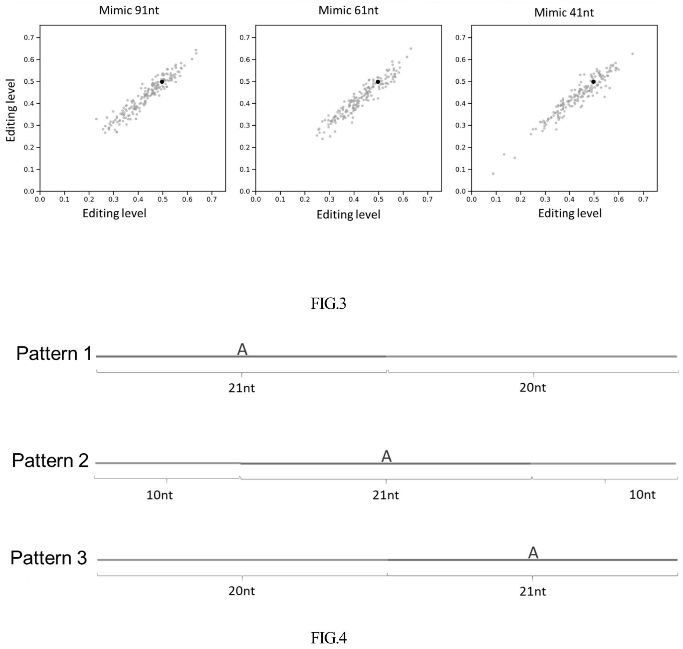

FIG. 3 illustrates editing levels of mcRNAs designed for a UAG motif of GAPDH gene in one embodiment of the present invention. FIG. 3 shows the results of the editing levels of the mcRNAs with combinations of mimic domains and binding domains of different lengths designed on the UAA motif of the GAPDH gene in one embodiment of the present disclosure. As shown in the figure, the lengths of the mimic domains are 91 nt, 61 nt, and 41 nt, respectively, and the lengths of the corresponding binding domains at both ends are 30 nt, 45 nt, and 55 nt, respectively. In the figure, the black dot represents the editing level of the mcRNA without structural feature and with only one mismatch design of A and C bases at the editing site, while other dots represent the editing levels of the mcRNAs with structural features, and the horizontal and vertical coordinates represent the editing levels of each mcRNA in two biological replicates.
FIG. 4 illustrates design patterns of mimic domains and binding domains of mcRNAs for different motifs of the GAPDH gene in one embodiment of the present disclosure. FIG. 4 shows the mcRNA design patterns on different motifs of the GAPDH gene in one embodiment of the present disclosure, mainly involving the positions and lengths of the mimic domains and the binding domains. As shown in the figure, the design mainly has three patterns. The first one is to extend, as the length of the mimic domain, 6 bases upstream and 14 bases downstream with the editing site as the center. The second one is to extend, as the length of the mimic domain, 10 bases upstream and 10 bases downstream with the editing site as the center. The third one is to extend, as the length of the mimic domain, 14 bases upstream and 6 bases downstream with the editing site as the center. In the first pattern, there is a binding domain of 20 bases downstream of the editing site. In the second pattern, there is a binding domain of 10 bases at each of two ends. In the third mode, there is a binding domain of 20 bases upstream of the editing site. The length of each reference design is 41 nt.

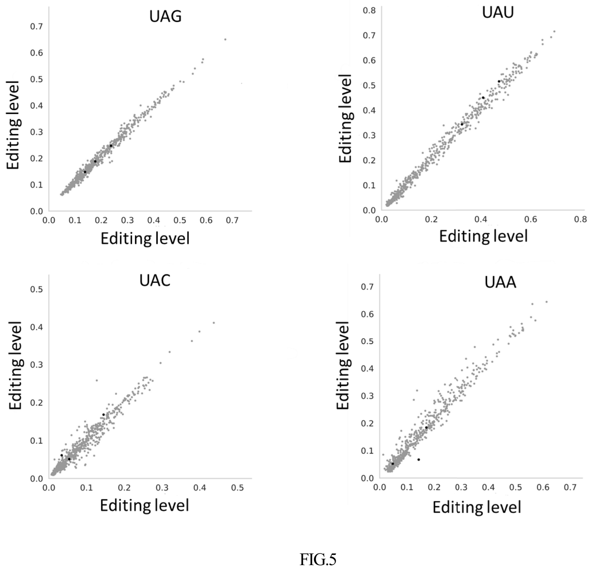

FIG. 5 illustrates editing levels of mcRNAs designed for a UAN motif of the GAPDH gene in one embodiment of the present disclosure. FIG. 5 shows a distribution of the editing levels of the mcRNAs designed for the UAN motif of the GAPDH gene in one embodiment of the present disclosure, where the included motifs are UAG, UAU, UAC, and UAA, respectively. The black dot represents the editing level of the mcRNA without structural feature and with only one mismatch design of A and C bases at the editing site, while other dots represent the editing levels of the mcRNAs with structural features designed in the present disclosure, and the horizontal and vertical coordinates represent the editing levels of each mcRNA in two biological replicates.

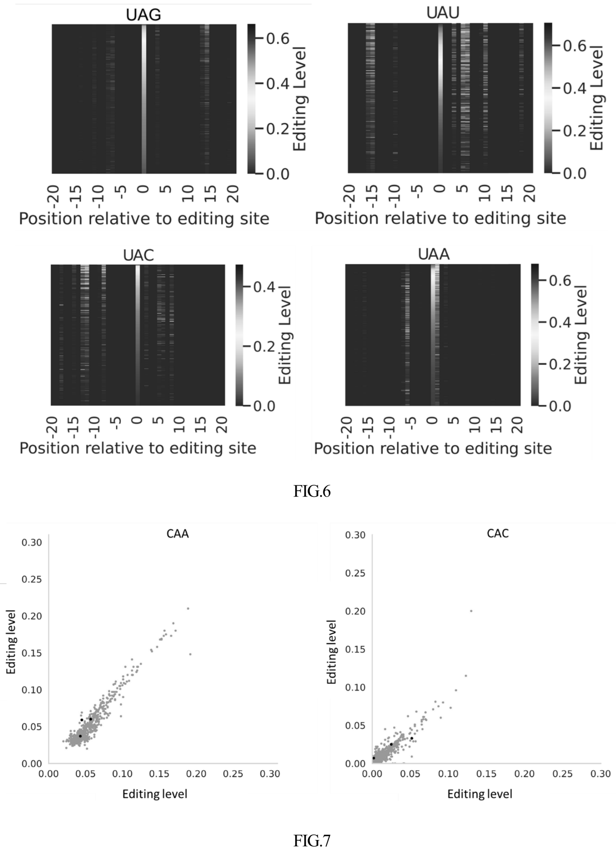

FIG. 6 illustrates target sites and off-target editing of mcRNAs designed for the UAN motif of the GAPDH gene in one embodiment of the present disclosure. FIG. 6 shows off-target editing heat maps of the mcRNAs designed for the UAN motif of the GAPDH gene at the target sites and in the upstream and downstream regions of the target sites in one embodiment of the present disclosure. The UAN motif includes UAG, UAU, UAC, and UAA. In the figure, the position 0 on the horizontal axis is the target site, -1 to -20 is a range upstream of the editing site, and 1 to 20 is a range downstream of the editing site. The vertical axis shows the editing levels of each mcRNA on different A bases. Arranged downward according to the highest editing sequences of the target sites, colors, blue to red, indicate the change trend of the editing levels of the A base sites from low to high.
FIG. 7 illustrates editing levels of mcRNAs designed for a CAN motif of the GAPDH gene in one embodiment of the present disclosure. FIG. 7 shows a distribution of the editing levels of the mcRNAs designed for the CAN motif of the GAPDH gene in one embodiment of the present disclosure, where the included motifs are CAA and CAC. The black dot represents the editing level of the mcRNA without structural feature and with only one mismatch design of A and C bases at the editing site, while other dots represent the editing levels of the mcRNAs with structural features designed in the present disclosure, and the horizontal and vertical coordinates represent the editing levels of each mcRNA in two biological replicates.

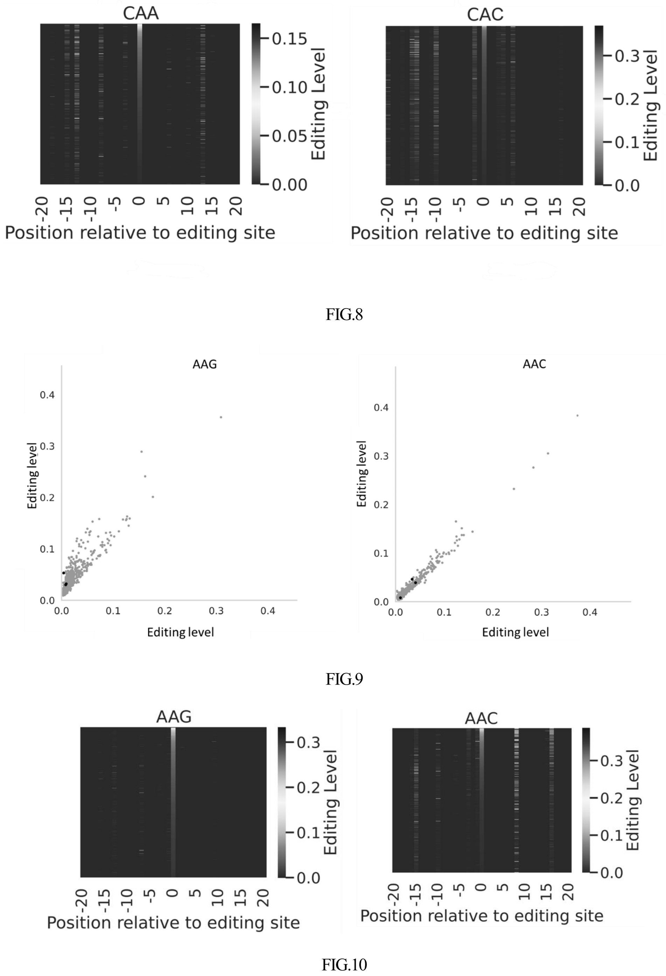

FIG. 8 illustrates target sites and off-target editing of mcRNAs designed for the CAN motif of the GAPDH gene in one embodiment of the present disclosure. FIG. 8 shows off-target editing heat maps of the mcRNAs designed for the CAN motif of the GAPDH gene at the target sites and in the upstream and downstream regions of the target sites in one embodiment of the present disclosure. The CAN motif includes CAA and CAC. In the figure, the position 0 on the horizontal axis is the target site, -1 to -20 is a range upstream of the editing site, and 1 to 20 is a range downstream of the editing site. The vertical axis shows the editing levels of each mcRNA on different A bases. Arranged downward according to the highest editing sequences of the target sites, colors, blue to red, indicate the change trend of the editing levels of the A base sites from low to high.
FIG. 9 illustrates editing levels of mcRNAs designed for an AAN motif of the GAPDH gene in one embodiment of the present disclosure. FIG. 9 shows a distribution of the editing levels of the mcRNAs designed for the AAN motif of the GAPDH gene in one embodiment of the present disclosure, where the included motifs are AAG and AAC. The black dot represents the editing level of the mcRNA without structural feature and with only one mismatch design of A and C bases at the editing site, while other dots represent the editing levels of the mcRNAs with structural features designed in the present disclosure, and the horizontal and vertical coordinates represent the editing levels of each mcRNA in two biological replicates.
FIG. 10 illustrates target sites and off-target editing of mcRNAs designed for the AAN motif of the GAPDH gene in one embodiment of the present disclosure. FIG. 10 shows off-target editing heat maps of the mcRNAs designed for the AAN motif of the GAPDH gene at the target sites and in the upstream and downstream regions of the target sites in one embodiment of the present disclosure. The AAN motif includes AAG and AAC. In the figure, the position 0 on the horizontal axis is the target site, -1 to -20 is a range upstream of the editing site, and 1 to 20 is a range downstream of the editing site. The vertical axis shows the editing levels of each mcRNA on different A bases. Arranged downward according to the highest editing sequences of the target sites, colors, blue to red, indicate the change trend of the editing levels of the A base sites from low to high.

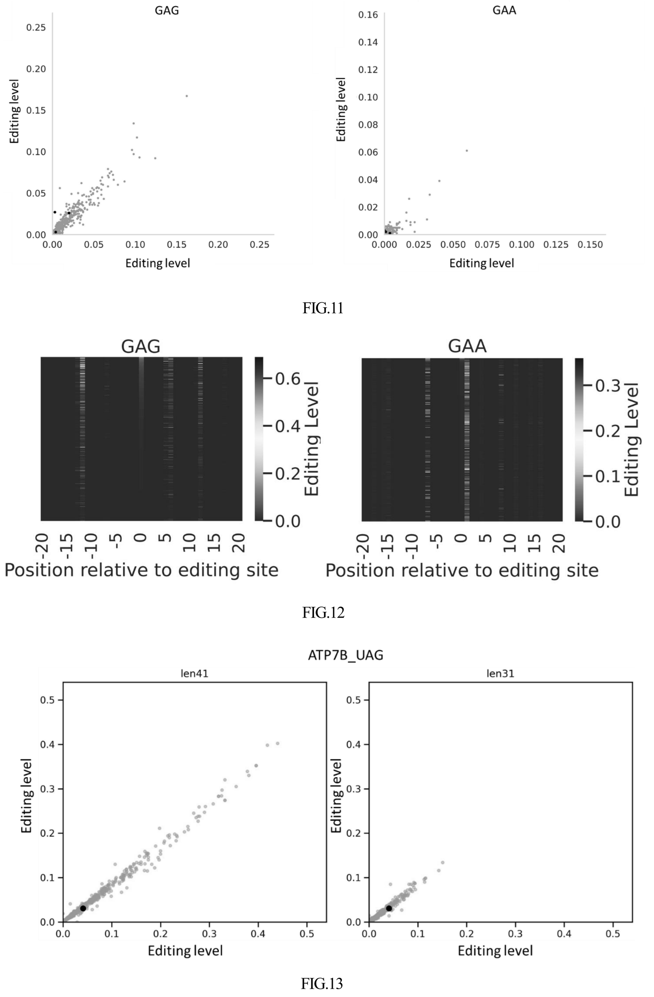

FIG. 11 illustrates editing levels of mcRNAs designed for a GAN motif of the GAPDH gene in one embodiment of the present disclosure. FIG. 11 shows a distribution of the editing levels of the mcRNAs designed for the GAN motif of the GAPDH gene in one embodiment of the present disclosure, where the included motifs are GAG and GAA. The black dot represents the editing level of the mcRNA without structural feature and with only one mismatch design of A and C bases at the editing site, while other dots represent the editing levels of the mcRNAs with structural features designed in the present disclosure, and the horizontal and vertical coordinates represent the editing levels of each mcRNA in two biological replicates.
FIG. 12 illustrates target sites and off-target editing of mcRNAs designed for the GAN motif of the GAPDH gene in one embodiment of the present disclosure. FIG. 12 shows off-target editing heat maps of the mcRNAs designed for the GAN motif of the GAPDH gene at the target sites and in the upstream and downstream regions of the target sites in one embodiment of the present disclosure. The GAN motif includes GAG and GAA. In the figure, the position 0 on the horizontal axis is the target site, -1 to -20 is a range upstream of the editing site, and 1 to 20 is a range downstream of the editing site. The vertical axis shows the editing levels of each mcRNA on different A bases. Arranged downward according to the highest editing sequences of the target sites, colors, blue to red, indicate the change trend of the editing levels of the A base sites from low to high.
FIG. 13 illustrates editing levels of mcRNAs designed for a UAG motif of ATP7B gene in one embodiment of the present disclosure. FIG. 13 shows the editing levels of the mcRNAs of different lengths on the UAG motif of the ATP7B gene in one embodiment of the present disclosure, where the black dot indicates that the control mcRNA without secondary structural feature and with only one mismatch of A and C bases at the editing site; the horizontal and vertical coordinates represent the editing levels of each mcRNA in two biological replicates; len41 indicates that the mcRNA is 41 nt in length, and len31 indicates that the mcRNA is 31 nt in length.

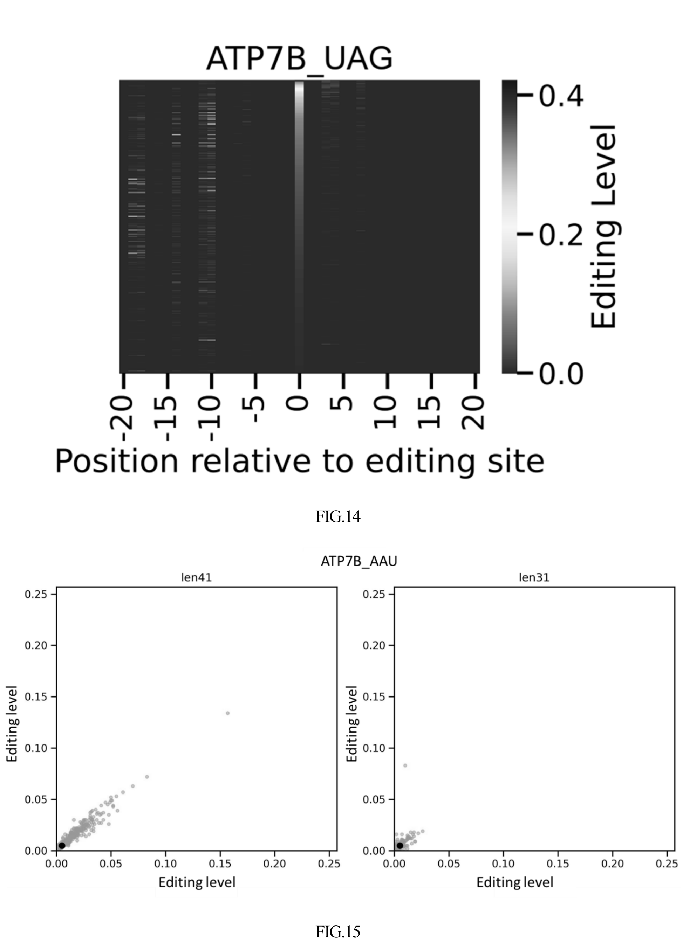

FIG. 14 illustrates target sites and off-target editing of mcRNAs designed for the UAG motif of the ATP7B gene in one embodiment of the present disclosure. FIG. 14 shows a heat map of all mcRNAs of the UAG motif of the ATP7B gene edited at the target sites and off-target edited upstream and downstream in one embodiment of the present disclosure. In the figure, the position 0 on the horizontal axis is the target site, -1 to -20 is a range upstream of the editing site, and 1 to 20 is a range downstream of the editing site. The vertical axis shows the editing levels of each mcRNA on different A bases. Arranged downward according to the highest editing sequences of the target sites, colors, blue to red, indicate the change trend of the editing levels of the A base sites from low to high.
FIG. 15 illustrates editing levels of mcRNAs designed for an AAU motif of the ATP7B gene in one embodiment of the present disclosure. FIG. 15 shows the editing levels of the mcRNAs of different lengths on the AAU motif of the ATP7B gene in one embodiment of the present disclosure, where the black dot indicates that the control mcRNA without secondary structural feature and with only one mismatch of A and C bases at the editing site; the horizontal and vertical coordinates represent the editing levels of each mcRNA in two biological replicates; len41 indicates that the mcRNA is 41 nt in length, and len31 indicates that the mcRNA is 31 nt in length.

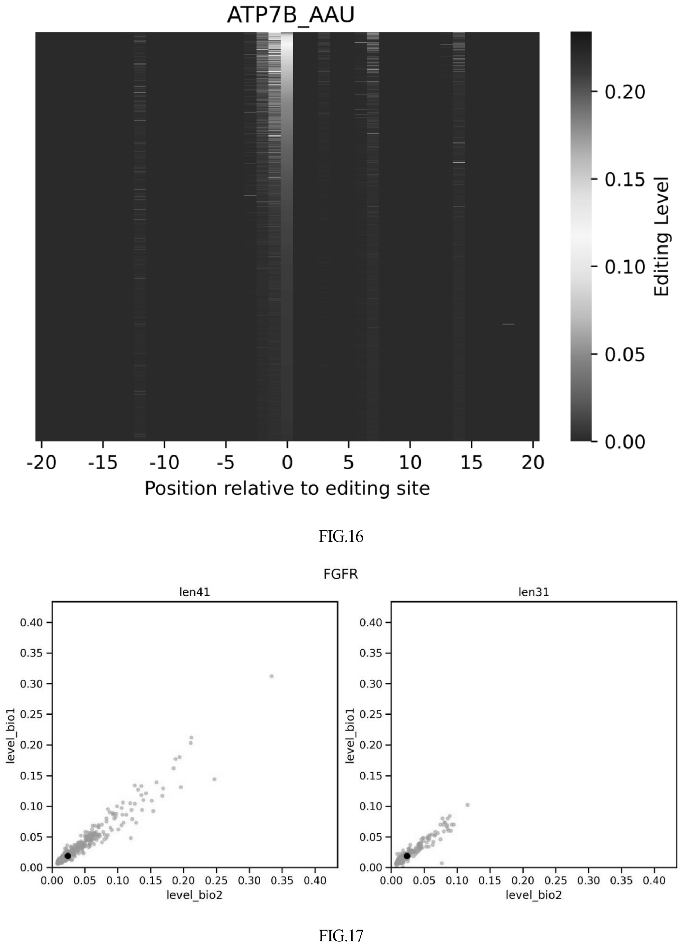

FIG. 16 illustrates target sites and off-target editing of mcRNAs designed for the AAU motif of the ATP7B gene in one embodiment of the present disclosure. FIG. 16 shows a heat map of all mcRNAs of the AAU motif of the ATP7B gene edited at the target sites and off-target edited upstream and downstream in one embodiment of the present disclosure. In the figure, the position 0 on the horizontal axis is the target site, -1 to -20 is a range upstream of the editing site, and 1 to 20 is a range downstream of the editing site. The vertical axis shows the editing levels of each mcRNA on different A bases. Arranged downward according to the highest editing sequences of the target sites, colors, blue to red, indicate the change trend of the editing levels of the A base sites from low to high.
FIG. 17 illustrates editing levels of mcRNAs designed for a CAG motif of FGFR gene in one embodiment of the present disclosure. FIG. 17 shows the editing levels of the mcRNAs of different lengths on the CAG motif of the FGFR gene in one embodiment of the present disclosure, where the black dot indicates that the control mcRNA without secondary structural feature and with only one mismatch of A and C bases at the editing site; the horizontal and vertical coordinates represent the editing levels of each mcRNA in two biological replicates; len41 indicates that the mcRNA is 41 nt in length, and len31 indicates that the mcRNA is 31 nt in length.

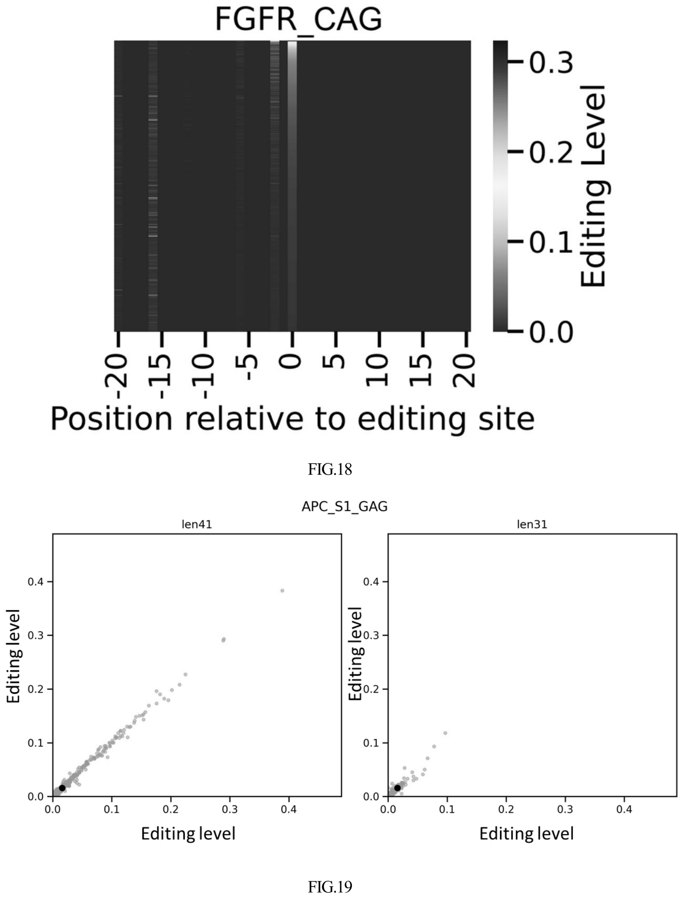

FIG. 18 illustrates target sites and off-target editing of mcRNAs designed for the CAG motif of the FGFR gene in one embodiment of the present disclosure. FIG. 18 shows a heat map of all mcRNAs of the CAG motif of the FGFR gene edited at the target sites and off-target edited upstream and downstream in one embodiment of the present disclosure. In the figure, the position 0 on the horizontal axis is the target site, -1 to -20 is a range upstream of the editing site, and 1 to 20 is a range downstream of the editing site. The vertical axis shows the editing levels of each mcRNA on different A bases. Arranged downward according to the highest editing sequences of the target sites, colors, blue to red, indicate the change trend of the editing levels of the A base sites from low to high.
FIG. 19 illustrates editing levels of mcRNAs designed for a GAG motif of APC gene in one embodiment of the present disclosure. FIG. 19 shows the editing levels of the mcRNAs of different lengths on the GAG motif of the APC gene in one embodiment of the present disclosure, where the black dot indicates that the control mcRNA without secondary structural feature and with only one mismatch of A and C bases at the editing site; the horizontal and vertical coordinates represent the editing levels of each mcRNA in two biological replicates; len41 indicates that the mcRNA is 41 nt in length, and len31 indicates that the mcRNA is 31 nt in length.

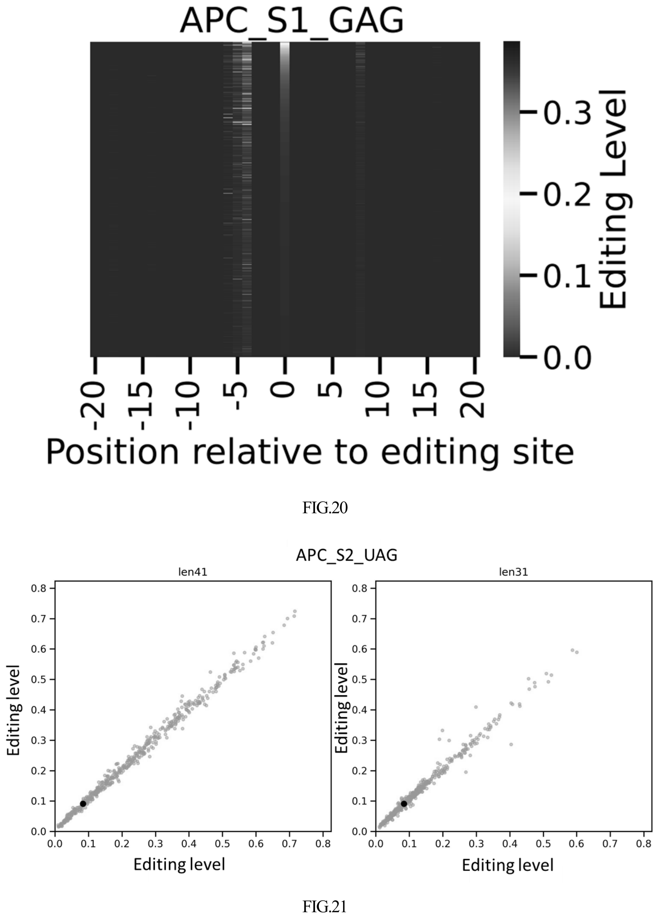

FIG. 20 illustrates target sites and off-target editing of mcRNAs designed for the GAG motif of the APC gene in one embodiment of the present disclosure. FIG. 20 shows a heat map of all mcRNAs of the CAG motif of the APC gene edited at the target sites and off-target edited upstream and downstream in one embodiment of the present disclosure. In the figure, the position 0 on the horizontal axis is the target site, -1 to -20 is a range upstream of the editing site, and 1 to 20 is a range downstream of the editing site. The vertical axis shows the editing levels of each mcRNA on different A bases. Arranged downward according to the highest editing sequences of the target sites, colors, blue to red, indicate the change trend of the editing levels of the A base sites from low to high.
FIG. 21 illustrates editing levels of mcRNAs designed for a UAG motif of the APC gene in one embodiment of the present disclosure. FIG. 21 shows the editing levels of the mcRNAs of different lengths on the UAG motif of the APC gene in one embodiment of the present disclosure, where the black dot indicates that the control mcRNA without secondary structural feature and with only one mismatch of A and C bases at the editing site; the horizontal and vertical coordinates represent the editing levels of each mcRNA in two biological replicates; len41 indicates that the mcRNA is 41 nt in length, and len31 indicates that the mcRNA is 31 nt in length.

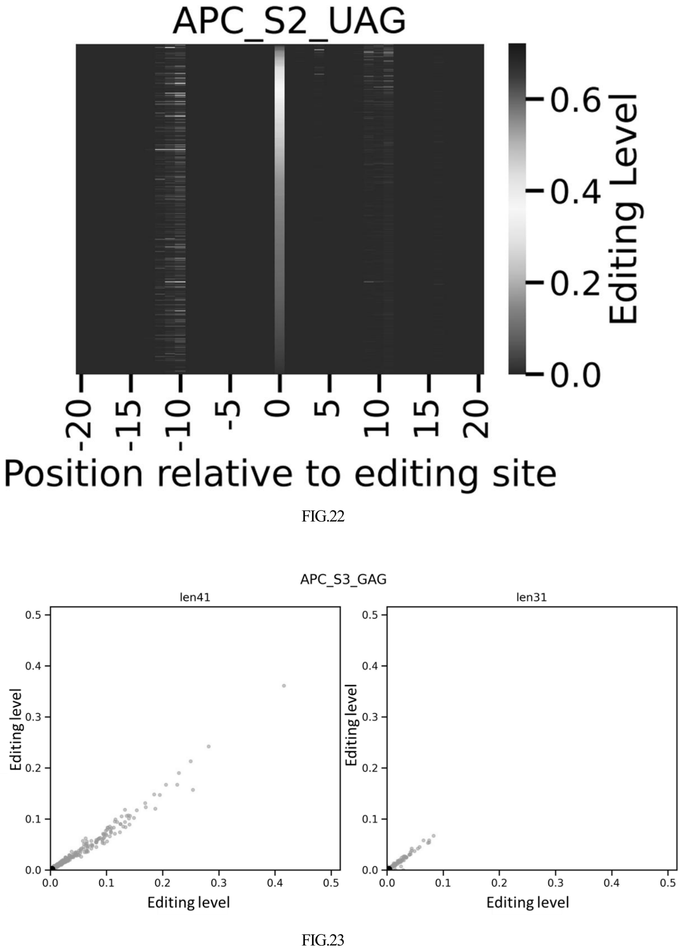

FIG. 22 illustrates target sites and off-target editing of mcRNAs designed for the UAG motif of the APC gene in one embodiment of the present disclosure. FIG. 22 shows a heat map of all mcRNAs of the UAG motif of the APC gene edited at the target sites and off-target edited upstream and downstream in one embodiment of the present disclosure. In the figure, the position 0 on the horizontal axis is the target site, -1 to -20 is a range upstream of the editing site, and 1 to 20 is a range downstream of the editing site. The vertical axis shows the editing levels of each mcRNA on different A bases. Arranged downward according to the highest editing sequences of the target sites, colors, blue to red, indicate the change trend of the editing levels of the A base sites from low to high.
FIG. 23 illustrates editing levels of mcRNAs designed for another GAG motif of the APC gene in one embodiment of the present disclosure. FIG. 23 shows the editing levels of the mcRNAs of different lengths on the another GAG motif of the APC gene in one embodiment of the present disclosure, where the black dot indicates that the control mcRNA without secondary structural feature and with only one mismatch of A and C bases at the editing site; the horizontal and vertical coordinates represent the editing levels of each mcRNA in two biological replicates; len41 indicates that the mcRNA is 41 nt in length, and len31 indicates that the mcRNA is 31 nt in length.

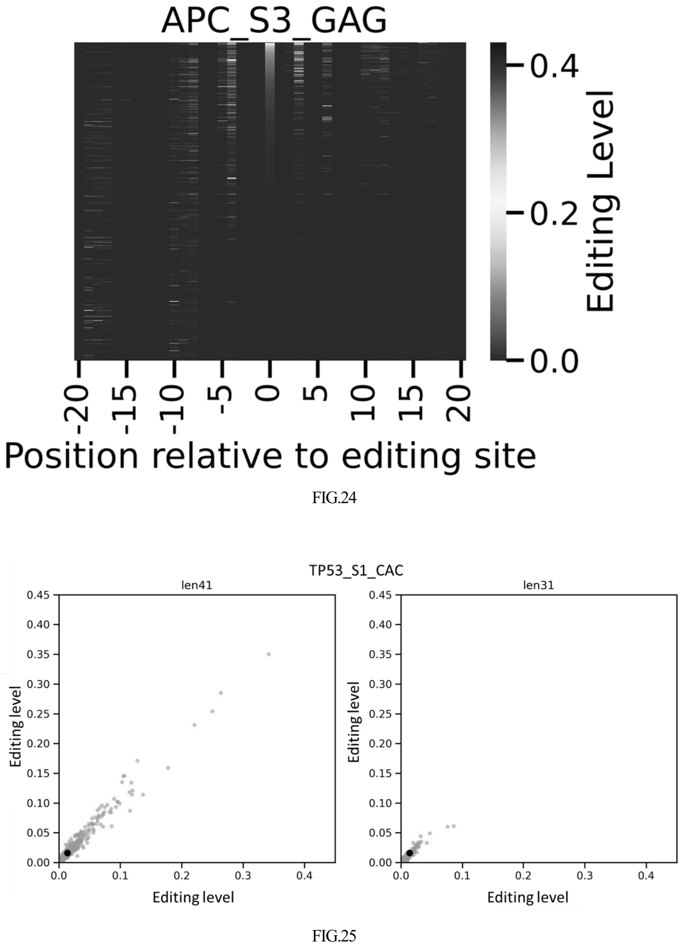

FIG. 24 illustrates target sites and off-target editing of mcRNAs designed for another GAG motif of the APC gene in one embodiment of the present disclosure. FIG. 24 shows a heat map of all mcRNAs of another GAG motif of the APC gene edited at the target sites and off-target edited upstream and downstream in one embodiment of the present disclosure. In the figure, the position 0 on the horizontal axis is the target site, -1 to -20 is a range upstream of the editing site, and 1 to 20 is a range downstream of the editing site. The vertical axis shows the editing levels of each mcRNA on different A bases. Arranged downward according to the highest editing sequences of the target sites, colors, blue to red, indicate the change trend of the editing levels of the A base sites from low to high.
FIG. 25 illustrates editing levels of mcRNAs designed for a CAC motif of TP53 gene in one embodiment of the present disclosure. FIG. 25 shows the editing levels of the mcRNAs of different lengths on the CAC motif of the TP53 gene in one embodiment of the present disclosure, where the black dot indicates that the control mcRNA without secondary structural feature and with only one mismatch of A and C bases at the editing site; the horizontal and vertical coordinates represent the editing levels of each mcRNA in two biological replicates; len41 indicates that the mcRNA is 41 nt in length, and len31 indicates that the mcRNA is 31 nt in length.

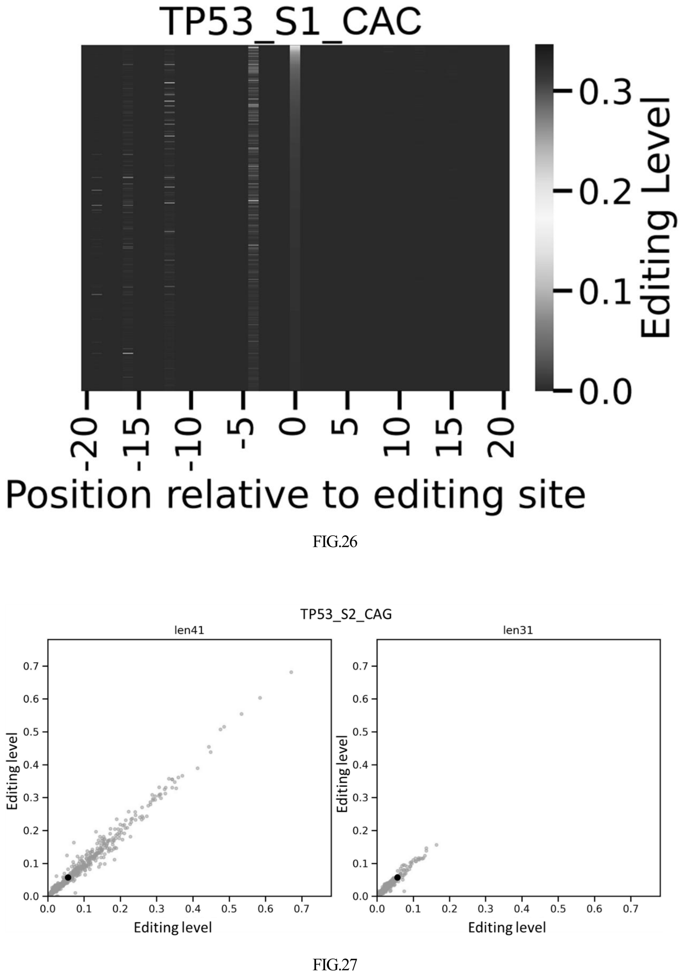

FIG. 26 illustrates target sites and off-target editing of mcRNAs designed for the CAC motif design of the TP53 gene in one embodiment of the present disclosure. FIG. 26 shows a heat map of all mcRNAs of the CAC motif of the TP53 gene edited at the target sites and off-target edited upstream and downstream in one embodiment of the present disclosure. In the figure, the position 0 on the horizontal axis is the target site, -1 to -20 is a range upstream of the editing site, and 1 to 20 is a range downstream of the editing site. The vertical axis shows the editing levels of each mcRNA on different A bases. Arranged downward according to the highest editing sequences of the target sites, colors, blue to red, indicate the change trend of the editing levels of the A base sites from low to high.
FIG. 27 illustrates editing levels of mcRNAs designed for a CAG motif of the TP53 gene in one embodiment of the present disclosure. FIG. 27 shows the editing levels of the mcRNAs of different lengths on the CAG motif of the TP53 gene in one embodiment of the present disclosure, where the black dot indicates that the control mcRNA without secondary structural feature and with only one mismatch of A and C bases at the editing site; the horizontal and vertical coordinates represent the editing levels of each mcRNA in two biological replicates; len41 indicates that the mcRNA is 41 nt in length, and len31 indicates that the mcRNA is 31 nt in length.

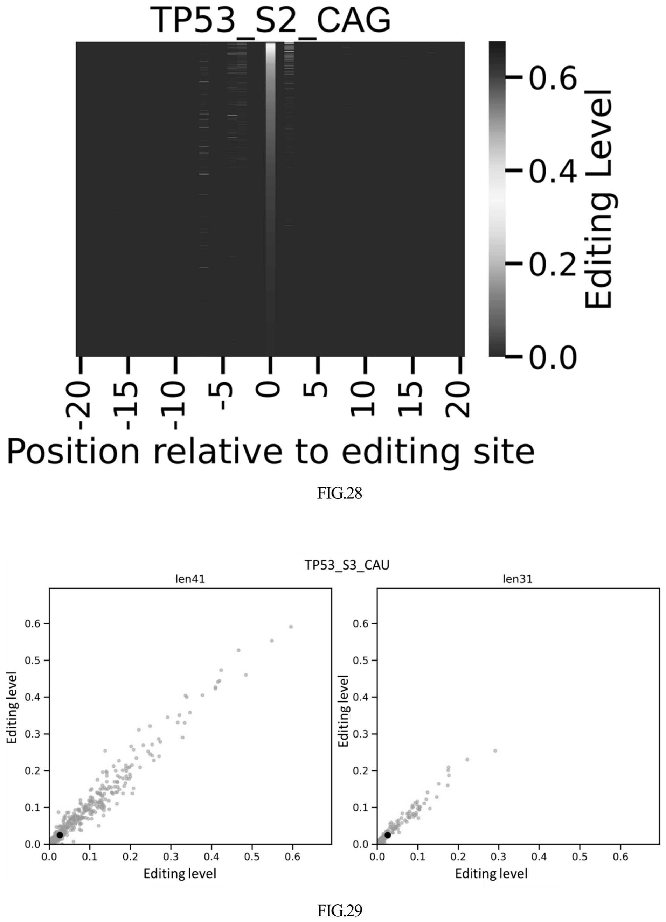

FIG. 28 illustrates target sites and off-target editing of mcRNAs designed for the CAG motif of the TP53 gene in one embodiment of the present disclosure. FIG. 28 shows a heat map of all mcRNAs of the CAG motif of the TP53 gene edited at the target sites and off-target edited upstream and downstream in one embodiment of the present disclosure. In the figure, the position 0 on the horizontal axis is the target site, -1 to -20 is a range upstream of the editing site, and 1 to 20 is a range downstream of the editing site. The vertical axis shows the editing levels of each mcRNA on different A bases. Arranged downward according to the highest editing sequences of the target sites, colors, blue to red, indicate the change trend of the editing levels of the A base sites from low to high.
FIG. 29 illustrates editing levels of mcRNAs designed for a CAU motif of the TP53 gene in one embodiment of the present disclosure. FIG. 29 shows the editing levels of the mcRNAs of different lengths on the CAU motif of the TP53 gene in one embodiment of the present disclosure, where the black dot indicates that the control mcRNA without secondary structural feature and with only one mismatch of A and C bases at the editing site; the horizontal and vertical coordinates represent the editing levels of each mcRNA in two biological replicates; len41 indicates that the mcRNA is 41 nt in length, and len31 indicates that the mcRNA is 31 nt in length.

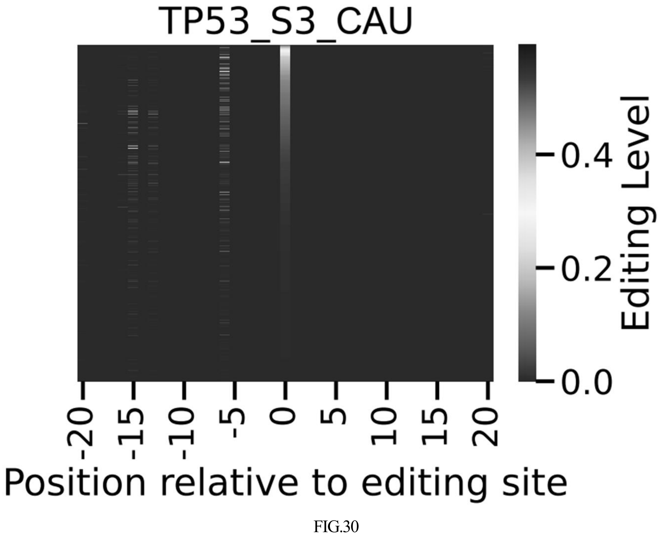

FIG. 30 illustrates target sites and off-target editing of mcRNAs designed for the CAU motif design of the TP53 gene in one embodiment of the present disclosure. FIG. 30 shows a heat map of all mcRNAs of the CAU motif of the TP53 gene edited at the target sites and off-target edited upstream and downstream in one embodiment of the present disclosure. In the figure, the position 0 on the horizontal axis is the target site, -1 to -20 is a range upstream of the editing site, and 1 to 20 is a range downstream of the editing site. The vertical axis shows the editing levels of each mcRNA on different A bases. Arranged downward according to the highest editing sequences of the target sites, colors, blue to red, indicate the change trend of the editing levels of the A base sites from low to high.

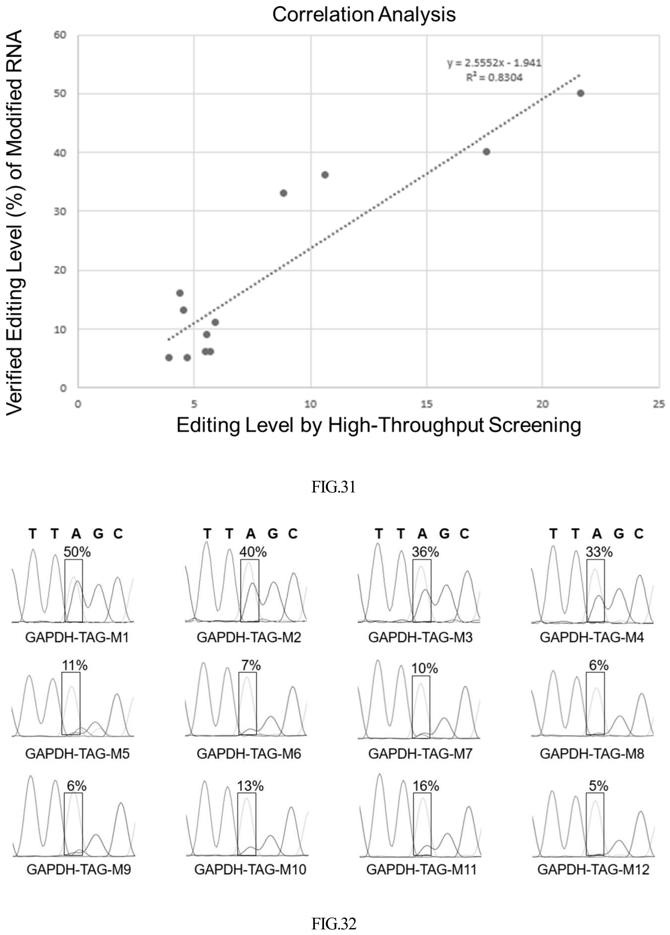

FIG. 31 illustrates verification and analysis of editing levels of mcRNAs obtained by high-throughput sequencing in one embodiment of the present disclosure. FIG. 31 shows verification, in an individual low-throughput manner, of the editing levels of the mcRNAs obtained by high-throughput sequencing in one embodiment of the present disclosure. The horizontal axis indicates the editing level of each mcRNA measured in the high-throughput sequencing method, and the vertical axis indicates an editing level obtained by Sanger sequencing after each mcRNA is separately synthesized into a modified RNA and transfected into a cell. A higher value of the correlation coefficient R2 indicates a higher correlation between the horizontal and vertical coordinates.
FIG. 32 illustrates a Sanger sequencing raw result of an editing level of a single mcRNA on GAPDH in one embodiment of the present disclosure. FIG. 32 shows a raw peak profile of an editing level of a single modified mcRNA transfected into a cell obtained by first-generation sequencing in one embodiment of the present disclosure. As shown in the figure, different bases correspond to peaks of different colors, with the A base corresponding to the green peak and the G base corresponding to the black peak. When peaks of different colors appear at a site, it indicates that there is a different base composition at the site, indicating the editing level of the A base in this result. The editing level is calculated by dividing the peak area of the G base by the sum of the peak areas of A the base and the G base at the site.

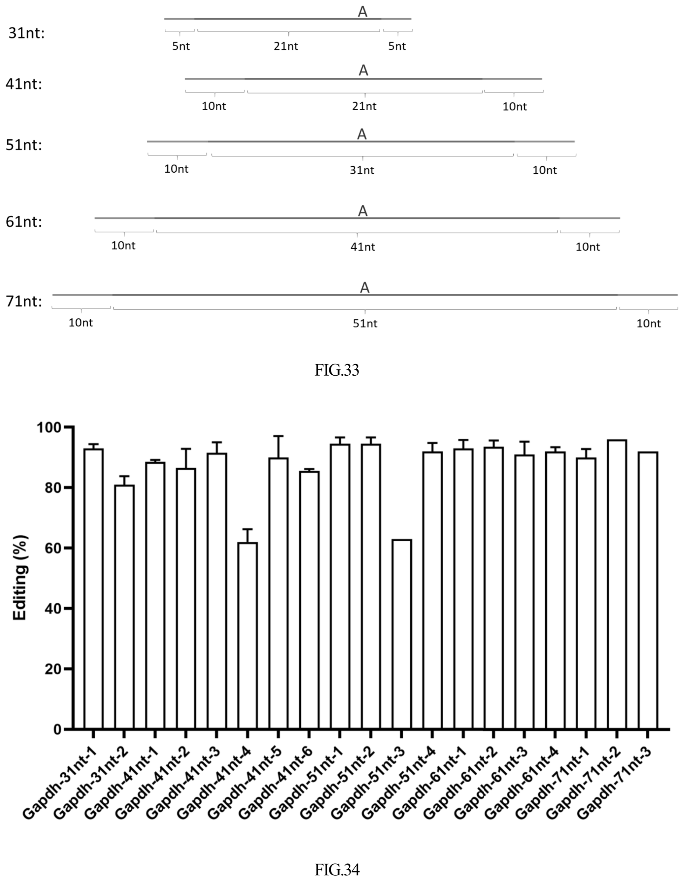

FIG. 33 illustrates mcRNA design patterns of different lengths of the UAG motif of the mouse GAPDH gene in one embodiment of the present disclosure. FIG. 33 shows mcRNAs designed with different lengths and different combinations of mimic domains and binding domains for the UAG motif of the mouse GAPDH gene in one embodiment of the present disclosure. As shown in the figure, the binding domains at both ends of the mcRNA having a length of 31 nt are 5 nt, while the mimic domain in the middle is 21 nt; the binding domains at both ends of the mcRNA having a length of 41 nt are 10 nt, while the mimic domain in the middle is 21 nt; the binding domains at both ends of the mcRNA having a length of 51 nt are 10 nt, while the mimic domain in the middle is 31 nt; the binding domains at both ends of the mcRNA having a length of 61 nt are 10 nt, while the mimic domain in the middle is 41 nt; and the binding domains at both ends of the mcRNA having a length of 71 nt are 10 nt, while the mimic domain in the middle is 51 nt.
FIG. 34 is a diagram showing editing levels of mcRNAs of different lengths for the UAG motif of the mouse GAPDH gene in one embodiment of the present disclosure. FIG. 34 shows mcRNAs of different lengths designed for the UAG motif of the mouse GAPDH gene in one embodiment of the present disclosure. Different numbers of mcRNAs are randomly selected from groups of different lengths to verify the editing level of a single mcRNAs. The cells used are mouse primary hepatocytes. The horizontal axis indicates mcRNAs of different lengths, and the vertical axis indicates the editing level of each mcRNA.

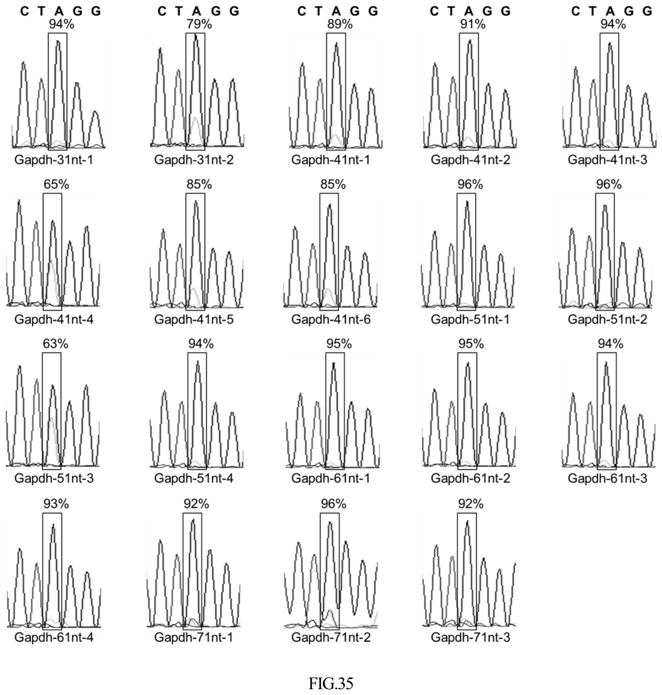

FIG. 35 illustrates raw peak profiles, obtained by first-generation sequencing Sanger, of editing levels of mcRNAs designed for the UAG motif of the mouse GAPDH gene in one embodiment of the present disclosure (biological replicate 1). FIG. 35 shows a raw peak profile of an editing level obtained by performing first-generation sequencing on a single modified mcRNA which is designed for the UAG motif of the mouse GAPDH gene and is transfected into a cell in one embodiment of the present disclosure (biological replicate 1). As shown in the figure, different bases correspond to peaks of different colors, with the A base corresponding to the green peak and the G base corresponding to the black peak. When peaks of different colors appear at a site, it indicates that there is a different base composition at the site, indicating the editing level of the A base in this result. The editing level is calculated by dividing the peak area of the G base by the sum of the peak areas of A the base and the G base at the site.

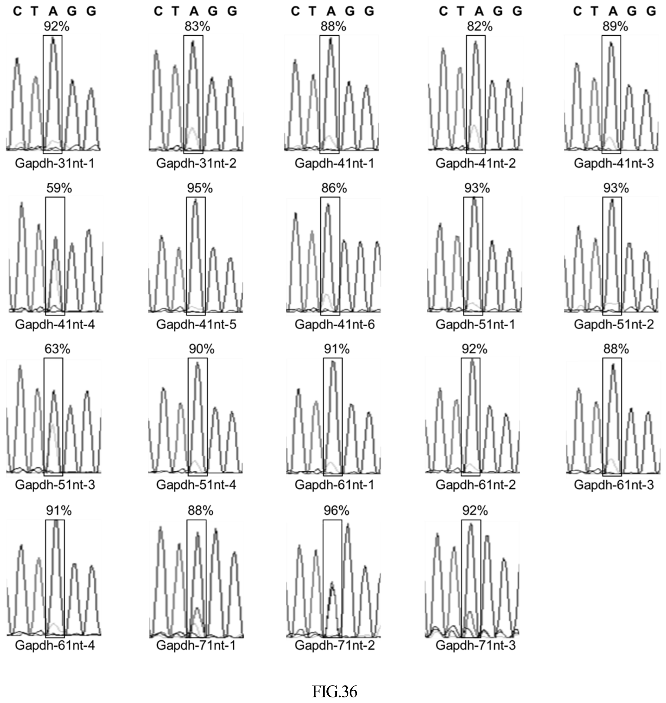

FIG. 36 illustrates raw peak profiles, obtained by first-generation sequencing Sanger, of editing levels of mcRNAs designed for the UAG motif of the mouse GAPDH gene in one embodiment of the present disclosure (biological replicate 2). FIG. 36 shows a raw peak profile of an editing level obtained by performing first-generation sequencing on a single modified mcRNA which is designed for the UAG motif of the mouse GAPDH gene and is transfected into a cell in one embodiment of the present disclosure (biological replicate 2). As shown in the figure, different bases correspond to peaks of different colors, with the A base corresponding to the green peak and the G base corresponding to the black peak. When peaks of different colors appear at a site, it indicates that there is a different base composition at the site, indicating the editing level of the A base in this result. The editing level is calculated by dividing the peak area of the G base by the sum of the peak areas of A the base and the G base at the site.

### DETAILED DESCRIPTION

Hereinafter, the technical solutions of the present disclosure will be further described with reference to specific examples, which do not represent a limitation on the scope of protection of the present disclosure. Some non-essential modifications and adjustments made by others in accordance with the concept of the present disclosure still fall within the scope of protection of the present disclosure.

Unless otherwise specified, the reagents, methods, and devices employed in the present disclosure are conventional reagents, methods, and devices in the art.

Unless otherwise specified, the reagents and materials used in the following examples are commercially available.

### Materials and Methods

### I. Construction of Human ADAR1 Expression Plasmid

The full-length sequence of the human ADAR1 was amplified by PCR and a homology arm was added. The pcDNA3.1 vector was linearized by double digestion with NheI and EcoRI digestion and an insertion fragment was ligated to the vector by homologous recombination.

### II. Construction of High-Throughput Screening mcRNA Vector Library

First, the full-length fluorescent gene GFP and a gene fragment to be edited were amplified by PCR, with homologous sequences obtained by homologous recombination added to both ends. Moreover, the empty pcDNA3.1 vector was double-digested with NheI and HindIII, followed by linearized vector recovery. Multi-fragment recombination was then performed to achieve homologous recombination of the vector and the insertion fragment, completing the construction for a reporter gene vector. The mcRNA mixed sequence pool was amplified by PCR and a homology arm was added at the same time. The reporter gene vector was linearized by double digestion with BamHI and EcoRI, and the mcRNA sequence pool was inserted into the reporter gene vector by homologous recombination. After transformation and coating, all monoclones were collected together for plasmid extraction, and finally the mcRNA sequence pool plasmid library was obtained.

### III. HEK293 Cell Line Culture

### Steps of Cell Passage Experiment:

(1) An ultra-clean workbench was sterilized by ultraviolet irradiation for 30 minutes.
(2) Cells to be passaged were taken out from an incubator, and washed for three times with PBS first. The cells should be added, pipetted and discarded gently to prevent the cells from falling off. Then, 0.25% trypsin that could flood the cell surface was added. After the cells became rounded, a culture medium containing a serum was added to terminate digestion.
(3) The digested cell suspension was transferred into a 15 ml centrifuge tube and centrifuged at 1000 rpm at room temperature for 4 minutes.
(4) The supernatant was pipetted and discarded carefully. A fresh culture medium was added, and the cells were blown to form a single cell suspension.
(5) 20 ul Cell suspension was added to a 96-well cell culture plate, and then 20 ul trypan blue staining solution was added thereto. After blowing and mixing well, 20 ul mixture was aspirated and added to a cell counting plate, and cells were counted under a microscope.
(6) The volume of the cell suspension required for a specific number of cells was calculated according to the size of the inoculated cell culture plate, and a corresponding volume of fresh cell culture medium was added thereto and together put in a cell incubator for culture.

### IV. Steps of Cell Line Transfection Experiment:

(1) The required cell culture plate was set according to the amount of cells required by the experiment, and a certain amount of culture medium and a certain amount of cells were added to the corresponding culture plate according to the method of cell passage. The cell transfection experiment could be carried out when 70% confluence could be reached after cell growth for 24 hours.
(2) The ultra-clean workbench was sterilized by ultraviolet irradiation for 30 minutes.
(3) A mixed transfection reagent solution was prepared according to the instructions provided by the transfection reagent lipofectamine 3000, and let to stand at room temperature for 5 minutes.
(4) The cells were taken out, and the transfection solution was carefully added to the cells, gently mixed evenly and put in the incubator.

### V. Construction of ADAR1 Overexpressed HEK293 Cell Strain

According to the above steps of cell passage and transfection, the ADAR1 expression vector was transfected into HEK293 cells, and the screening drug G418 was added 48 hours later. The solution was changed every two days until there were no viable cells in the non-transfected group, thereby completing the screening of the HEK293 cell strain stably transfected with ADAR1.

### VI. Construction and Quality Inspection of Targeted Sequencing Library

The total cell RNAs could be extracted with a conventional RNA extraction kit to ensure that the RNAs were not obviously degraded. A conventional reverse transcription kit was adopted for reverse transcription reaction. During the first round of PCR amplification, sequencing linker sequences needed to be added at both ends of the amplification primer, and the number of cycles of the first round of amplification was controlled to be less than 20 cycles. The first-round PCR products were verified by agarose gel electrophoresis, followed by recovery of library fragments of the corresponding sizes. After purification, the second round of PCR amplification was carried out, where the second-round primer had sequencing linker sequences and specific base barcode sequences. The second round of PCR amplification was typically controlled to be less than 10 cycles. Again, the PCR products were subjected to agarose gel electrophoresis to confirm the sizes of the library fragments, and the purified library was recovered by gel cutting. Finally, the targeted sequencing library was subjected to second-generation sequencing.

### VII. Analysis of Targeted Sequencing Data

Firstly, the original data was subjected to linker removal processing by cutadapt software, and then the original read length after the linker removal was aligned to the reference sequence. According to the linker sequence, the editing of the reporter gene was corresponded to each mcRNA one to one, and then the editing level of the reporter gene was calculated to obtain the editing level of each mcRNA to the target site.

### VIII. Isolation and Culture of Mouse Primary Hepatocytes

(1) Preparation of 1 × HBSS solution: 100 mL of 10 × HBSS was put into a beaker, and then 850 mL of double distilled water was added thereto. Sodium bicarbonate powder was added to adjust the pH of the solution to 7.5. Finally, the solution was diluted to 1000 mL.
(2) Preparation of pre-perfusion fluid: 500 µL of EGTA solution with a concentration of 0.5 M (final concentration: 0.5 mM) was added to 500 mL 1 × HBSS solution, filtered and sterilized, and freshly prepared for immediate use.
(3) Preparation of perfusion fluid: calcium chloride (final concentration: 3 mM) and collagenase IV (final concentration: 100 U/mL) were added to 500 mL of 1 × HBSS solution, fully dissolved, filtered and sterilized, and freshly prepared for immediate use.
(4) Isotonic Percoll separation solution: 1 mL of 10 × HBSS solution was added to 10 mL of Percoll solution, thereby obtaining the isotonic Percoll separation solution with a concentration of 90%.
(5) An electro-thermostatic water cabinet was turned on for preheating, where the preheating temperature of the water cabinet was 42°C. Reagents that needed to be preheated included the pre-perfusion fluid and the perfusion fluid.
(6) Surface sterilization: the surgical instruments and the operating table were sterilized with 75% alcohol.
(7) DMEM was pre-cooled on ice.
(8) A mouse was anesthetized by intraperitoneal injection.
(9) After the mouse was completely anesthetized, the mouse was fixed on a foam board, and the whole mouse was sterilized with 75% alcohol.
(10) Along the medioventral line, the skin and peritoneum of the mouse abdomen were cut open and fixed with a needle.
(11) A cotton swab was used to move the large and small intestines of the mouse to the right, exposing the portal vein (the vein through which blood flows into the liver, between the liver and stomach) and the inferior vena cava (the vein through which blood flows out of the liver) of the mouse.
(12) The mouse liver was gently pulled down with a cotton swab such that the superior vena cava at the junction of the liver and diaphragm was found and clamped with a vein clamp to block the blood flow back to the heart.
(13) A retention needle was inserted into the hepatic portal vein. When there was blood backflow in the hose after the needle core was withdrawn, it indicated successful insertion into the blood vessel. The hose of a peristaltic pump was connected into the retention needle and the inferior vena cava was cut off.
(14) The peristaltic pump was started to perfuse the pre-perfusion fluid into the mouse liver. The pre-perfusion fluid was always kept in a water bath at 42°C, the perfusion flow rate was 5 mL/min, and the perfusion time was 5 min. During the perfusion process, the inferior vena cava was compressed with a cotton swab every 30 s for 7 s, so that the pre-perfusion fluid filled the mouse liver (the liver was swelling due to water filling). The perfused liver should be white, and the liquid left by the inferior vena cava should not be red.
(15) The peristaltic pump was stopped. The pre-perfusion fluid was replaced with perfusion fluid (the perfusion fluid was always kept in the water bath at 42°C) to continue to perfuse the liver, where the flow rate was adjusted to 3 mL/min, and the perfusion time was 8 min. During the perfusion process, the inferior vena cava was compressed with a cotton swab every 30 s for 7 s, so that the pre-perfusion fluid filled the mouse liver. The perfused liver should be soft and inelastic, and cracked lines could be seen on the surface of the liver.
(16) The liver was gently separated with scissors and tweezers (at this time, the liver became very fragile, so as to avoid breaking the capsule and causing hepatocytes to flow out), and put in a culture dish filled with DMEM. The surface of the liver was gently rinsed, and the gallbladder was removed (the whole process was carried out on ice).
(17) The liver was transferred to another clean culture dish filled with DMEM. The liver capsule was gently torn open with tip-curved forceps, and the liver was shaken. It could be seen that the hepatocytes flowed out, and the whole solution became turbid (the whole process was carried out on ice).
(18) The liver was shaken until all hepatocytes flowed out. At this time, only fibrous tissue was left in the whole liver. A rinsing solution was pipetted with a Pasteur pipette and filtered through a 70 µm cell strainer into a 50 mL centrifuge tube (the whole process was carried out on ice).
(19) After centrifuging with 50 g at 4°C for 2 minutes, a large amount of hepatocytes could be seen precipitated at the bottom.
(20) The supernatant was removed. The hepatocytes were resuspended with 7.5 mL of DMEM, then added with 5 mL of isotonic Percoll separation solution, mixed well, and centrifuged with 200 g at 4°C for 15 min.
(21) After density separation, the hepatocytes precipitated at the bottom were cells with good activity, and the cells suspended on the solution were dead cells and cells with poor activity. The supernatant was removed by suction in vacuum.
(22) The hepatocytes were rinsed twice with 15 mL of DMEM, and resuspended and precipitated with a hepatocyte plating medium, followed by cell counting and adjusting the cell density to 2 × 10⁵ cells/mL. The cells were seeded into a 12-well cell culture plate.
(23) After 4 hours, after the hepatocytes were completely adhered to the wall, a hepatocyte maintenance medium was used instead for next experiment.

### IX. Preparation of Sample for First-Generation Sequencing Sanger

Firstly, the total RNAs of cells were extracted and subjected to quality inspection by using a conventional commercially available RNA extraction kit, and the purified total RNAs were digested with DNases before the reverse transcription reaction. After reversal, the upstream and downstream sequences of the editing site were amplified with a common PCR amplification enzyme, and purified, and finally subjected to Sanger sequencing.

### X. Measurement of Editing Level of RNA with Sanger Data

With the original sequencing file obtained by Sanger sequencing, the editing level of the editing site needed to be measured. In the disclosure, EditR software (https://moriaritylab.shinyapps.io/editr_v10/) was adopted for calculation and analysis. The software would analyze the peak of each of the A, T, G, and C bases in the upstream and downstream regions of the editing site, and the calculation approach for the editing level was 100 * (1-A peak/sum of A + T + C + G peaks).

### XI. Synthesis of Modified mcRNA

In some examples, the modified backbones and base sequences were chemically synthesized. The following types were mainly included: phosphorothioate backbone modification (with a structure as shown in Formula I), and ribose LNA modification (with a structure as shown in Formula II).

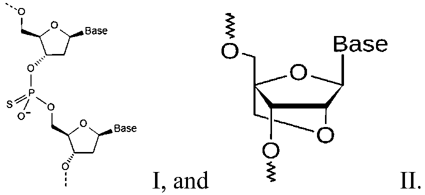

### Example 1. Design of 151 nt Long mcRNA by MASSA Method to Edit Specific Site of Human GAPDH Gene

In this example, a design method (FIG. 2), i.e., mimic ADAR substrates structure approach (MASSA), was develop by simulating the structural features of the natural double-stranded RNA substrate with a high editing level in the human body, and an mcRNA with certain structural characteristics was formed with upstream and downstream sequences of a target site, thereby achieving efficient target RNA editing.

In this example, the design of the present disclosure was verified by selecting the site where the motif No. 1323 of the human GAPDH gene was UAA. The mcRNA was designed by extending upstream and downstream, each for 75 nt, with a total length of 151 nt, with the editing site as the center. In this example, different lengths of mimic domains and binding domains were designed, where the lengths of the mimic domains were 41 nt, 61 nt, and 91 nt, and the lengths of the corresponding binding domains at both ends were 55 nt, 45 nt, and 30 nt, respectively. Furthermore, a control mcRNA without secondary structure characteristics was also designed in this example, and only the position aligned with the editing site was set to the C base, while other positions were complementary base pairs. The specific implementation steps of this example were as follows:

### 1) Preparation of mcRNA Reference Substrate Structure

According to the UAA editing motifs selected in this example, the secondary structures of the reference substrates of the corresponding UAA motifs were selected, and the features of the secondary structures of the corresponding lengths were extracted (as shown in Table 1). The strand where the editing site of the reference substrate was located was the editing strand of the reference substrate, and the other strand was the complementary strand of the reference substrate.

### 2) Construction of mcRNA Sequence Complementarily Paired with Target Editing Site

With the A bases of the UAA motifs on the GAPDH to be edited as centers, the mimic domains of the corresponding lengths were selected by extending upstream and downstream. The strand where the site to be edited was located was the target editing strand, and the strand where the mcRNA was located was the mcRNA strand.

### 3) Design of Mimic Domain of mcRNA Sequence

The base pairs of each target editing strand and each mcRNA sequence strand were aligned with those of the editing strand of the reference substrate and its complementary strand one by one. When the bases of the two editing strands were the same, the mcRNA strand sequence was directly set as the complementary strand sequence of the reference substrate. When the bases of the two were different, they were designed to be the same structure. For example, there was a mismatch of one base at a certain site, and the base at the corresponding position of the mcRNA sequence strand was designed as a mismatch. The sequence design of the whole mimic domain was completed according to such a principle.

### 4) Design of Binding Domain of mcRNA Sequence

On the basis of step 3, the complementarily paired sequences were directly extended at both ends of the mcRNA to complete the sequence design of the entire mcRNA. In this example, the lengths of the corresponding binding domains at both ends were 55 nt, 45 nt, and 30 nt, respectively.

### 5) Export of mcRNA Sequence Library

The RNAfold software was used to calculate the minimum free energy of the pairing region of the mcRNA and the target editing sequence, and the sequence information was exported, thereby completing the design of the mcRNA sequence library (see Table 2 for the mcRNA sequences designed in this example, in particular mcRNA661 to mcRNA1205, with the reference secondary structures of the mcRNA sequences shown in Table 1).

### 6) High-Throughput Verification of mcRNA Sequence Editing Effect

The synthesized mcRNA sequence library was constructed into a plasmid vector, and then the plasmid was transfected into ADAR1 overexpressed HEK293 cells. After 48 hours, the RNAs were extracted and the target site editing level corresponding to each mcRNA was analyzed by targeted library sequencing (as shown in FIG. 3).

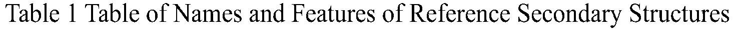

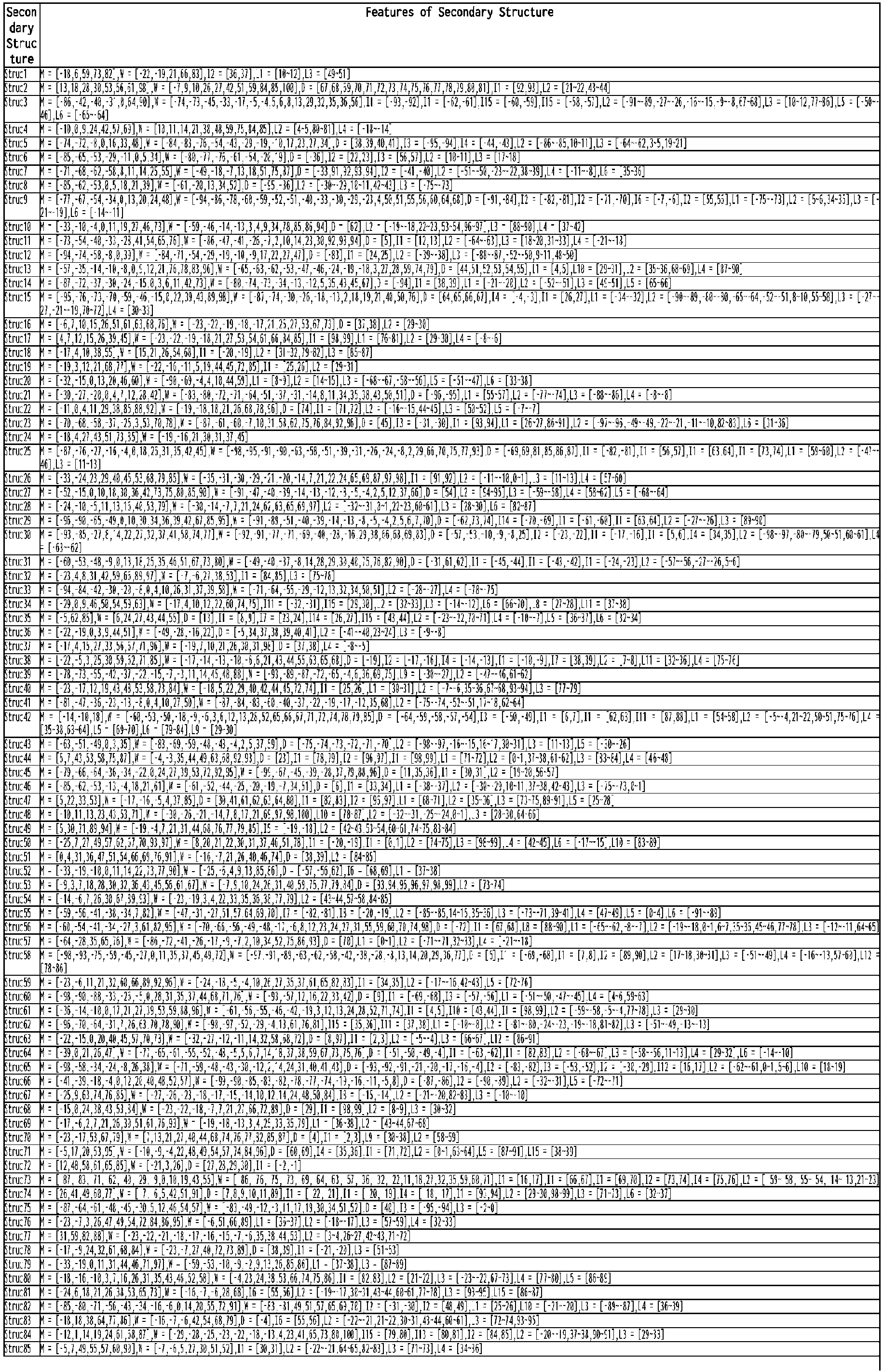

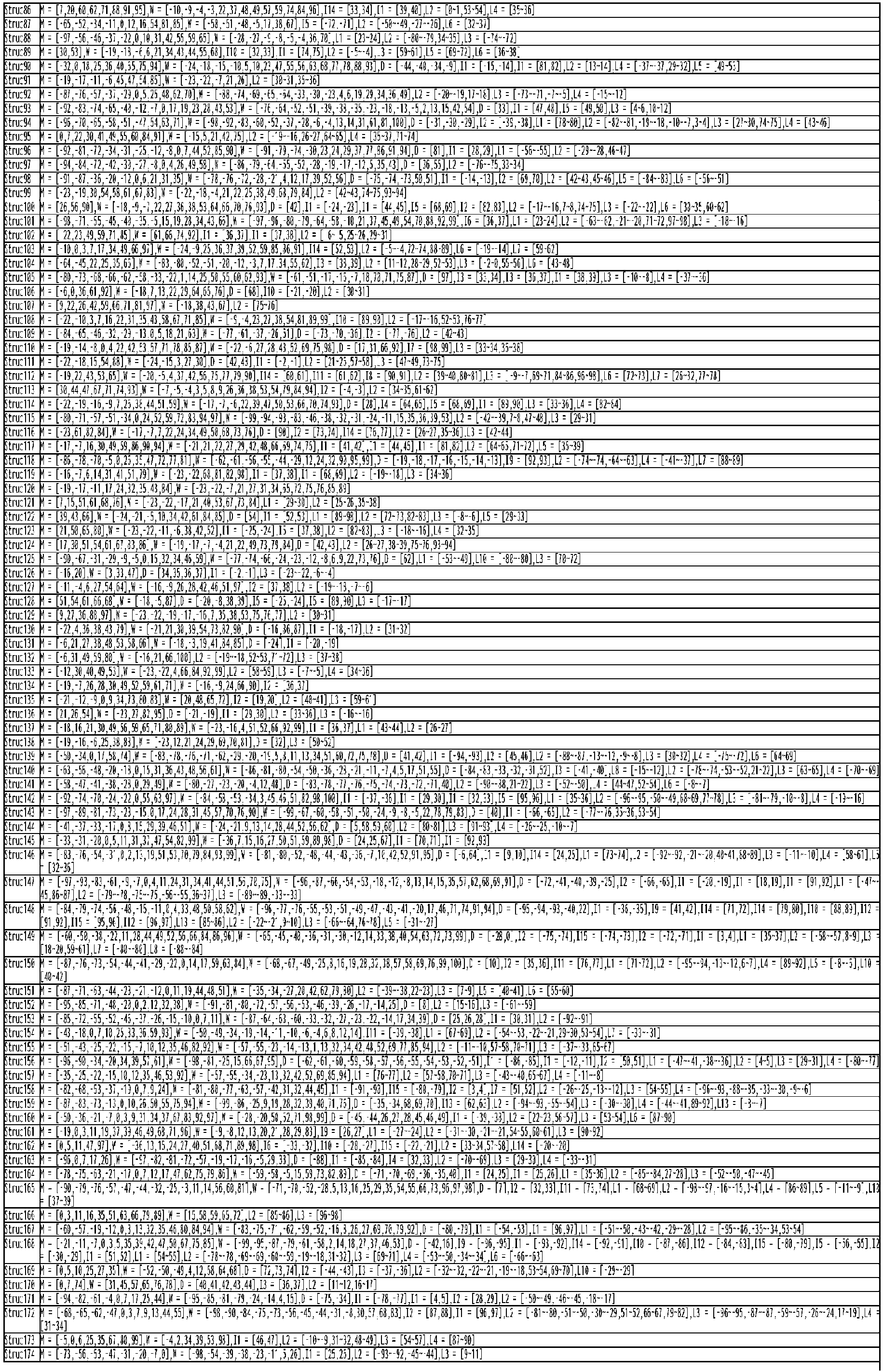

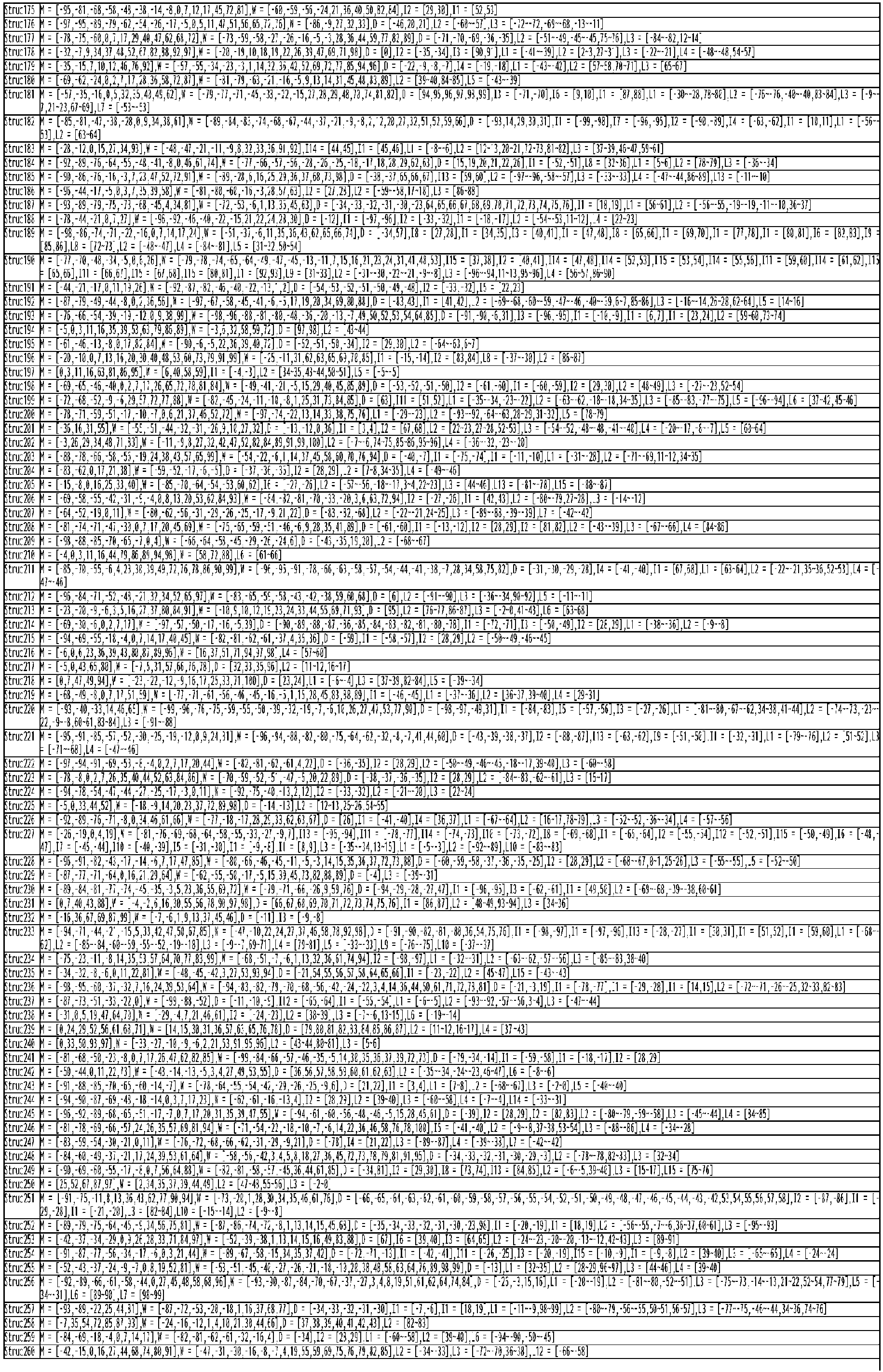

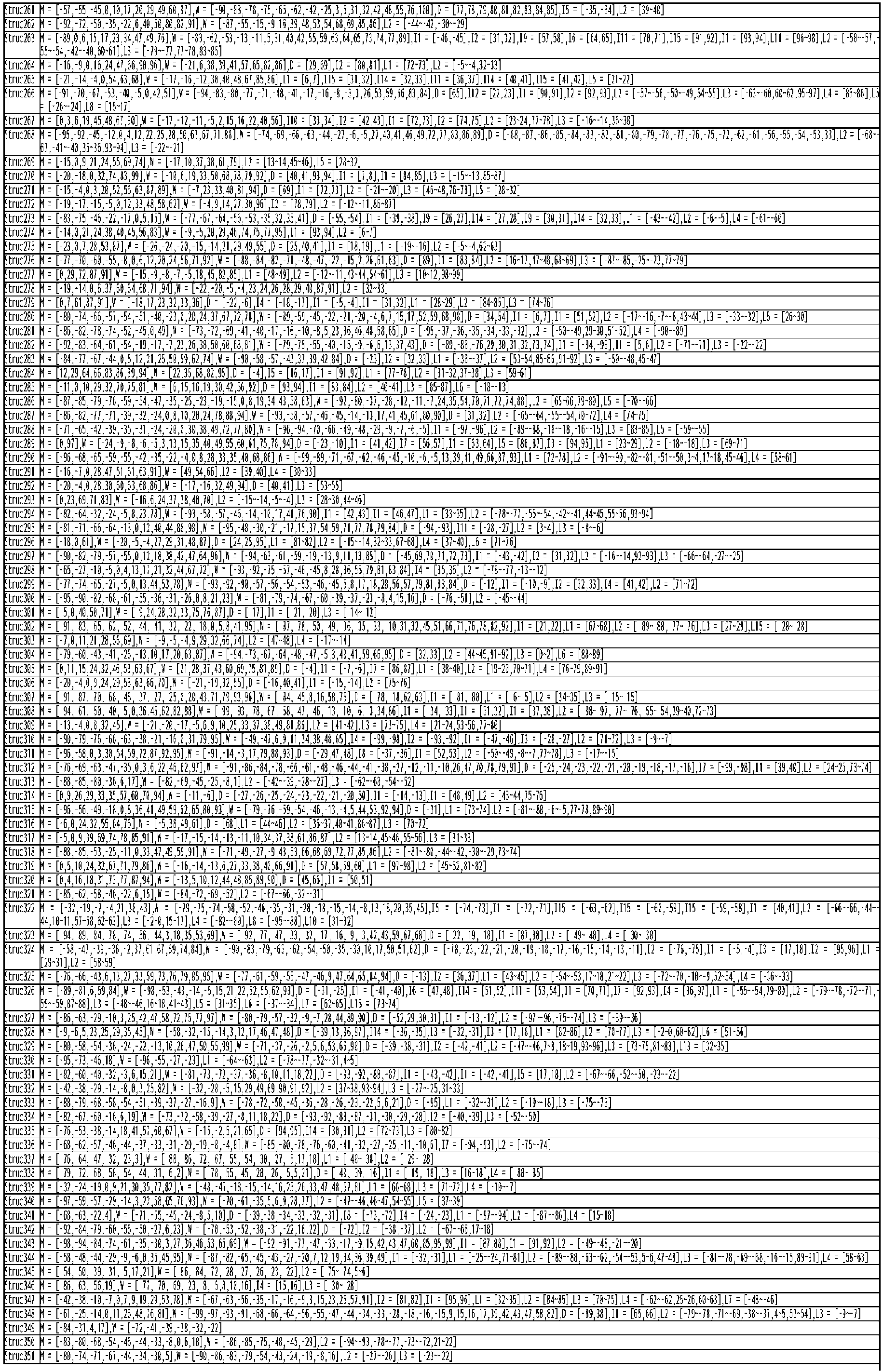

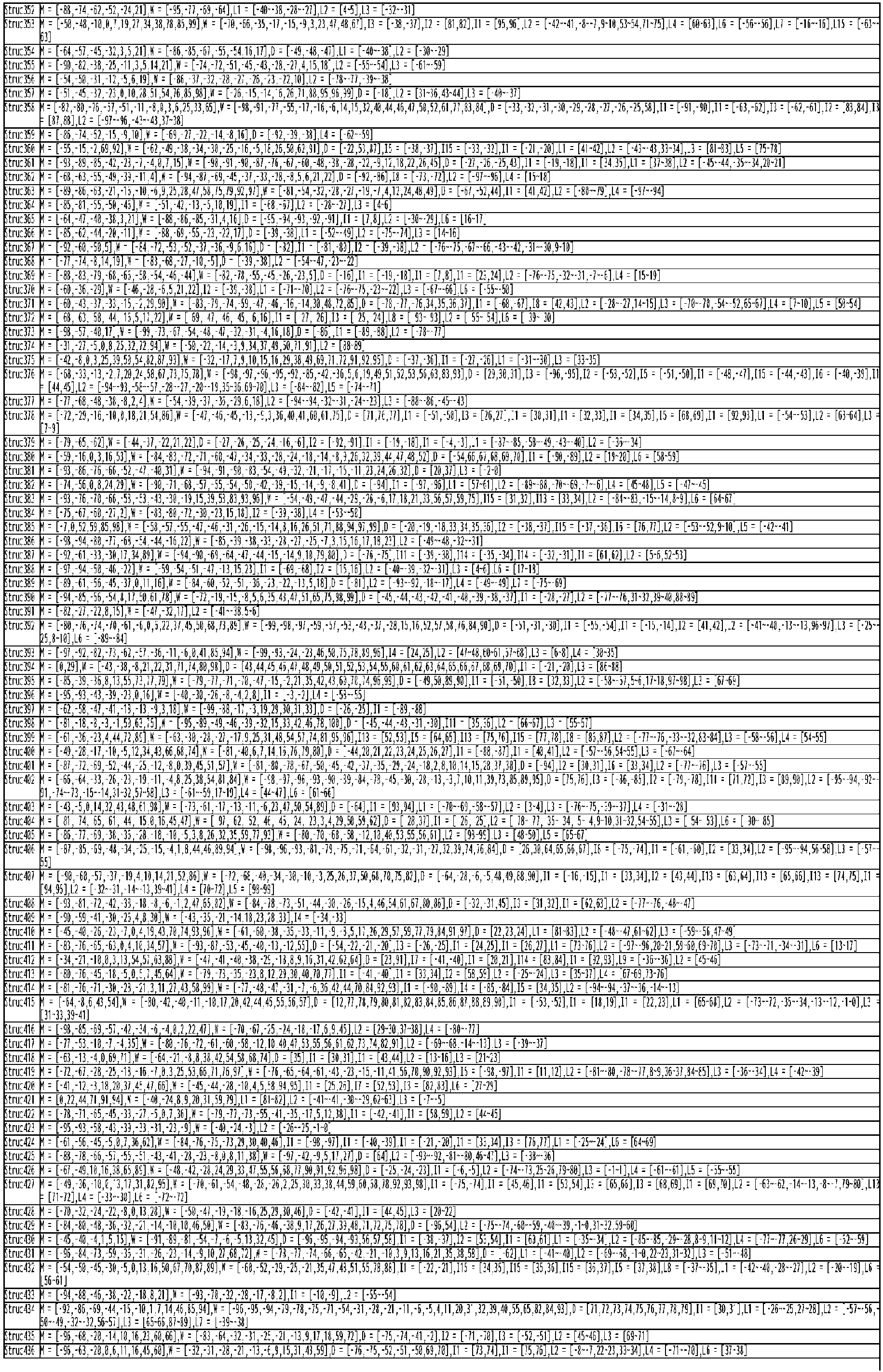

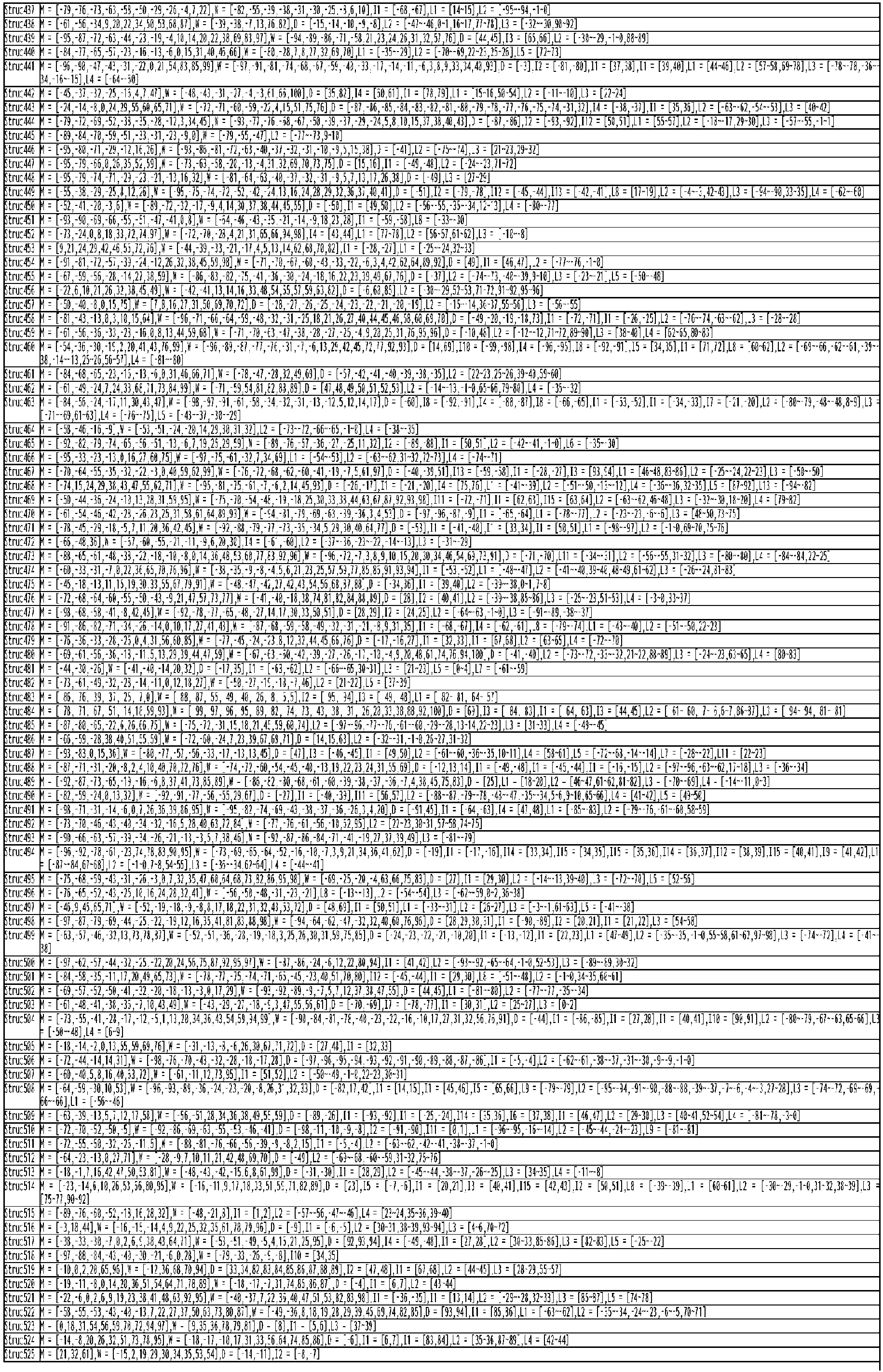

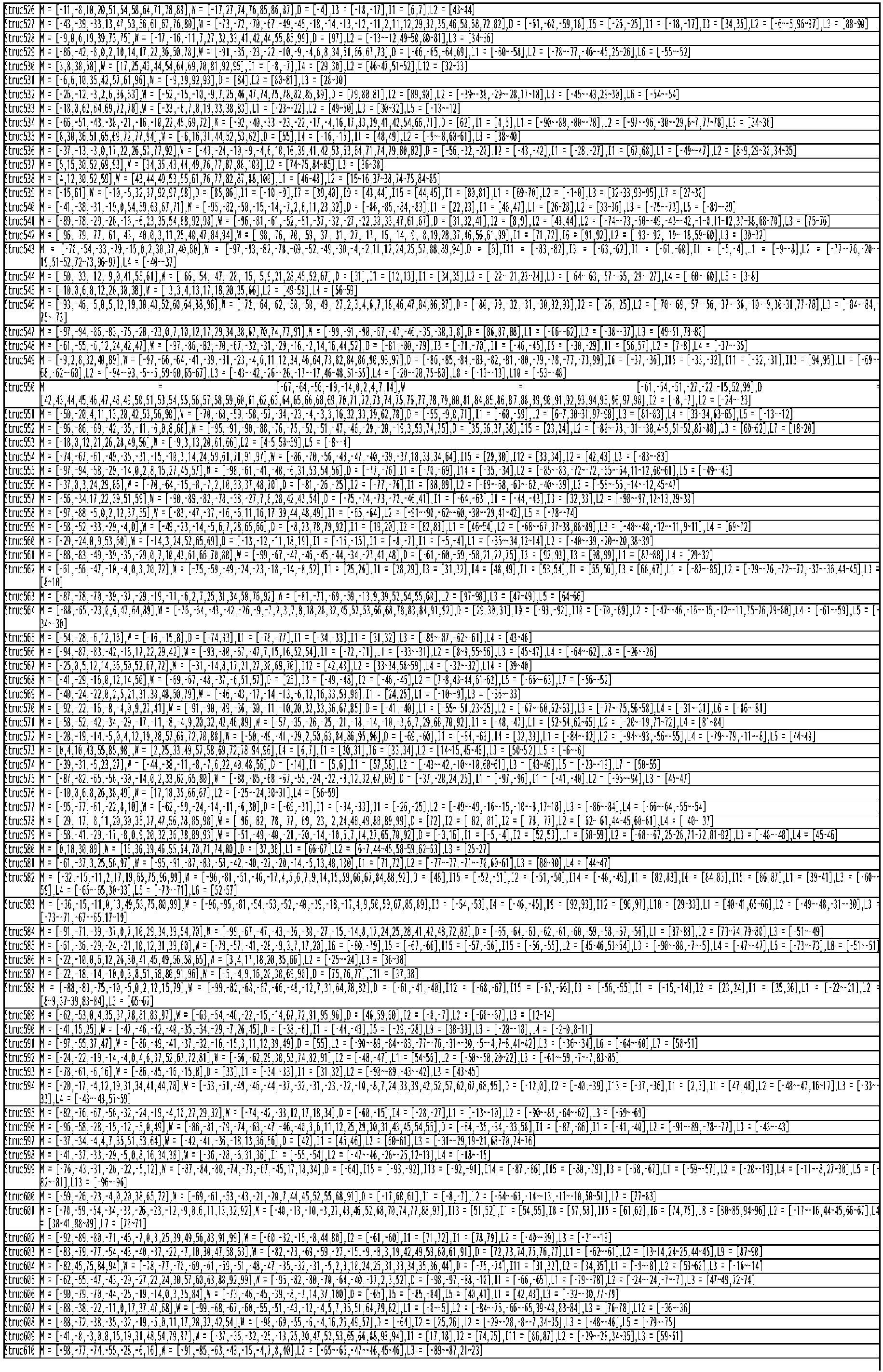

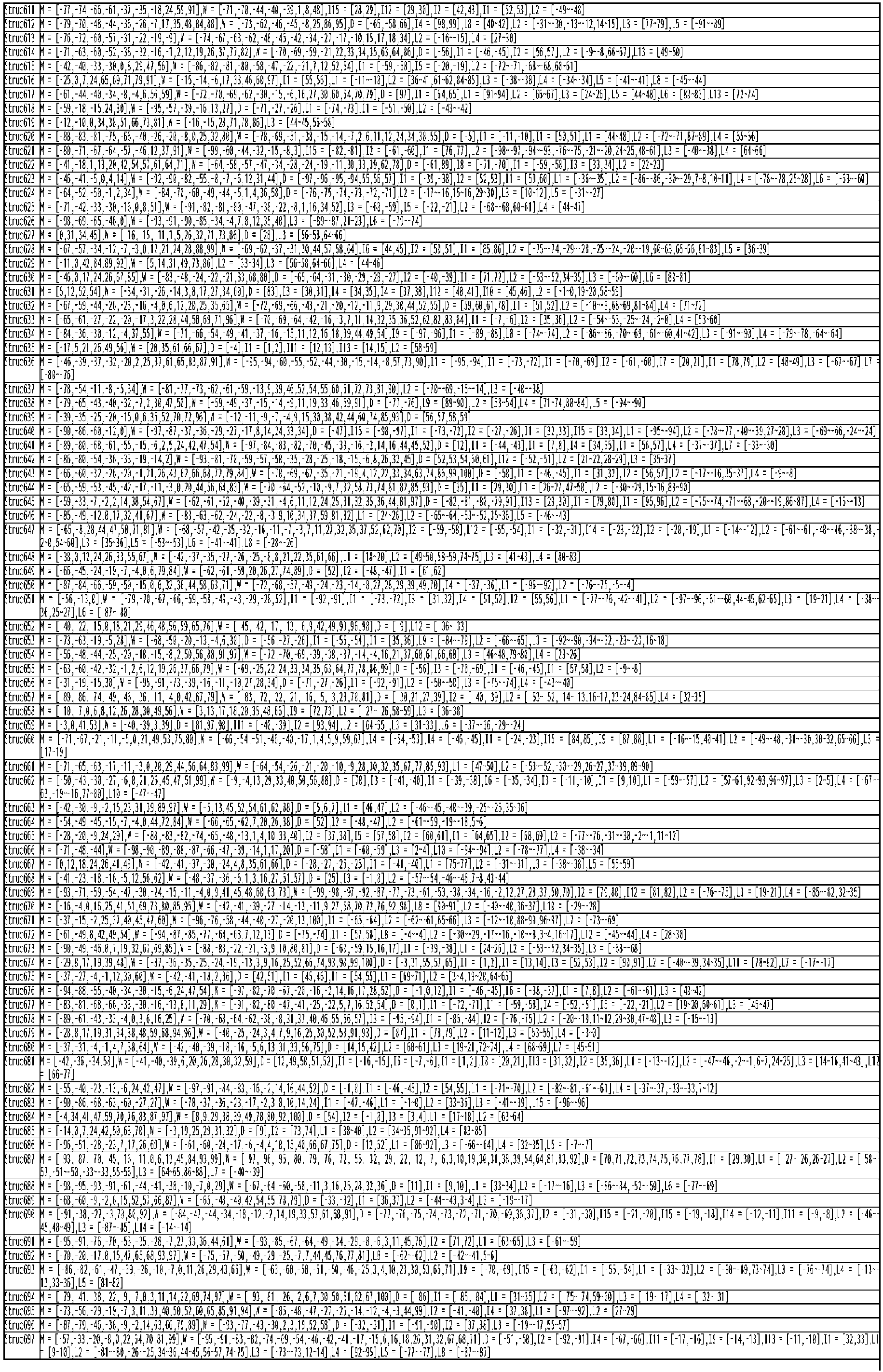

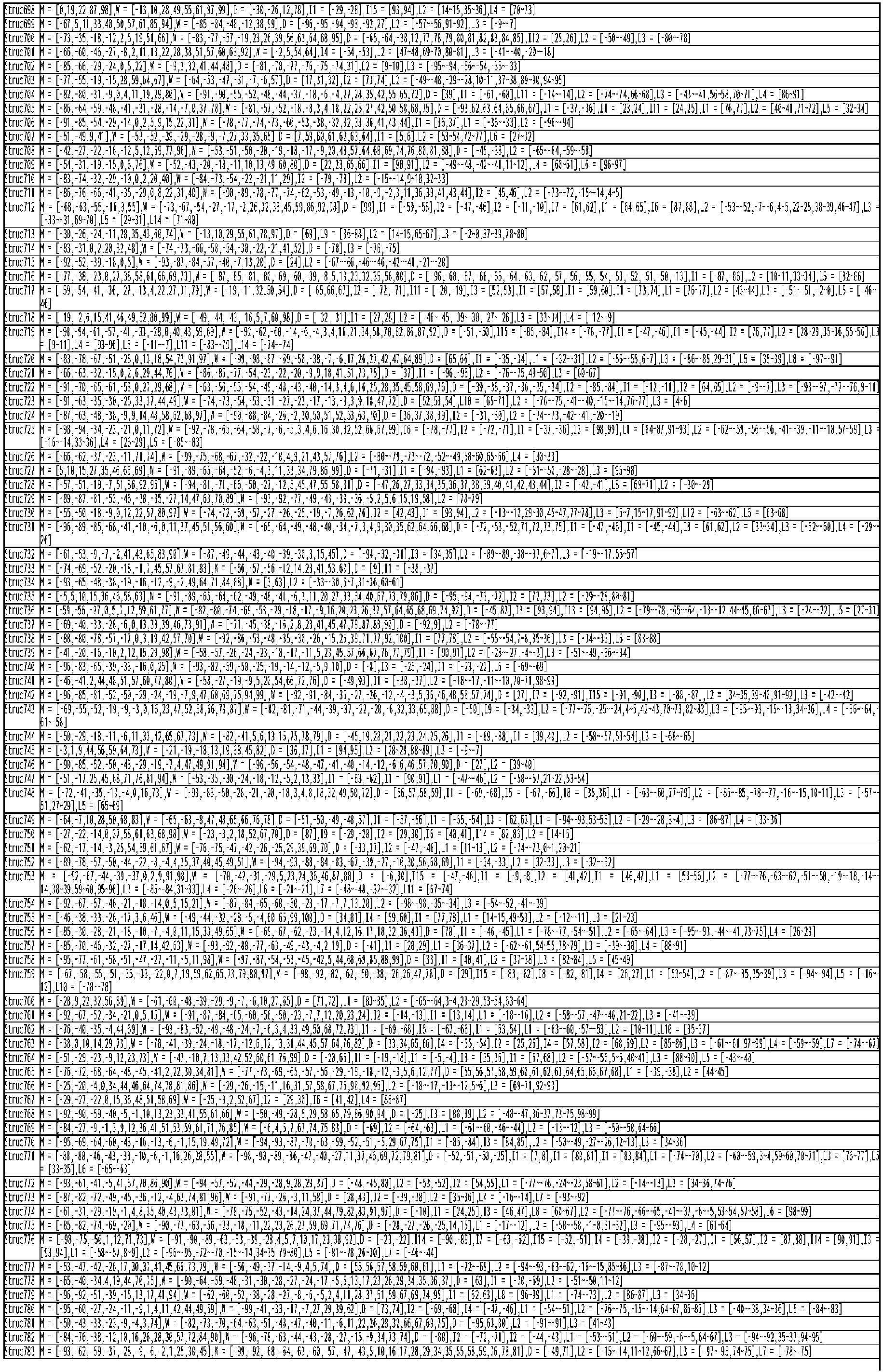

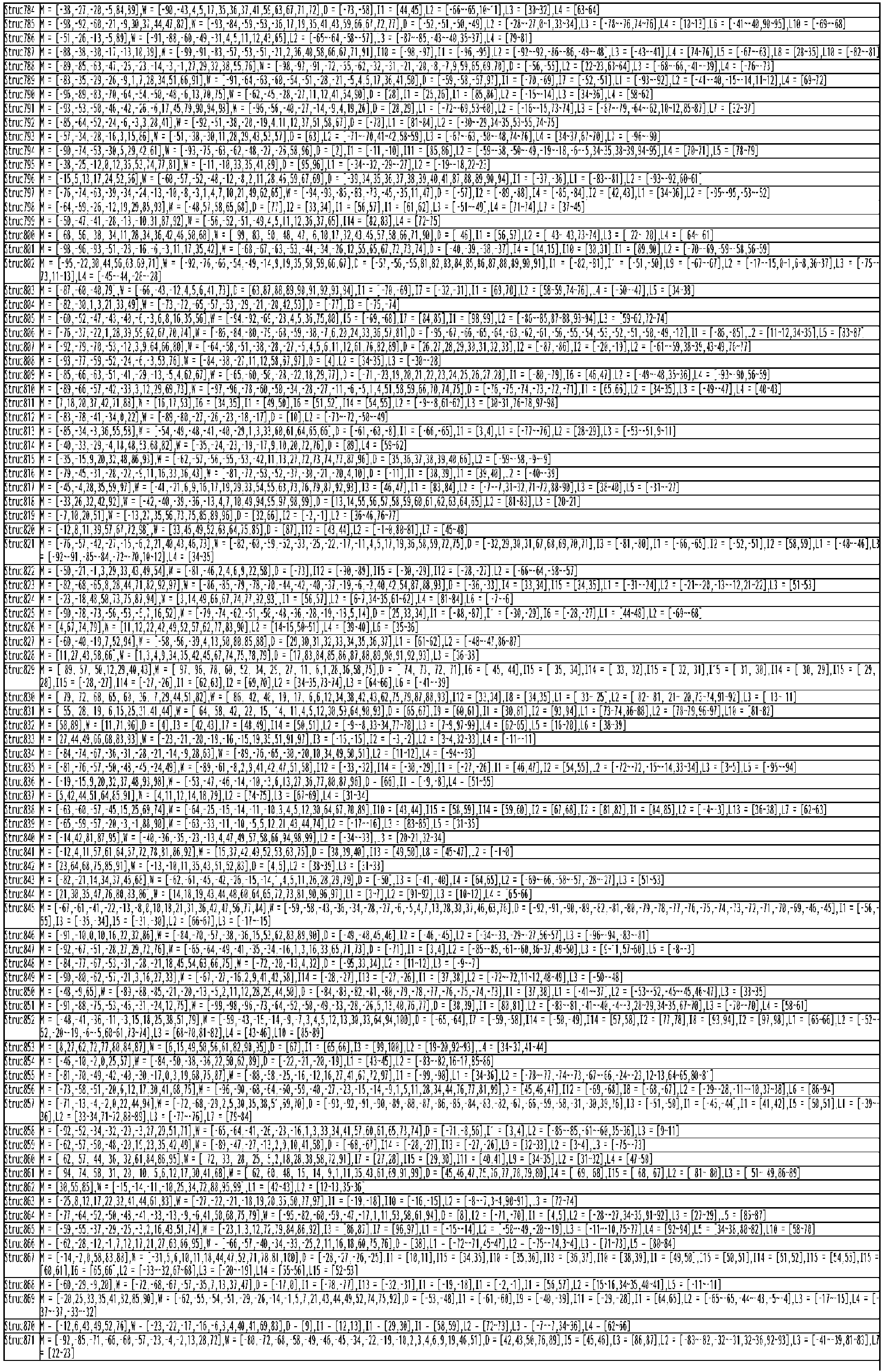

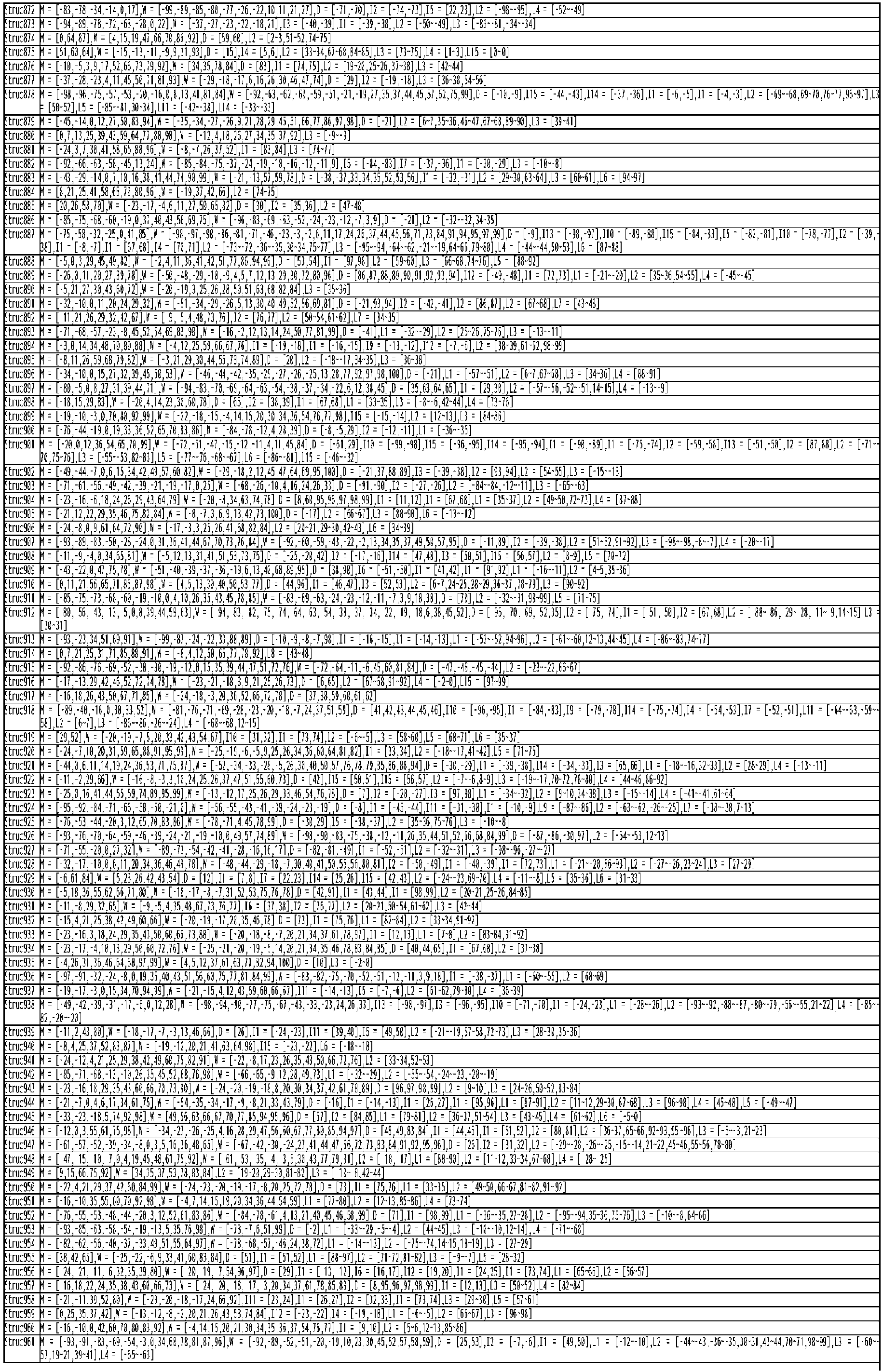

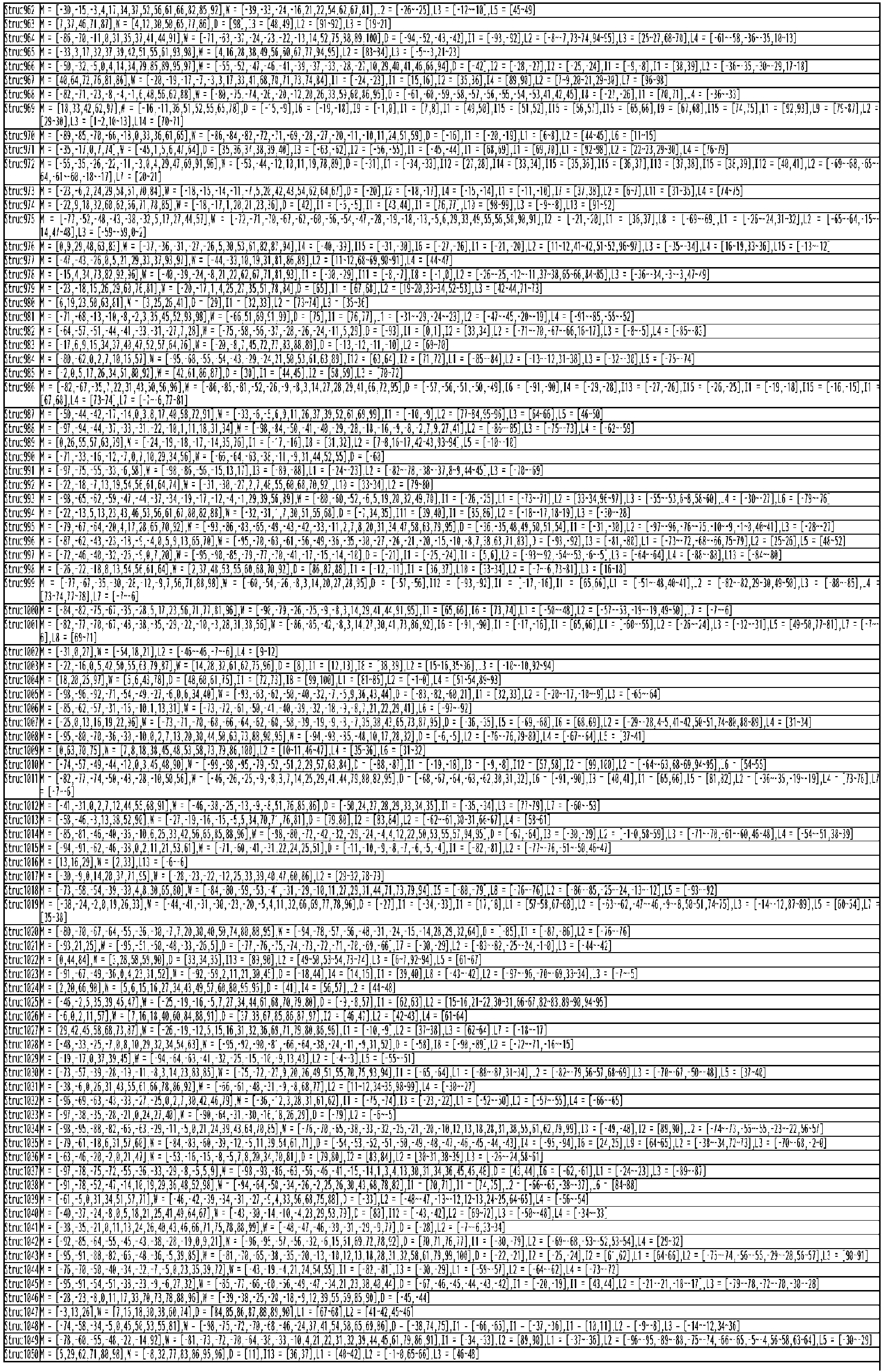

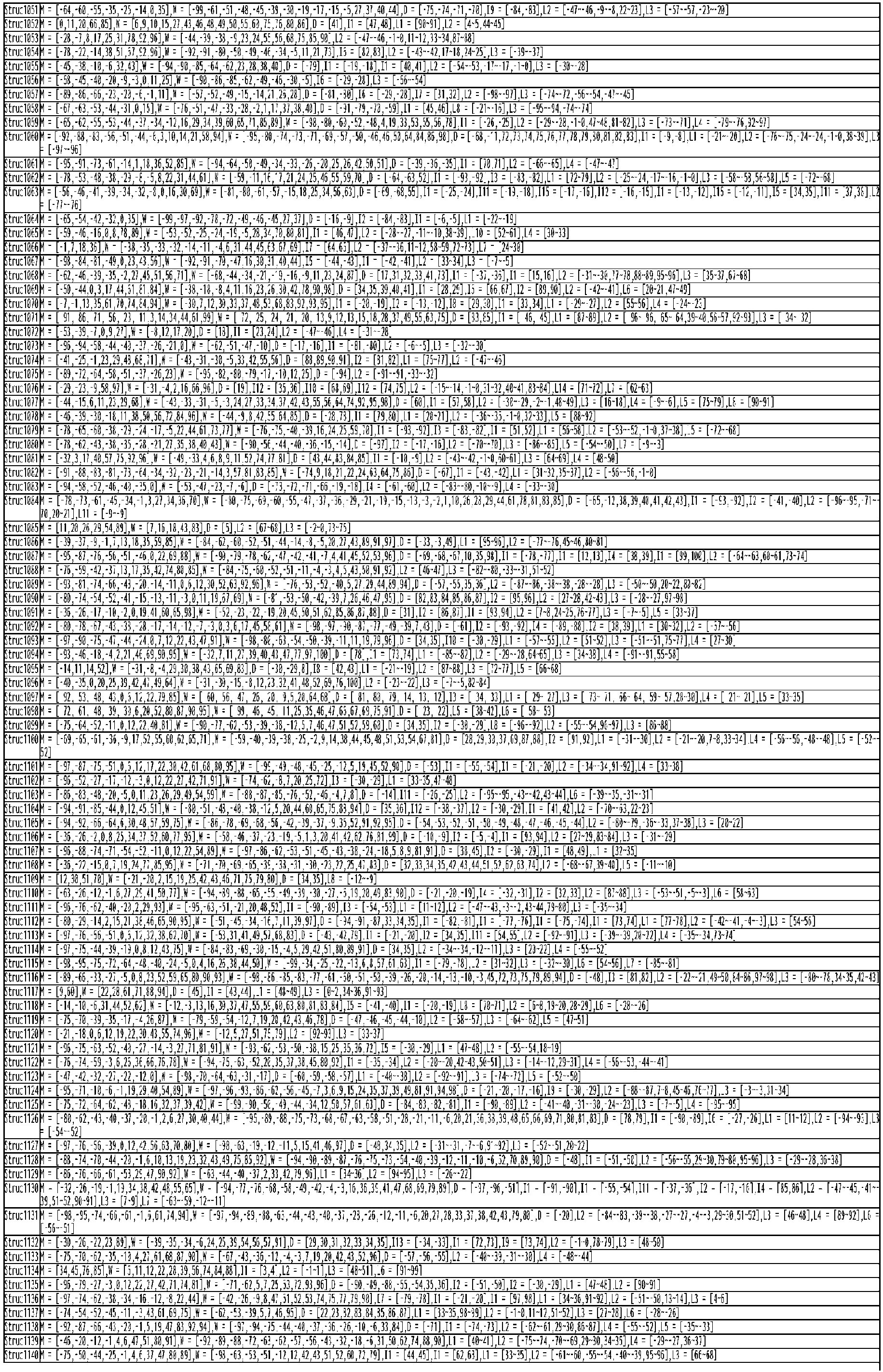

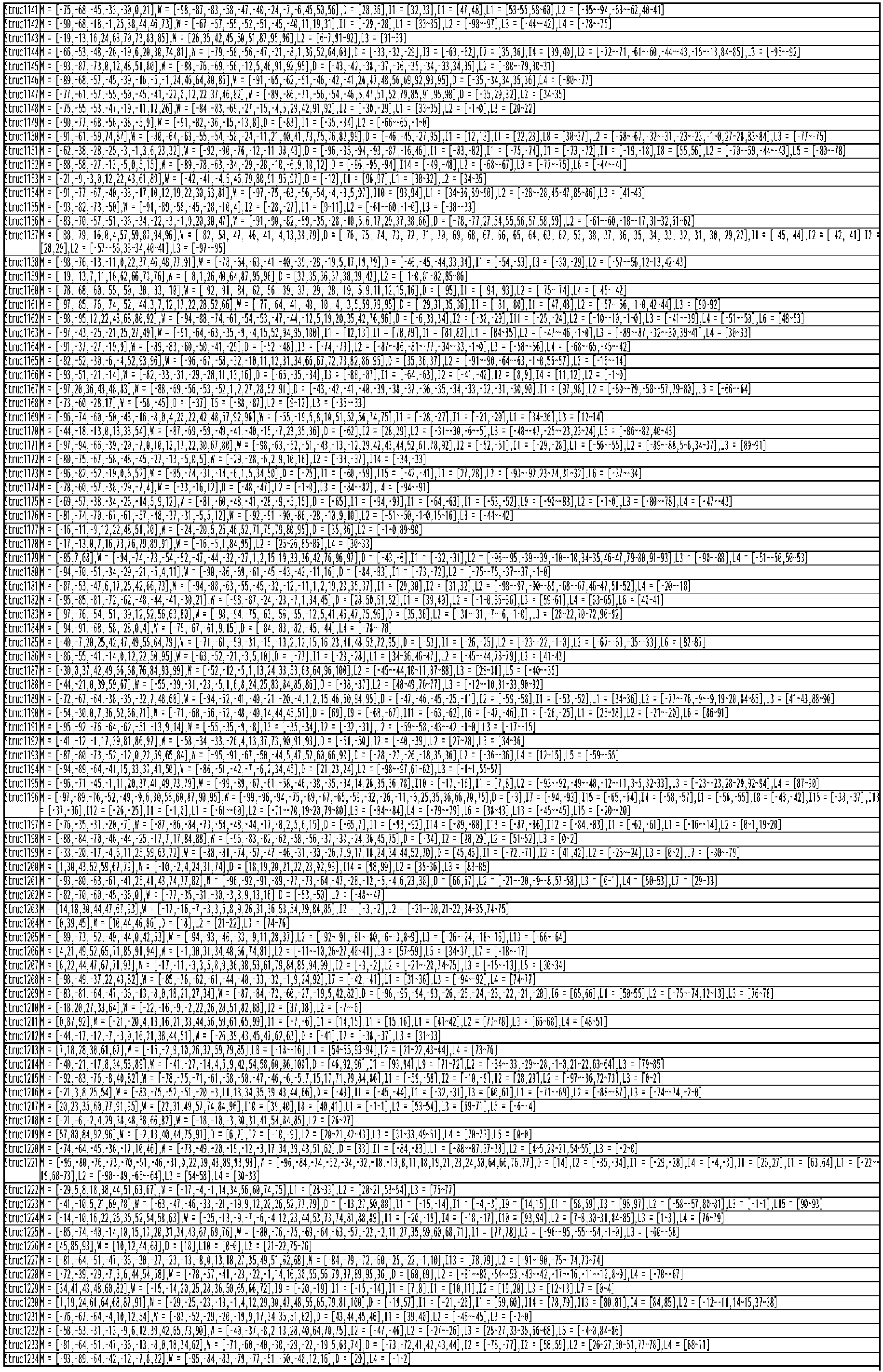

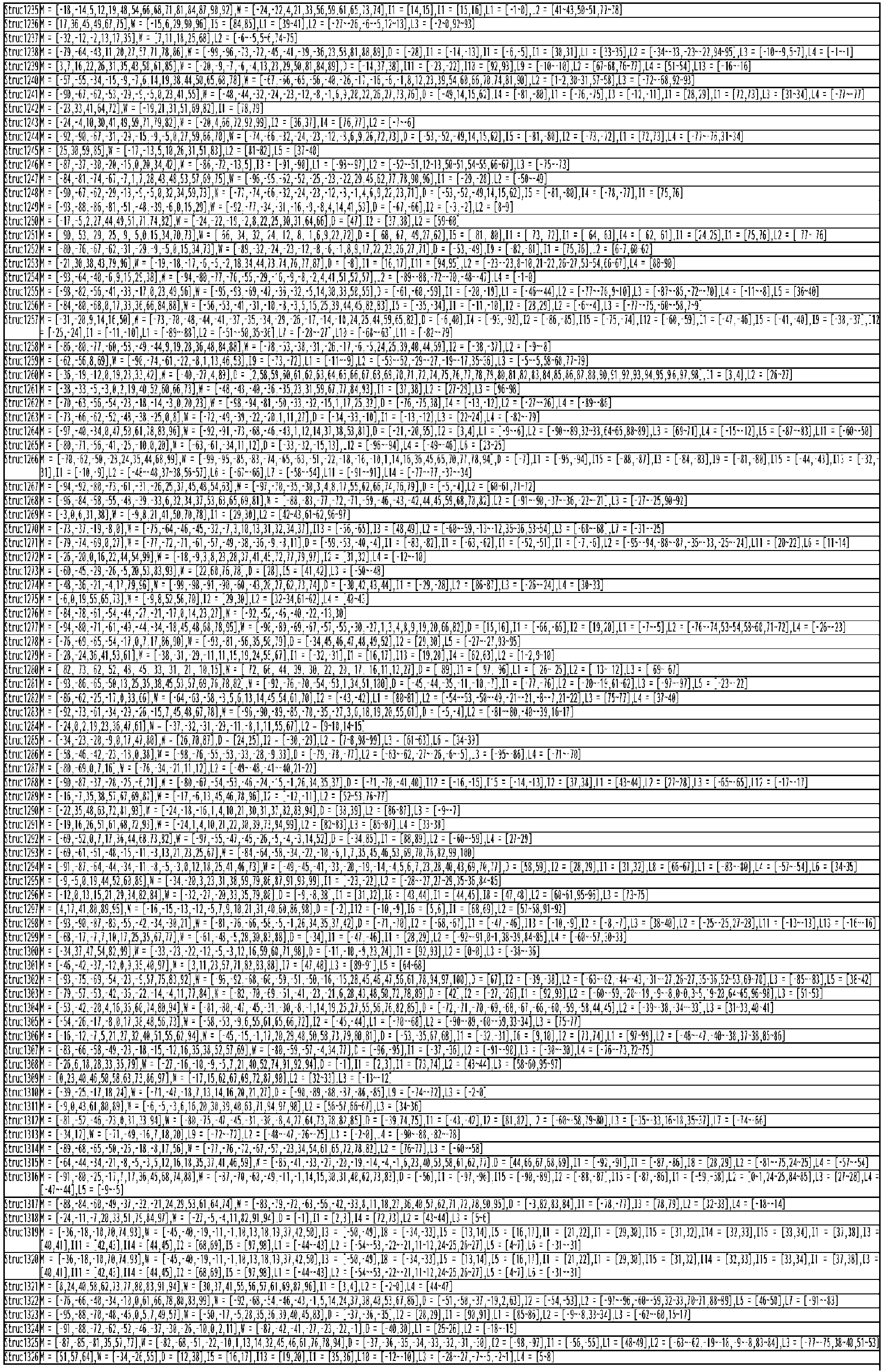

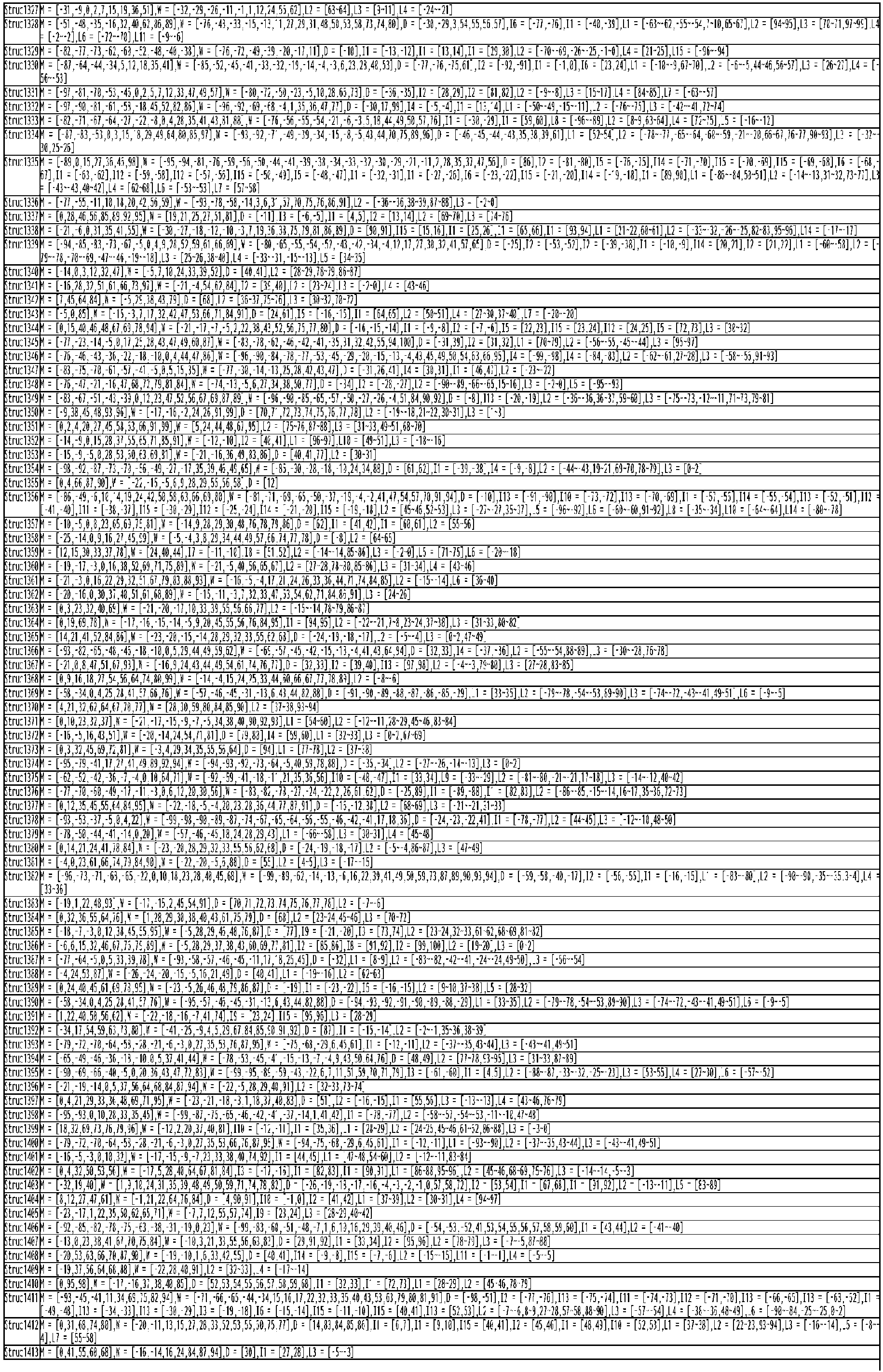

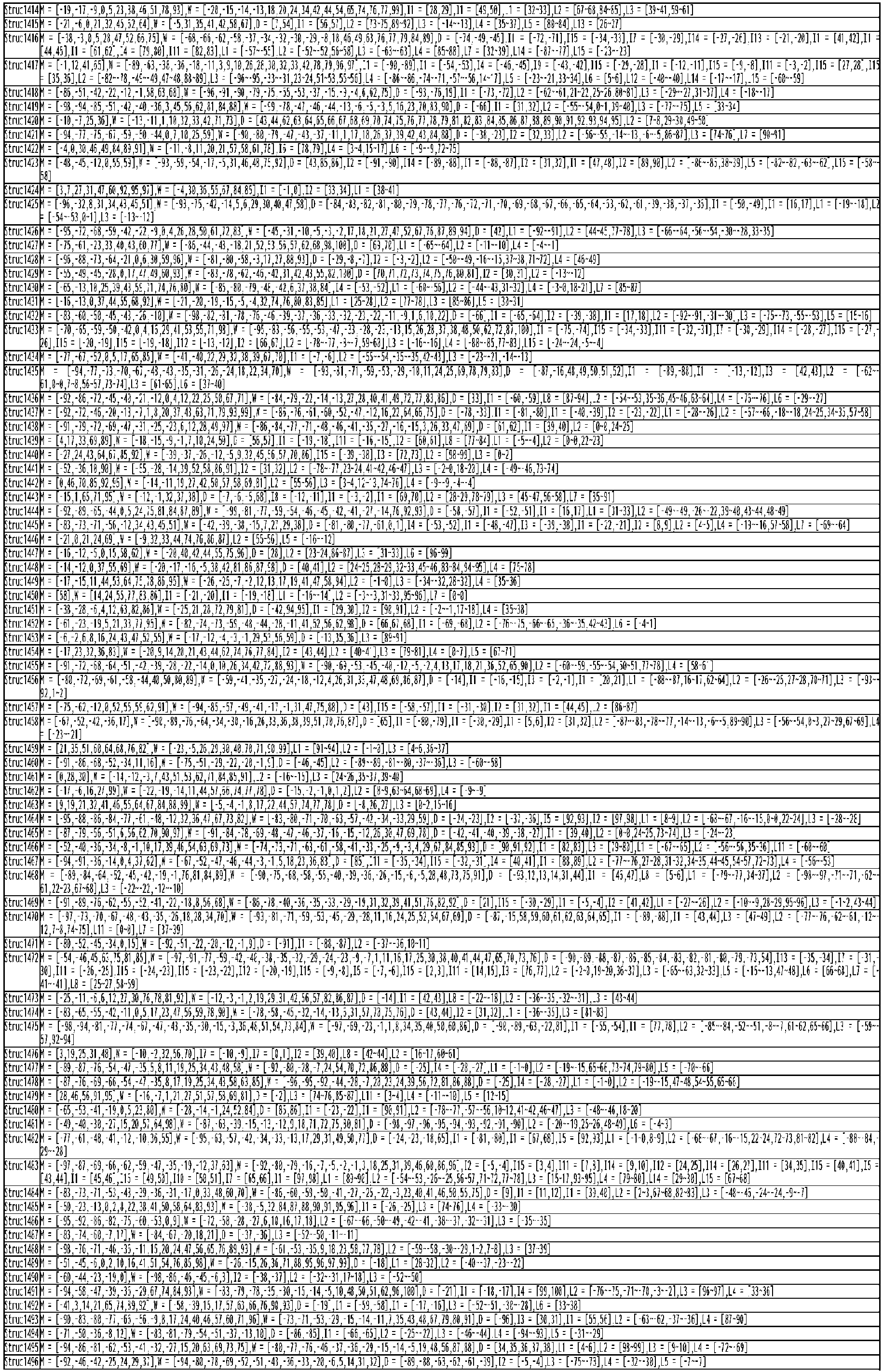

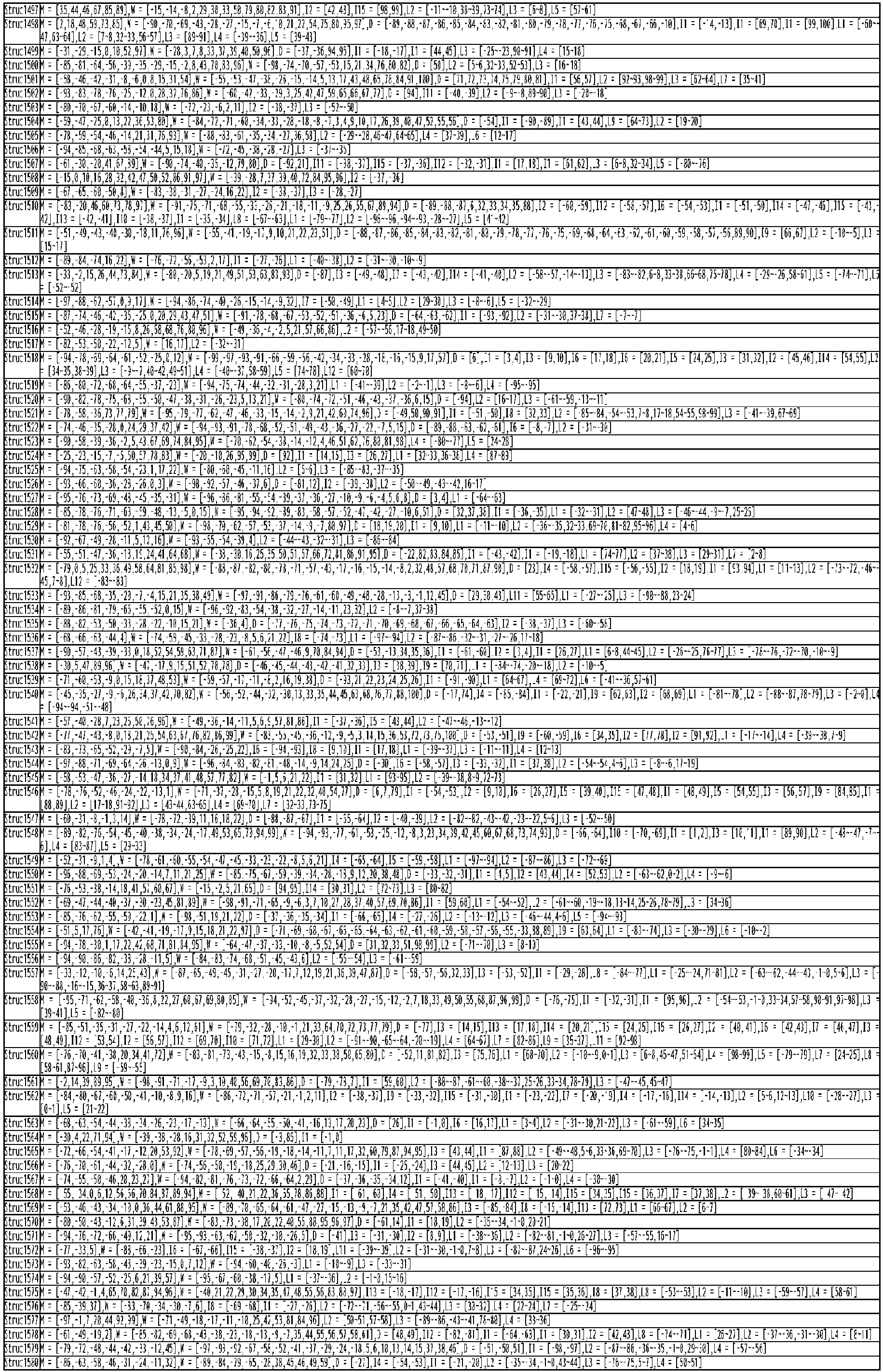

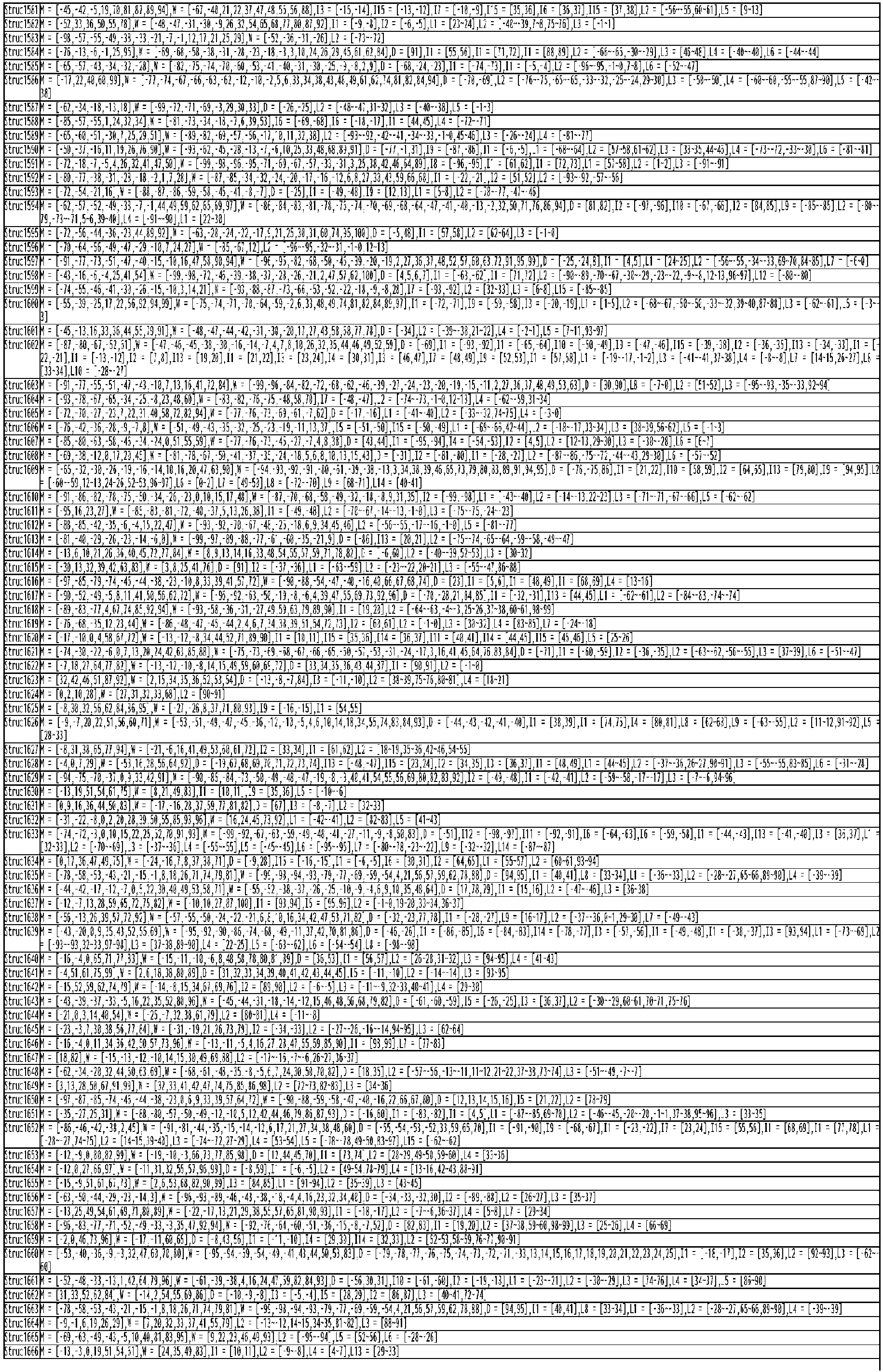

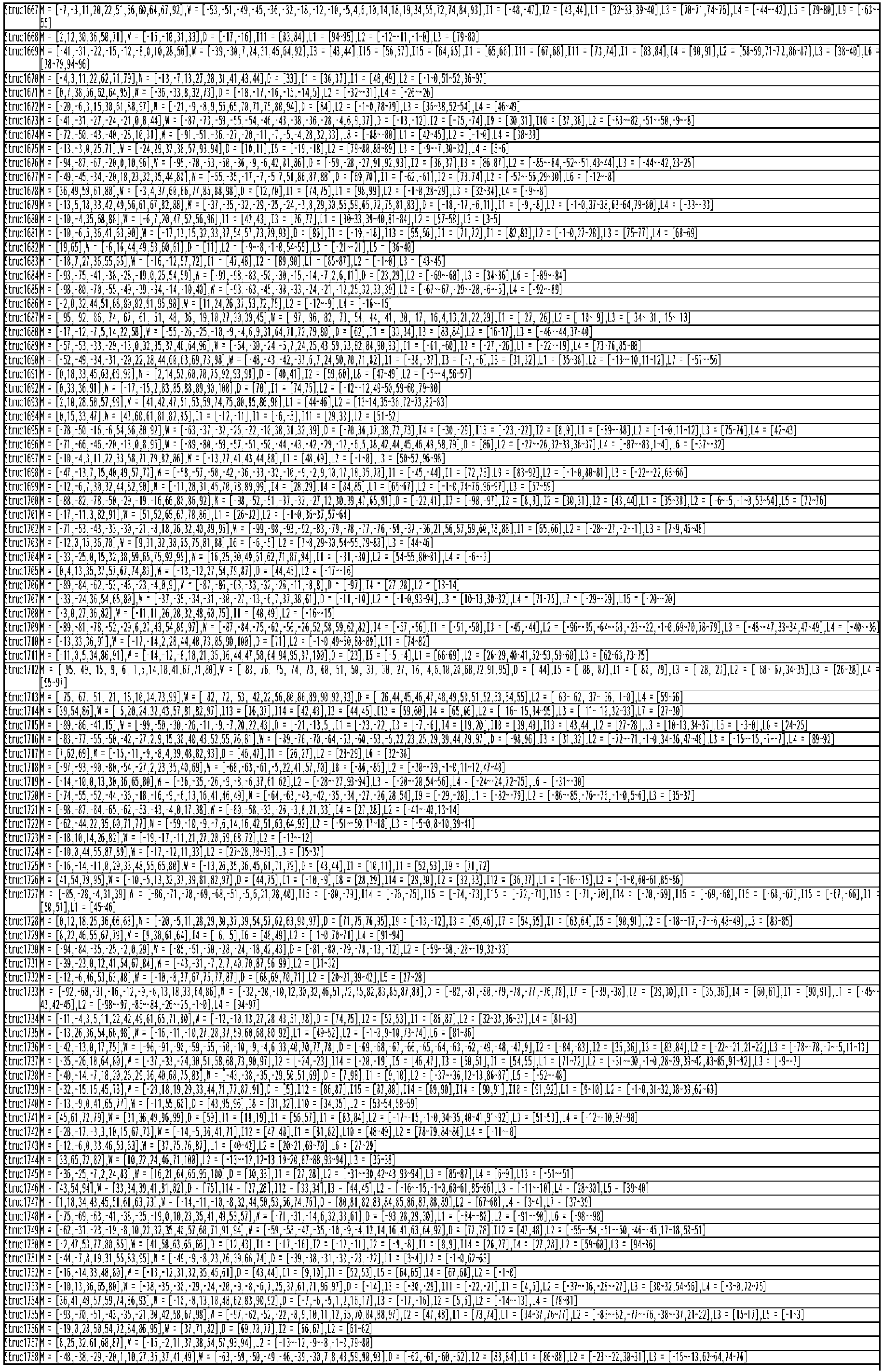

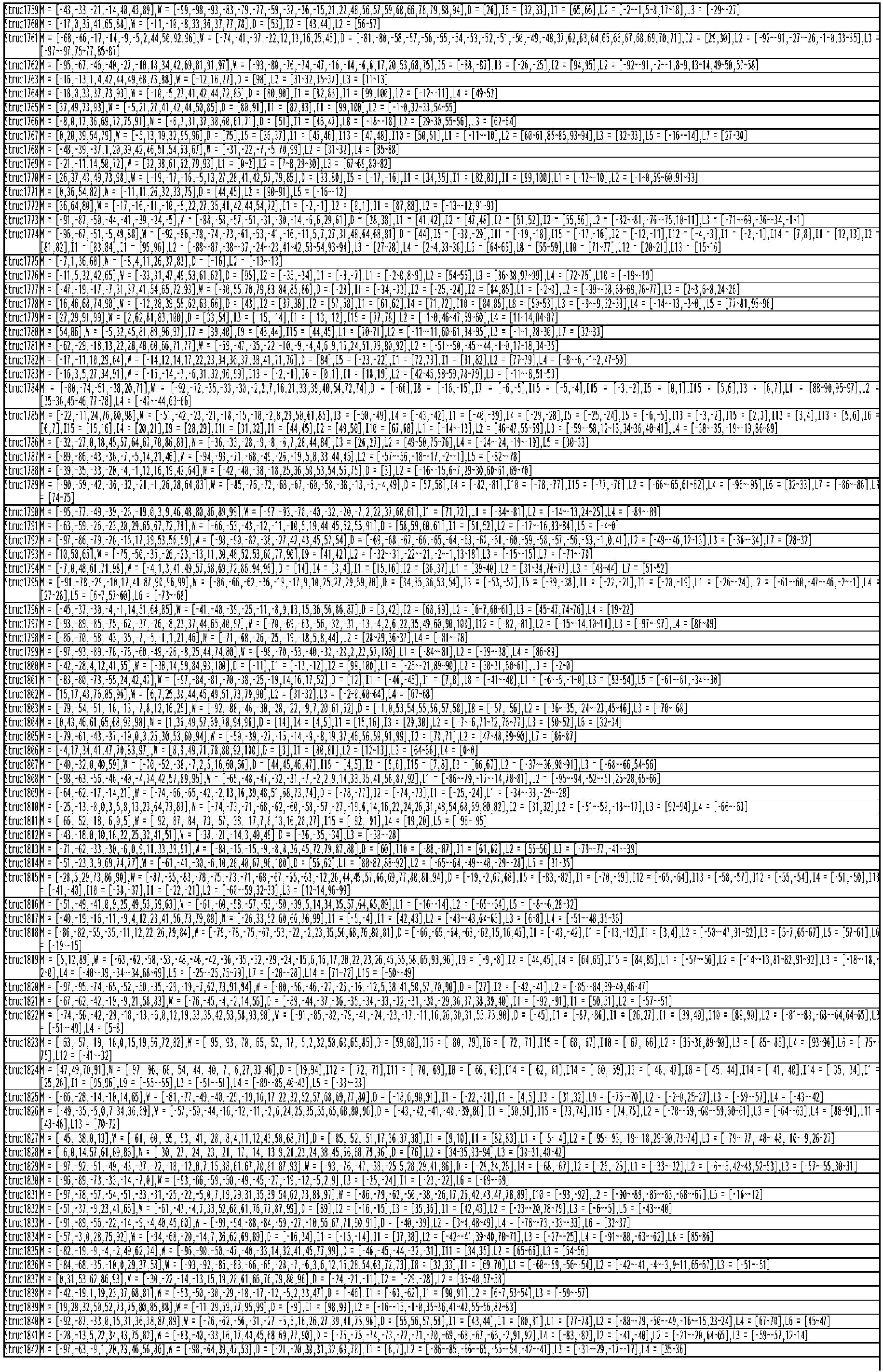

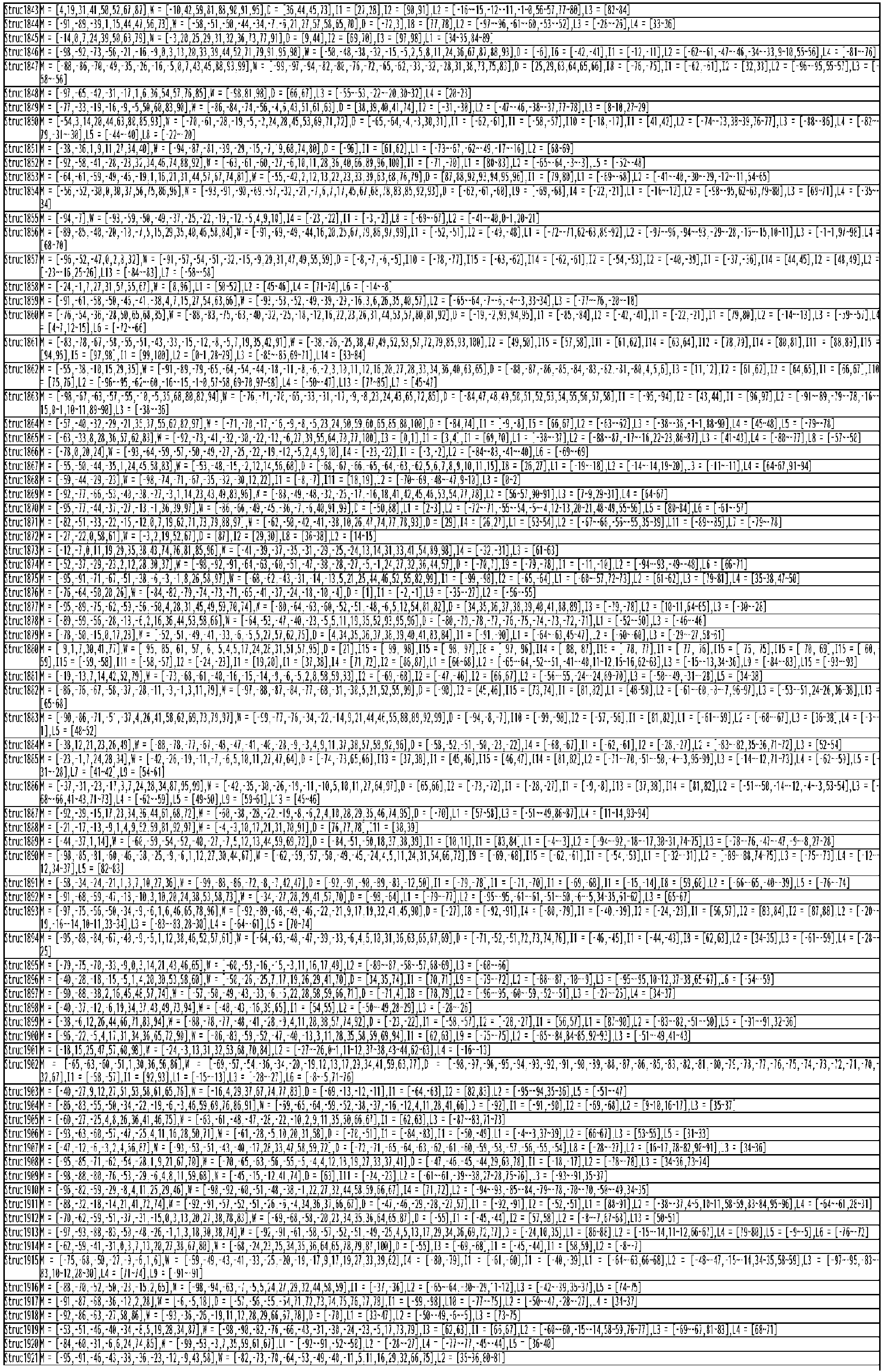

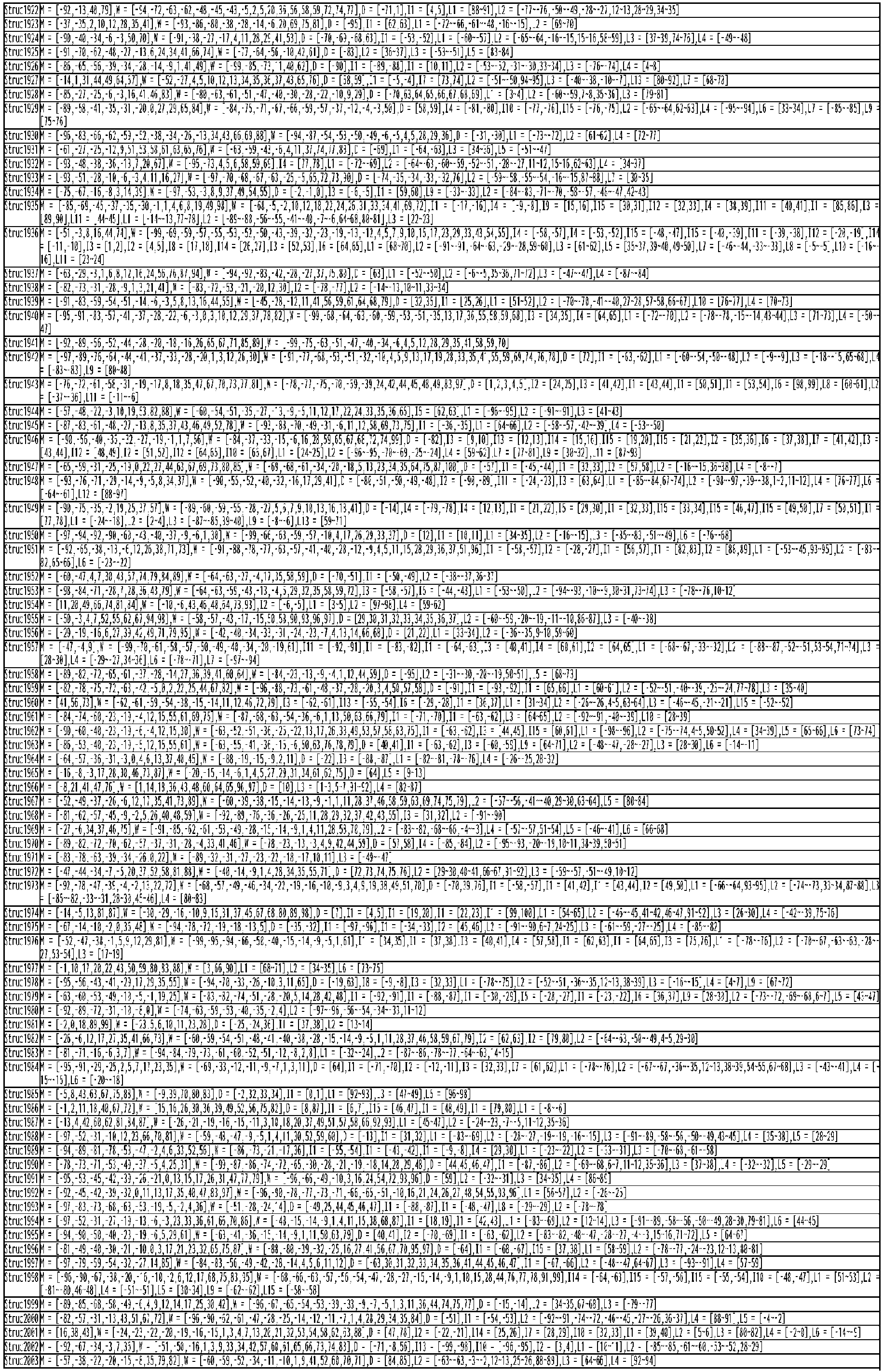

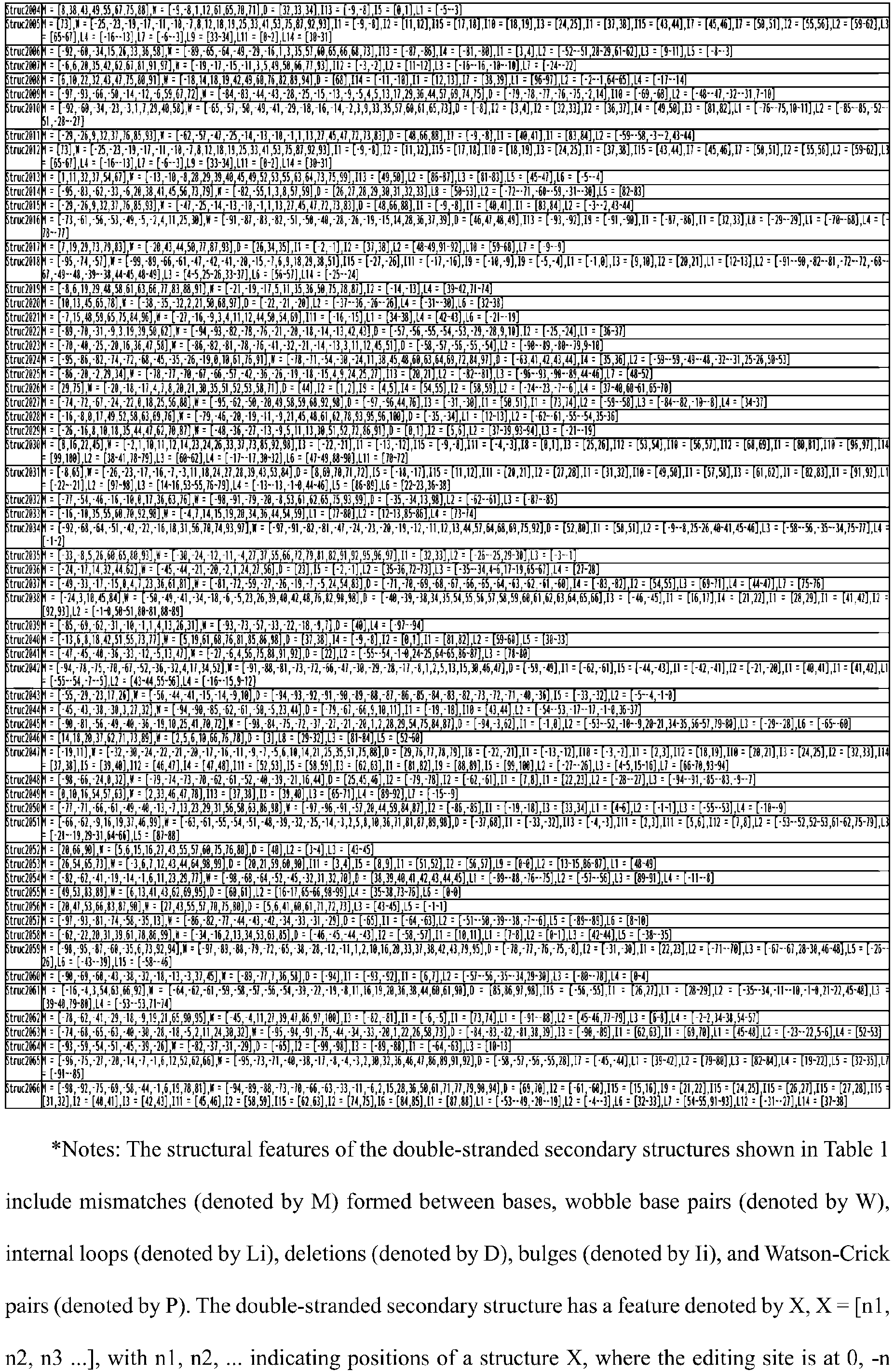

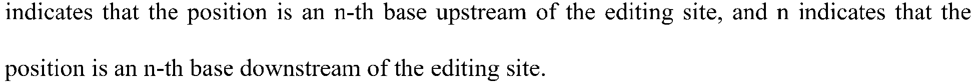

When the double-stranded secondary structure X is M, M = [n1, n2, n3 ...] indicates that mismatches are present at positions of n1, n2, n3 ... from the editing site. When the double-stranded secondary structure X is W, W = [n1, n2, n3 ...] indicates that wobble base pairs are present at positions of n1, n2, n3 ... from the editing site. When the double-stranded secondary structure X is D, D = [n1, n2, n3...] indicates that deletions are present at positions of n1, n2, n3 ... from the editing site. When X is Ii, Ii = [n1, n2], with i being any positive integer indicating that i bases are inserted between n1 and n2 from the editing site. When the double-stranded secondary structure X is Li, Li = [n1 to n2, n3 to n4 ...], with i being any positive integer indicating that internal loops having a size of i nt are formed in position ranges of n1 to n2, n3 to n4 ... from the editing site. In any double-stranded secondary structure, all positions are P except where X is one or more of M, W, D, Ii, and Li.

Table 2 is shown at the end of the specification, which is a part of the present disclosure, and is incorporated herein by reference in its entirety.

The results showed that the mcRNAs designed with different combinations of mimic domains and binding domains by simulating the secondary structures of the present disclosure were higher than structureless mcRNAs, different mimic domain lengths from 41 nt to 91 nt did not significantly affect the editing levels, and the editing levels of all the mcRNAs were high editing levels of 30% to 70%. This example demonstrated that mcRNA sequences higher than structureless ones could be selected from the 151 nt long mcRNAs designed based on the secondary structures of the present disclosure.

### Example 2. Design of 41 nt Long mcRNA by MASSA Method to Edit Different Sites of Human GAPDH Gene

This example was intended to design, using the MASSA method, a series of mcRNAs according to the features of the secondary structures of the present disclosure for different base motifs for verification of the editing effects thereof. The names of the reference secondary structures and the mcRNA sequences are shown in Table 2, and the features of the reference secondary structures are shown in Table 1. In this example, the A bases of 10 sites of GAPDH were randomly selected to verify the editing effects of the mcRNAs designed according to the features of the secondary structure. The 10 sites were as follows. The motif at position 787 of GAPDH was a UAG motif, and the mcRNAs designed for this motif were mcRNA1206 to mcRNA1871, with the secondary structures of the reference substrates and the features thereof shown in Table 1. The motif at position 1257 was UAU, and the mcRNAs designed for this motif were mcRNA3881 to mcRNA4540, with the secondary structures of the reference substrates and the features thereof shown in Table 1. The motif at position 1275 was UAC, and the mcRNAs designed for this motif were mcRNA4541 to mcRNA5200, with the secondary structures of the reference substrates and the features thereof shown in Table 1. The motif at position 1323 was a UAA motif, and the mcRNAs designed for this motif were mcRNA1 to mcRNA660, with the secondary structures of the reference substrates and the features thereof shown in Table 1. The motif at position 816 was an AAC motif, and the mcRNAs designed for this motif were mcRNA8157 to mcRNA8789, with the secondary structures of the reference substrates and the features thereof shown in Table 1. The bases at position 873 was in an AAG motif, and the mcRNAs designed for this motif were mcRNA7516 to mcRNA8156, with the secondary structures of the reference substrates and the features thereof shown in Table 1. The motif at position 860 was a CAC motif, and the mcRNAs designed for this motif were mcRNA5861 to mcRNA6520, with the secondary structures of the reference substrates and the features thereof shown in Table 1. The motif at position 971 was a CAA motif, and the mcRNAs designed for this motif were mcRNA5201 to mcRNA5860, with the secondary structures of the reference substrates and the features thereof shown in Table 1.

In this example, different editing modes were designed to further verify the editing effects of the mcRNAs designed according to the secondary structures, with the specific design shown in FIG. 4. The design mainly had three patterns. The first one was to extend, as the length of the mimic domain, 6 bases upstream and 14 bases downstream with the editing site as the center. The second one was to extend, as the length of the mimic domain, 10 bases upstream and 10 bases downstream with the editing site as the center. The third one was to extend, as the length of the mimic domain, 14 bases upstream and 6 bases downstream with the editing site as the center. In the first pattern, there was a binding domain of 20 bases downstream of the editing site. In the second pattern, there was a binding domain of 10 bases at each of two ends. In the third mode, there was a binding domain of 20 bases upstream of the editing site. In each pattern, there would be a structureless mcRNA of the same length, and there would be a base A-C mismatch at the opposite site to the editing site. The lengths of the reference designs were all 41 nt. Sequences were designed and the editing levels of the mcRNAs were verified with reference to the approaches in Example 1 (FIGs. 5 to 12).

The results showed that the combinations of the mimic domain and binding domain lengths in different patterns in this example could allow for selection of a large number of mcRNA sequences higher than structureless ones at different sites and on different motifs. Specifically, for the UAN motif shown in FIG. 5, the editing levels of the structureless mcRNA of the UAU motif in the three patterns were from 30% to 55%, and the editing levels of the structureless mcRNAs of the other three motifs were below 30%. There were 59% mcRNAs with the simulated secondary structure features designed in the UAG motif higher than one of three structureless mcRNAs, and there were 76% mcRNAs with the simulated secondary structure features designed in the UAA motif higher than one of three structureless mcRNAs. In order to further analyze the obtained mcRNA editing effects, the editing at the target site and the A bases upstream and downstream by the mcRNA at each site was compared in parallel, and a comparison on editing effect was made between the editing and off-target editing of each sequence using heat maps (as shown in FIG. 6). The results showed that there was high editing efficiency at the target site on the UAN motif, while there was only weak off-target editing upstream and downstream, indicating that the mcRNA designed with the secondary structure had high specificity and targeted editing capability. This was similar on the UAU and UAC motifs, except that on the UAA motif, the high editing mcRNA could edit one A base downstream of the editing site to a certain extent.

The CAN motif shown in FIG. 7 included the CAA motif and the CAC motif, where the editing level of the structureless mcRNA of the CAA motif was about 5%, and the editing level of the structureless mcRNA of the CAC motif was below 5%. The mcRNAs with structural characteristics designed according to the secondary structures of the present disclosure could achieve more than 20% editing at most, and a proportion of those with editing levels exceeding that of one of three structureless mcRNAs was 55% in the CAA motif and 94% in the CAC motif. The editing of the target site on the CAA motif was significantly higher than the off-target editing upstream and downstream, while there was a certain degree of off-target editing on the CAC motif (shown in FIG. 8).

The AAN motif shown in FIG. 9 included the AAG motif and the AAC motif. In the two motifs, the editing levels of the structureless mcRNAs were both about 5%. The mcRNAs with structural characteristics designed according to the secondary structures of the present disclosure could achieve up to 40% editing, and a proportion of those with editing levels exceeding that of one of three structureless mcRNAs was 95% in the AAG motif and 61% in the AAC motif. The mcRNA on the AAG motif exhibited almost no off-target editing, and there was a certain degree of off-target editing at two sites downstream of the editing site on the AAC motif (shown in FIG. 10).

The GAN motif shown in FIG. 11 included the GAG motif and the GAA motif. In the two motifs, the editing levels of the structureless mcRNAs were both below 5%, and the structureless mcRNAs almost had no editing effect in the GAA motif. The mcRNAs with structural characteristics designed according to the secondary structures of the present disclosure could achieve up to 20% editing in the GAG motif and up to 8% editing in the GAA motif, and a proportion of those with editing levels exceeding that of one of three structureless mcRNAs was 74% in the GAG motif and 94% in the GAA motif. The mcRNAs on the GAG and GAA motifs both had a certain degree of off-target editing, and the specific mcRNA target site editing on the GAA motif was low, but the off-target editing effect at the next position to the editing site was higher than that of the target site (shown in FIG. 12).

The results of this example demonstrated that the mcRNAs obtained based on the secondary structures of the present disclosure in combination with the method of designing mcRNAs could achieve high-efficiency editing on the target sites of different motifs, and the editing levels of more than 95% of structured mcRNAs at specific sites were higher than those of structureless mcRNAs.

### Example 3. Design of mcRNAs of Different Lengths by MASSA Method to Edit Different Sites of Human ATP7B Gene

This example was intended to verify that mcRNAs with high editing activity could be designed on the human ATP7B gene based on the features of the secondary structures of the present disclosure. A mutation in the ATP7B gene is the main cause of hepatolenticular degeneration. In this example, the A base at position 1532 on the ATP7B gene was selected. The C base upstream of this site was mutated to the T base, so the motif where the editing site after mutation was located was the UAG motif. Moreover, the G to A mutation site at base 3316 was also selected, and the motif of the site was the AAU motif. In this example, 31 nt and 41 nt were selected upstream and downstream of the editing site for designing the mcRNAs by the MASSA method with respect to the substrate secondary structures of the specific motifs, and the editing level of each mcRNA at the target site was measured by the high-throughput method. In this example, the mcRNA sequences designed for the UAG motif on the ATP7B gene were mcRNA2870 to mcRNA5860 (as shown in Table 2), with the reference secondary structures shown in Table 1.

The results showed that, as shown in FIG. 13, the 41 nt long mcRNA with structural features on the UAG motif could achieve up to 50% editing, while the 41 nt mcRNA without structural features achieved the editing level below 5%, and the 31 nt long mcRNA with structural features on the UAG motif exhibited the editing capability significantly lower than that at the length of 41 nt and could only achieve the editing level of about 20% at most, while the 31 nt structureless mcRNA also achieved the editing level below 5%. Compared with the structureless mcRNAs, the proportion of structured mcRNAs with higher editing levels than structureless ones was 51% for 41 nt and 17% for 31 nt. As shown in FIG. 14, the editing levels of all mcRNAs on the UAG motif of the ATP7B gene to the target sites were much higher than upstream and downstream off-target editing levels, and there was less off-target editing only upstream of the editing site.

As shown in FIG. 15, both 41 nt and 31 nt mcRNAs exhibited only weak editing capability on the AAU motif of the ATP7B gene, with the editing levels basically within 10%. Compared with the structured mcRNAs, the structureless mcRNAs exhibited almost no editing capability, and the proportion of mcRNAs with higher editing levels than structureless ones was 53% for the length of 41 nt and 14% for the length of 31 nt. As shown in FIG. 16, the A base upstream of the editing site on the AAU motif of the ATP7B gene was slightly off-target edited. This example further demonstrated that where the structureless mcRNAs could not be edited, by simulating the secondary structures of the present disclosure, about 15% editing at most could be achieved on the site difficult to edit.

The above results further demonstrated that the secondary structures of the present disclosure could be applied to edit different motifs on different genes.

### Example 4. Design of mcRNA by MASSA method to Edit Human FGFR Gene

This example was intended to verify that mcRNAs capable of efficient editing could be designed on the human FGFR gene based on the secondary structures of the present disclosure. Mutations in the FGFR gene are the causes of diseases such as achondroplasia, fatal osteodysplasia, and nanism. In this example, the site G358R of the FGFR gene having a G to A mutation was selected as the editing site, so the motif of the editing site after mutation was the CAG motif. In this example, 31 nt and 41 nt were selected upstream and downstream of the editing site for designing the mcRNAs by the MASSA method with respect to the substrate secondary structures of the specific motifs, and the editing level of each mcRNA at the target site was measured by the high-throughput method. In this example, the mcRNA sequences designed for this site were mcRNA13097 to mcRNA14086 (as shown in Table 2), with the reference secondary structures shown in Table 2.

The results showed that, as shown in FIG. 17, the editing levels of both 41 nt and 31 nt structureless mcRNAs on the CAG motif of the FGFR gene were only about 2%, and the mcRNAs designed based on the secondary structures of the present disclosure could achieve up to 35% editing at the length of 41 nt, while the 31 nt mcRNAs could achieve 10% editing. In all mcRNAs, there was significant off-target editing at two sites upstream of the editing site, but there was no off-target editing at all downstream of the editing site. Overall comparison showed that the editing levels of most mcRNA sequences designed based on the 41 nt target sequence were below 20%, the editing levels of almost all mcRNAs designed based on the 31 nt target sequence were below 10%, and among the 41 nt mcRNAs, the proportion of those with the editing levels higher than that of the structureless mcRNAs was about 50%. As shown in FIG. 18, the mcRNA of this CAG motif of FGFR exhibited completely no off-target editing downstream of the editing site, but exhibited weak off-target editing upstream of the editing site. This example once again demonstrated that mcRNAs capable of efficient editing could be designed on different genes and on different motifs based on the secondary structures of the present disclosure.

### Example 5. Design of mcRNA by MASSA method to Edit Human APC Gene

This example was intended to verify that mcRNAs capable of efficient editing could be designed on the human APC gene based on the secondary structures of the present disclosure. The APC gene is a tumor suppressor gene, and in some cases, the function of the protein will be lost due to mutations. For example, the G base at site 2627 of the gene is mutated to the T base, which causes this position and following two bases to produce a stop codon, thus affecting the translation of the protein. In this example, the A base next to this site was selected for editing, and the motif was GAG. In addition, a mutation from the C base to the T base is often produced at position 4099 of the gene, which also produces a stop codon. Next A base to this site was selected as the second editing site, and the motif was UAG. Another high-frequency mutation site is position 4348 where the C base is mutated to the T base, which also produces a stop codon. The last two A bases of this site were selected as the editing site, and the motif was GAG. In this example, 31 nt and 41 nt were selected upstream and downstream of the editing site for designing the mcRNAs by the MASSA method with respect to the substrate secondary structures of the specific motifs, and the editing level of each mcRNA at the target site was measured by the high-throughput method. The mcRNA sequences designed for the first editing site were mcRNA10110 to mcRNA11094 (as shown in Table 2), with the structures of the corresponding reference secondary structures shown in Table 1. The mcRNA sequences designed for the second editing site were mcRNA1872 to mcRNA2869 (as shown in Table 2), with the structures of the reference secondary structures shown in Table 1. The mcRNA sequences designed for the third editing site were mcRNA11095 to mcRNA12090 (as shown in Table 2), with the structures of the reference secondary structure shown in Table 1.

The results showed that, as shown in FIGs. 19 to 24, the editing levels of both 41 nt and 31 nt structureless mcRNAs on the UAG motif of the APC gene were only about 10%, and the mcRNAs designed based on the secondary structures of the present disclosure could achieve approximate 80% editing at most at the length of 41 nt, while the 31 nt mcRNAs could achieve 60% editing at most (FIG. 21). Different from this site, the editing levels of the other two GAG sites were lower than that of the UAG motif as a whole, with the overall editing of the 41 nt mcRNAs at the first site within 30% and the that of 31 n mcRNAs within 10%, and both 41 nt and 31 nt structureless mcRNAs at this site exhibited almost no editing (FIG. 19). The overall editing of the third editing site was close to that of the first editing site (FIG. 23). At the three sites, the mcRNAs capable of high editing for the target sites exhibited low off-target effects, where the off-target effect was lowest for the UAG motif, and at two sites of the GAG motif, few mcRNAs exhibited off-target editing upstream and downstream (as shown in FIGs. 20, 22, and 24). Among the three editing sites on the APC gene, the proportion of the 41 nt mcRNAs designed for the first GAG motif with higher editing levels than structureless mcRNAs was 36% in the total mcRNAs, and the proportion of the 31 nt mcRNAs was relatively low, only 8%. The proportion of the 41 nt mcRNAs designed for the second UAG motif with higher editing levels than structureless mcRNAs was 81% in the total mcRNAs, and the proportion of the 31 nt mcRNAs was 63%. The proportion of the 41 nt mcRNAs designed for the third GAG motif with higher editing levels than structureless mcRNAs was 61% in the total mcRNAs, and the proportion of the 31 nt mcRNAs was 29%. This example once again demonstrated that mcRNAs capable of efficient editing could be designed on different genes and on different motifs based on the secondary structures of the present disclosure.

### Example 6. Design of mcRNA by MASSA method to Edit Human TP53 Gene

This example was intended to verify that mcRNAs capable of efficient editing could be designed on the human TP53 gene based on the secondary structures of the present disclosure. The TP53 gene is a tumor suppressor gene, and in some cases, the function of the protein will be lost due to mutations. For example, the G base at site 524 of the gene is mutated to the A base such that the protein coding sequence is changed. In this example, this site was selected for editing, and the motif was CAC. In addition, a mutation from the G base to the A base is often produced at position 743 of this gene. This site was selected as the second editing site, and the motif was CAG. Another high-frequency mutation site is position 818 where the G base is mutated to the A base. This site was selected as the editing site, and the motif was CAU. In this example, 31 nt and 41 nt were selected upstream and downstream of these three editing sites for designing the mcRNAs by the MASSA method with respect to the substrate secondary structures of the specific motifs, and the editing level of each mcRNA at the target site was measured by the high-throughput method. The mcRNA sequences designed for the first editing site were mcRNA6521 to mcRNA7515 (as shown in Table 2), with the structures of the corresponding reference secondary structures shown in Table 1. The mcRNA sequences designed for the second editing site were mcRNA14087 to mcRNA15086 (as shown in Table 2), with the structures of the reference secondary structures shown in Table 1. The mcRNA sequences designed for the third editing site were mcRNA15087 to mcRNA16083 (as shown in Table 2), with the structures of the reference secondary structure shown in Table 1.

The results showed that, as shown in FIGs. 25 to 30, the editing motiy on the TP53 gene was CAN, where the CAG motif and the CAU motif exhibited higher overall editing effects than the CAC motif, the editing levels of both 41 nt and 31 nt structureless mcRNAs on the CAC motif were only about 2%, and the mcRNAs designed based on the secondary structures of the present disclosure could achieve approximate 35% editing at most at the length of 41 nt, while the 31 nt mcRNAs could achieve 10% editing at most (FIG. 25). On the CAG motif, the structureless mcRNAs exhibited only less than 10% editing under different length conditions, while the mcRNAs designed according to the secondary structures could achieve up to 70% editing at the length of 41 nt and up to 20% editing at the length of 31 nt (FIG. 27). On the CAU motif, the structureless mcRNAs exhibited almost no editing, while the mcRNAs designed according to the secondary structures could achieve up to 60% editing at the length of 41 nt and up to 30% editing at the length of 31 nt (FIG. 29). At the three sites, the mcRNAs capable of high editing for the target sites exhibited low off-target effects, where the off-target effect was lowest for the CAG motif, and on the CAG motif and the CAU motif, few mcRNAs exhibited off-target editing upstream (FIGs. 26, 28, and 30). Among the three editing sites on the TP53 gene, the proportion of the 41 nt mcRNAs designed for the first CAC motif with higher editing levels than structureless mcRNAs was 40% in the total mcRNAs, and the proportion of the 31 nt mcRNAs was relatively low, only 10%. The proportion of the 41 nt mcRNAs designed for the second CAG motif with higher editing levels than structureless mcRNAs was 57% in the total mcRNAs, and the proportion of the 31 nt mcRNAs was 10%. The proportion of the 41 nt mcRNAs designed for the third CAU motif with higher editing levels than structureless mcRNAs was 57% in the total mcRNAs, and the proportion of the 31 nt mcRNAs was 13%. This example once again demonstrated that mcRNAs capable of efficient editing could be designed on different genes and on different motifs based on the secondary structures of the present disclosure.

Based on the above examples, it was verified that the mcRNAs with high editing levels could be designed on different genes from different secondary structures. From the comparison data, it could be seen that the mcRNAs with high editing levels could be designed on different genes from the same secondary structure. For example, the site based on the UAG motif includes the GAPDH, APC, and ATP7B genes. As shown in Table 3, it was further demonstrated that the mcRNAs with high editing levels for any target site could be designed from the secondary structures of the present disclosure.

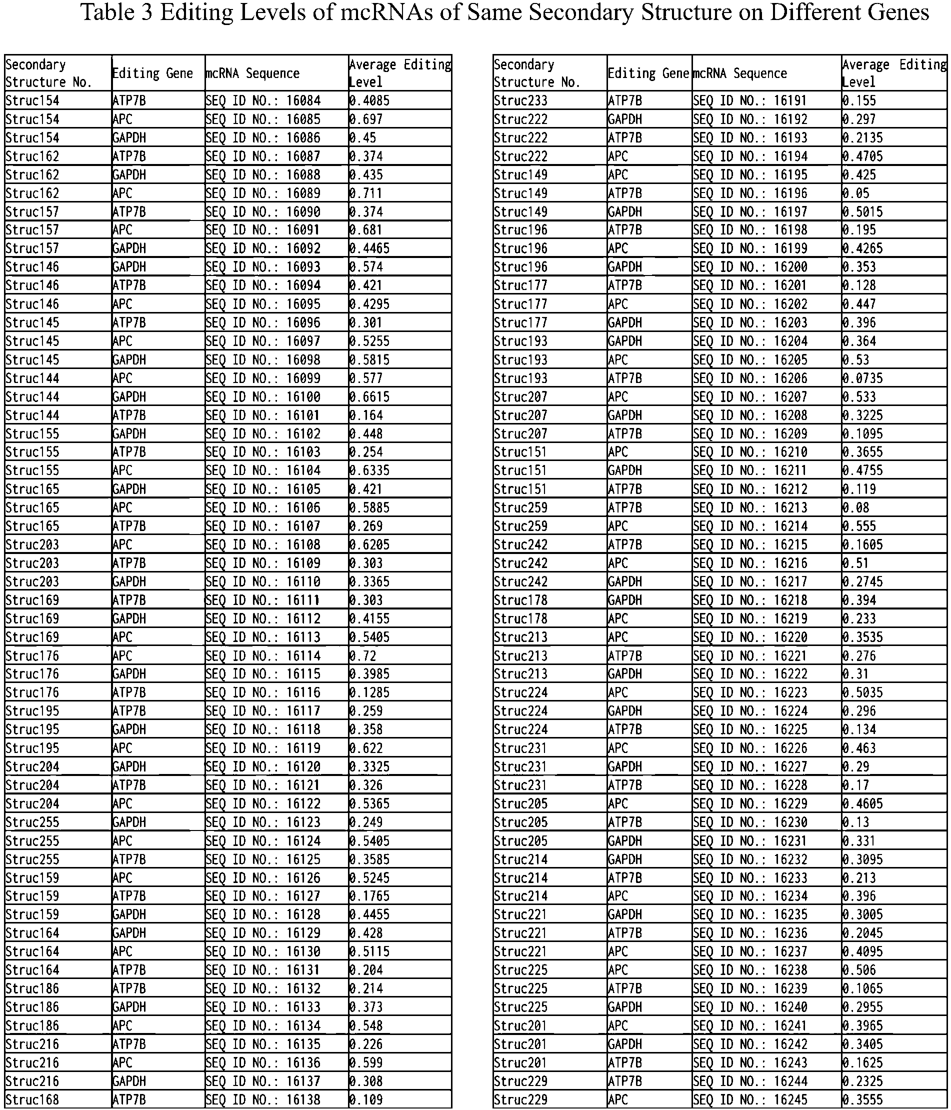

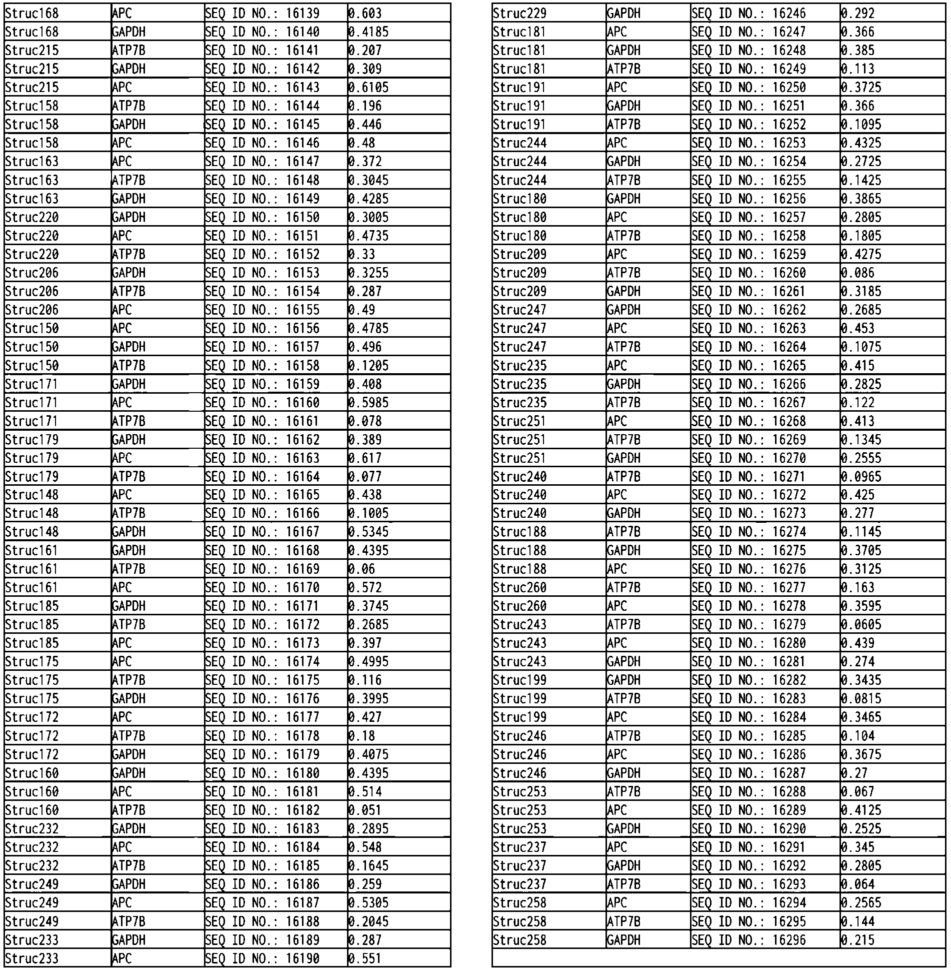

### Example 5. Verification of mcRNA Editing Effect Obtained by High-Throughput Sequencing in HEK293 Cells

The measurement of the editing levels of the mcRNAs designed based on the secondary structures of the present disclosure to the target site was completed by high-throughput sequencing. In order to further confirm the authenticity of the results of mcRNAs with different editing levels obtained by high-throughput sequencing, verification was performed on a single one of the mcRNA sequences measured on the UAG motif of the GAPDH gene in Example 2 in this example. In this example, mcRNA sequences with editing levels ranging from 3.93% to 21.66% were selected through high-throughput screening. The sequence information was GAPDH-UAG-S1 to GAPDH-UAG-S13 as shown in Table 4, and the corresponding chemically modified mcRNA sequences were GAPDH-UAG-M1 to GAPDH-UAG-M13. For the sequences selected by high-throughput screening, a single chemically modified mcRNA was transfected into cells to verify the editing levels (as shown in FIGs. 31 and 32). The stability of the modified mcRNAs was higher than that of the plasmid expressed mcRNAs, so the editing levels were higher than those selected by high-throughput screening. The chemical mcRNA modification strategy was to use phosphorothioate modification between bases and add 3 LNA modifications at each end. By analyzing the editing level selected by high-throughput screening and the editing level of a single modified mcRNA, it could be seen that they showed a strong correlation, with a correlation coefficient of up to 0.83 (as shown in FIG. 31), which further demonstrated the accuracy of the method of the present disclosure.

The editing levels of GAPDH-UAG-S1 to GAPDH-UAG-S12 in Table 4 were editing levels measured with a high-throughput hybrid system. The editing levels of GAPDH-UAG-M1 to GAPDH-UAG-M12 were the editing levels measured with a single RNA system.

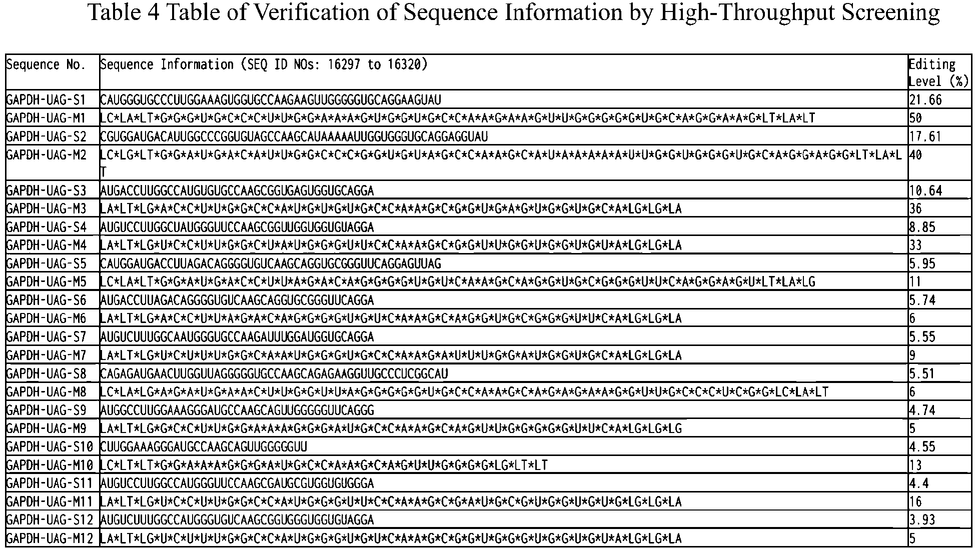

### Example 6. Design of mcRNA by MASSA method to Edit Murine GAPDH Gene

In order to verify the applicability of the present disclosure to different species, in this example, mcRNAs with five lengths of 31 nt, 41 nt, 51 nt, 61 nt, and 71 nt were designed by the MASSA method for the site 1222 of the murine GAPDH gene, and verification was performed on a single mcRNA. The specific combinations of binding domain lengths and mimic domain lengths were as shown in FIG. 33. Two 31 nt mcRNAs were selected according to the secondary structure simulation, where the editing site was at its 3' end, and the binding domains at both ends each had 5 bases. Six 41 nt mcRNAs were selected, where the editing site was in the center, and the binding domains at both ends each had 10 bases. Four 51 nt mcRNAs were selected, where the editing site was in the center, and the binding domains at both ends each had 10 bases. Four 61 nt mcRNAs were selected, where the editing site was in the center, and the binding domains at both ends each had 10 bases. Three 71 nt mcRNAs were selected, where the editing site was in the center, and the binding domains at both ends each had 10 bases. The names of the mcRNA reference secondary structures, the mcRNA sequences, and the modified sequences as mentioned above are shown in Table 5. This example was intended to verify the editing of the mcRNAs designed by the MASSA method in mouse cells, and compare the editing levels of the mcRNAs of different lengths. The method included the following steps.

### 1. Preparation of mcRNA Reference Substrate Structure

According to the mcRNA needs, the upstream and downstream lengths of the editing site were simulated to correspond to the reference substrate structures folded by RNAhybrid software, and the corresponding length range was taken. The sequence structure information within this range was reference secondary structure data. The strand where the editing site of the reference substrate was located was the editing strand of the reference substrate, and the other strand of the substrate was the complementary strand of the reference substrate.

### 2. Construction of mcRNA Sequence Complementarily Paired with Target Editing Site

Upstream and downstream mimic domains of corresponding lengths were selected with murine GAPDH site 1222 as the center. For example, the 31 nt mcRNA containing 5 nt binding domains was a complementarily paired sequence in which the editing site was extended 1 nt downstream and 19 nt upstream of position 25, where the strand where the site to be edited was located as the target editing strand, and the strand where the mcRNA was located was the mcRNA strand. For the 41 nt mcRNA containing 10 nt binding domains, the editing site was in the center and was extended 10 nt upstream and downstream.

### 3. Structural Design of Mimic Domain of mcRNA Sequence

The base pairs of each target editing strand and each mcRNA sequence strand were aligned with those of the editing strand of the reference substrate and its complementary strand one by one. When the bases of the two editing strands were the same, the mcRNA strand sequence was directly set as the complementary strand sequence of the reference substrate. When the bases of the two were different, they were designed to be the same structure. For example, there was a mismatch of one base at a certain site, and the base at the corresponding position of the mcRNA sequence strand was designed as a mismatch. The sequence design of the whole mimic domain was completed according to such a principle.

### 4. Structural Design of Binding Domain of mcRNA Sequence

On the basis of step 3, the complementarily paired sequences were directly extended at both ends of the mcRNA to complete the sequence design of the entire mcRNA, where the length of the binding domain could be customized as needed. In this example, a binding domain of 5 nt was designed at each end of the 31 nt sequence, and a binding domain of 10 nt was designed at each end of each of the 41 nt, 51 nt, 61 nt, and 71 nt sequences, as shown in FIG. 33.

### 5. Selection and Verification of mcRNA Sequence

The RNAfold software was used to calculate the minimum free energy of the pairing region of the mcRNA and target editing sequence, and the sequence information was exported, thereby completing the design of the mcRNA sequence library, and mcRNAs of different lengths were randomly selected to verify a single editing effect.

### 6. Selection and Verification of mcRNA Sequence

A plurality of mcRNAs of different lengths were randomly selected from the sequence library of step 5 for verification. In this example, the effects of different lengths and relative positions of the editing site on the mcRNAs on the editing levels were predominantly tested. The original mcRNA sequences and the modified mcRNA sequences are shown in Table 5. The synthesized mcRNA was transfected into mouse primary hepatocytes according to a transfection amount of 40 pmol per well of a 12-well plate. After 48 hours, the total RNAs were extracted and subsequently reverse-transcribed using random primers. Finally, the upstream and downstream sequences of the target site were amplified by PCR and subjected to Sanger sequencing.

As shown in FIGs. 34, 35, and 36, the results showed that the mcRNAs of different lengths for the murine GAPDH site 1222 could edit this site at high levels, and the mcRNAs of the five lengths could reach the editing levels of more than 90%.

The above results demonstrated that the mcRNAs designed by the MASSA method could edit specific sites of specific genes in mouse primary hepatocytes, and exhibited different editing effects with different combinations of mimic domain and binding domain lengths.

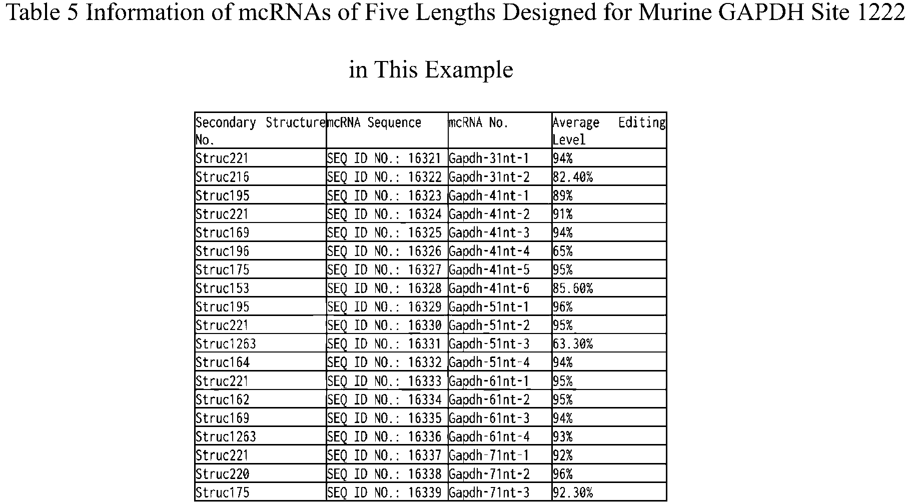

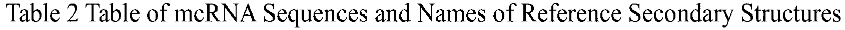

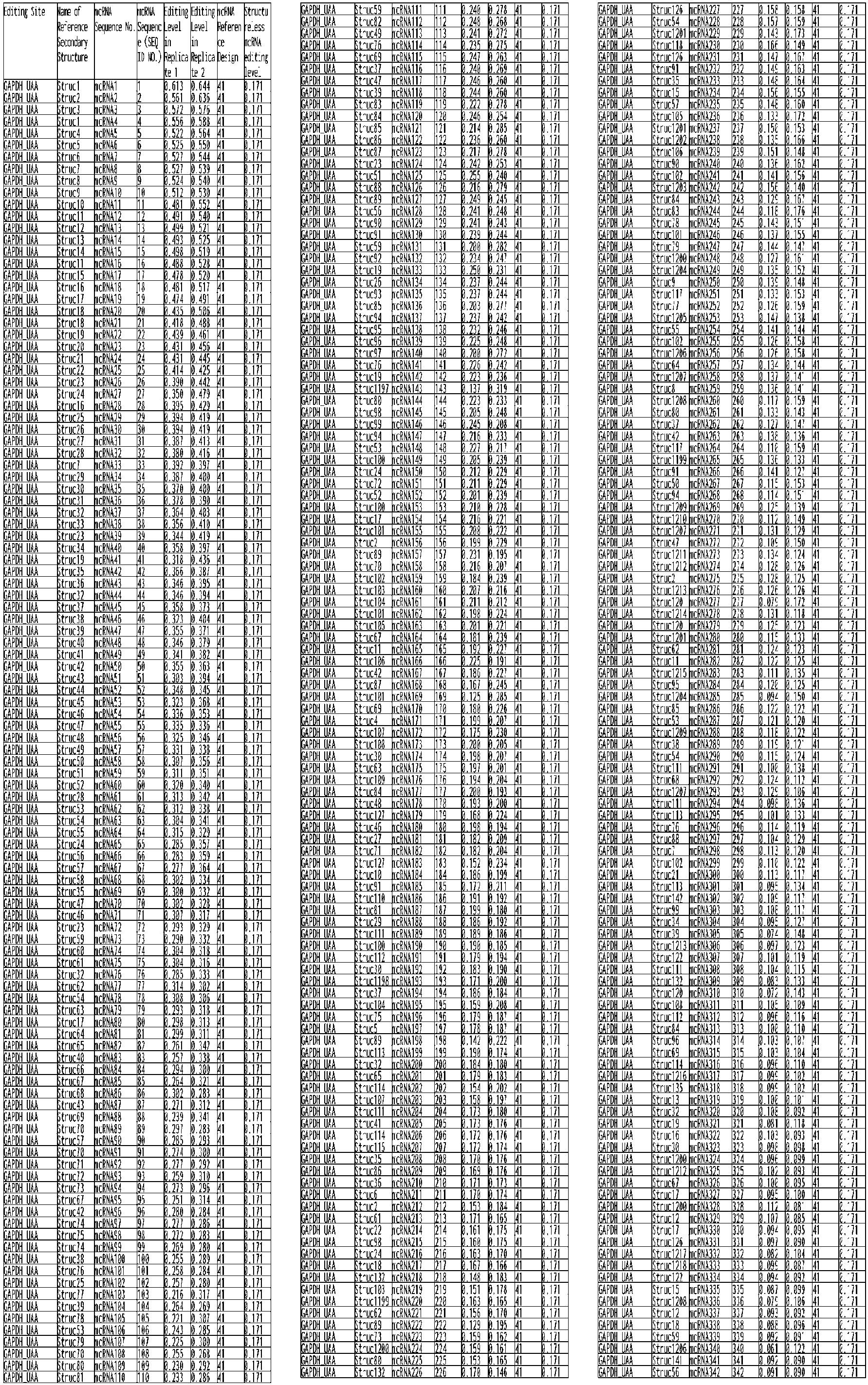

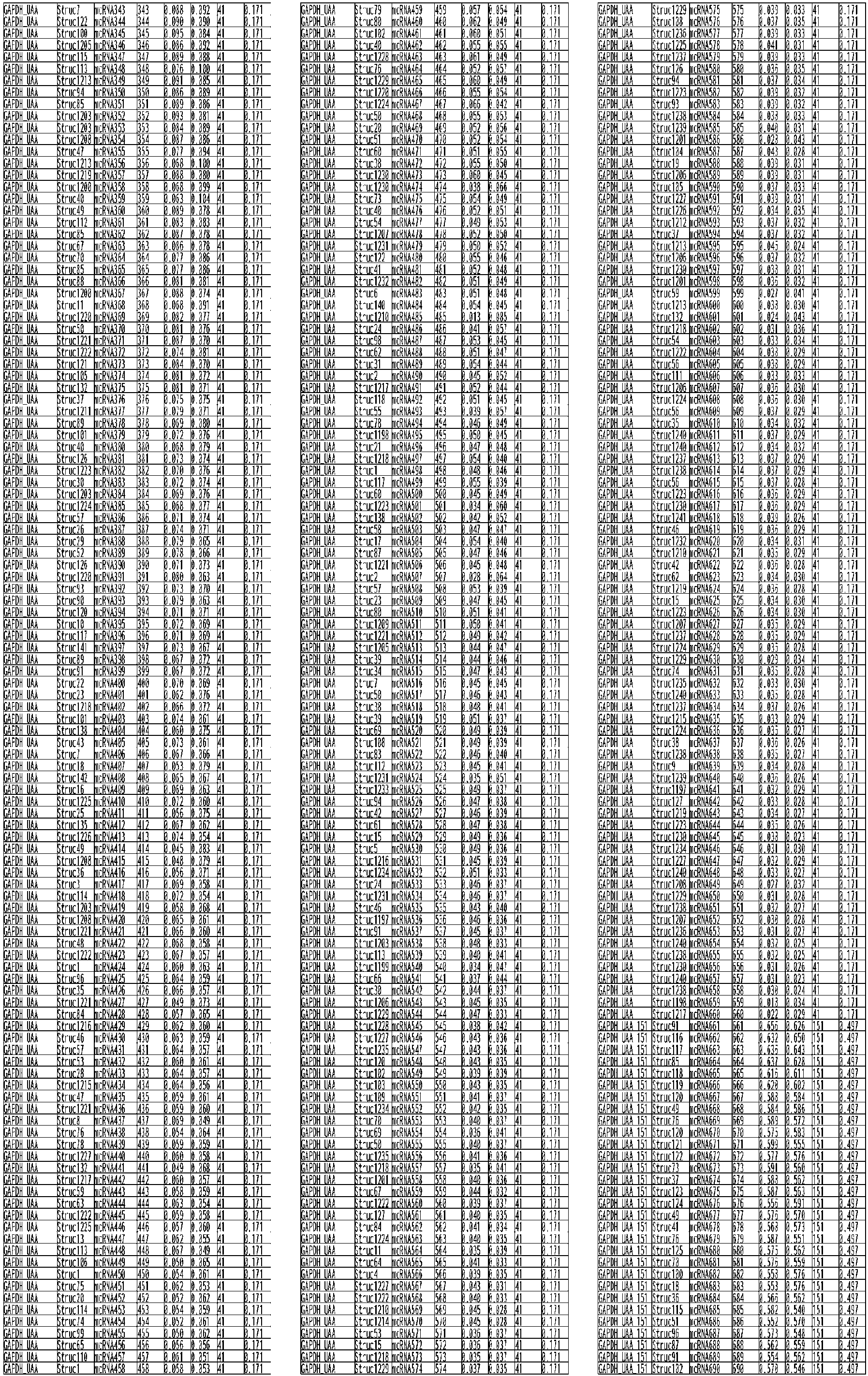

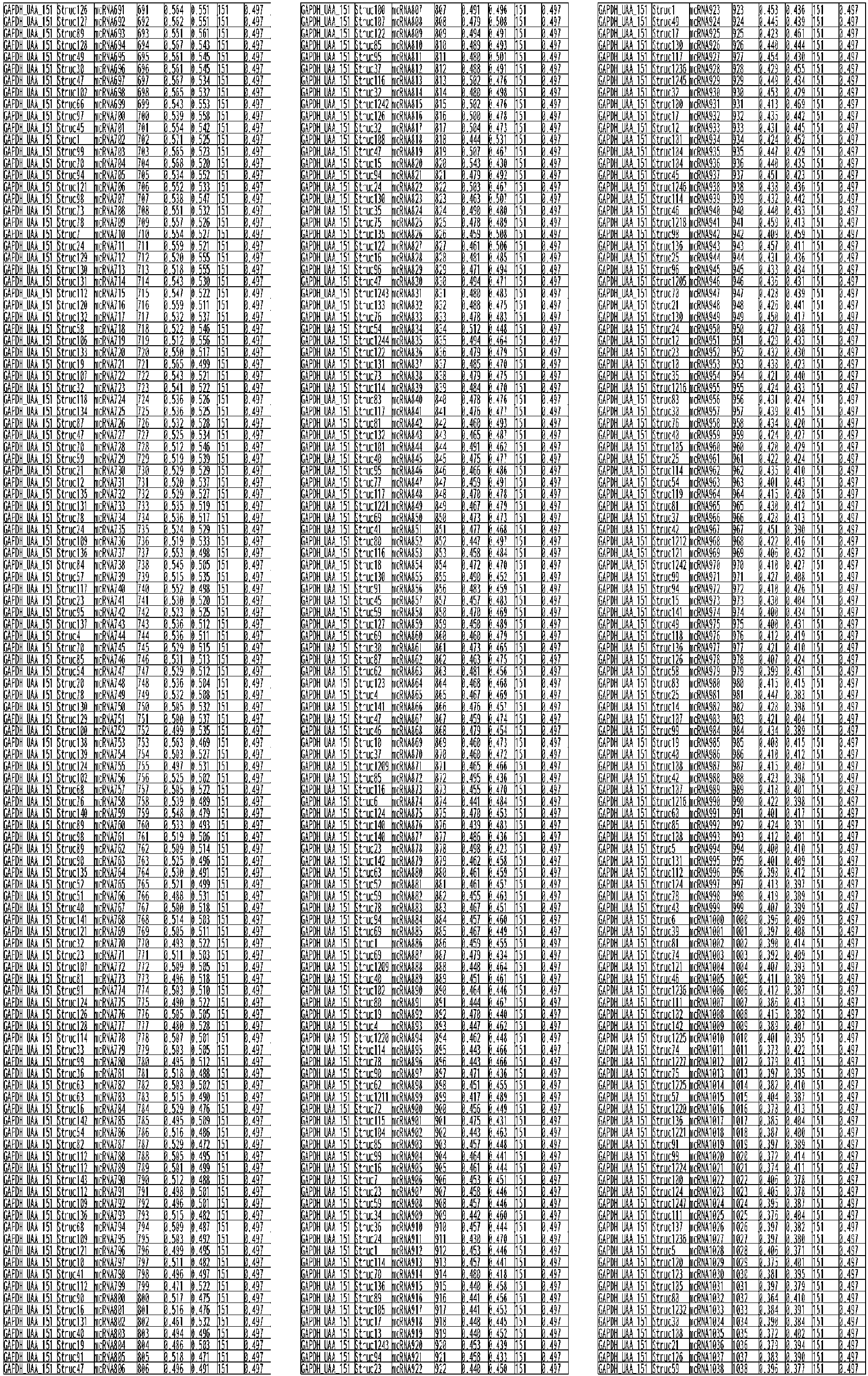

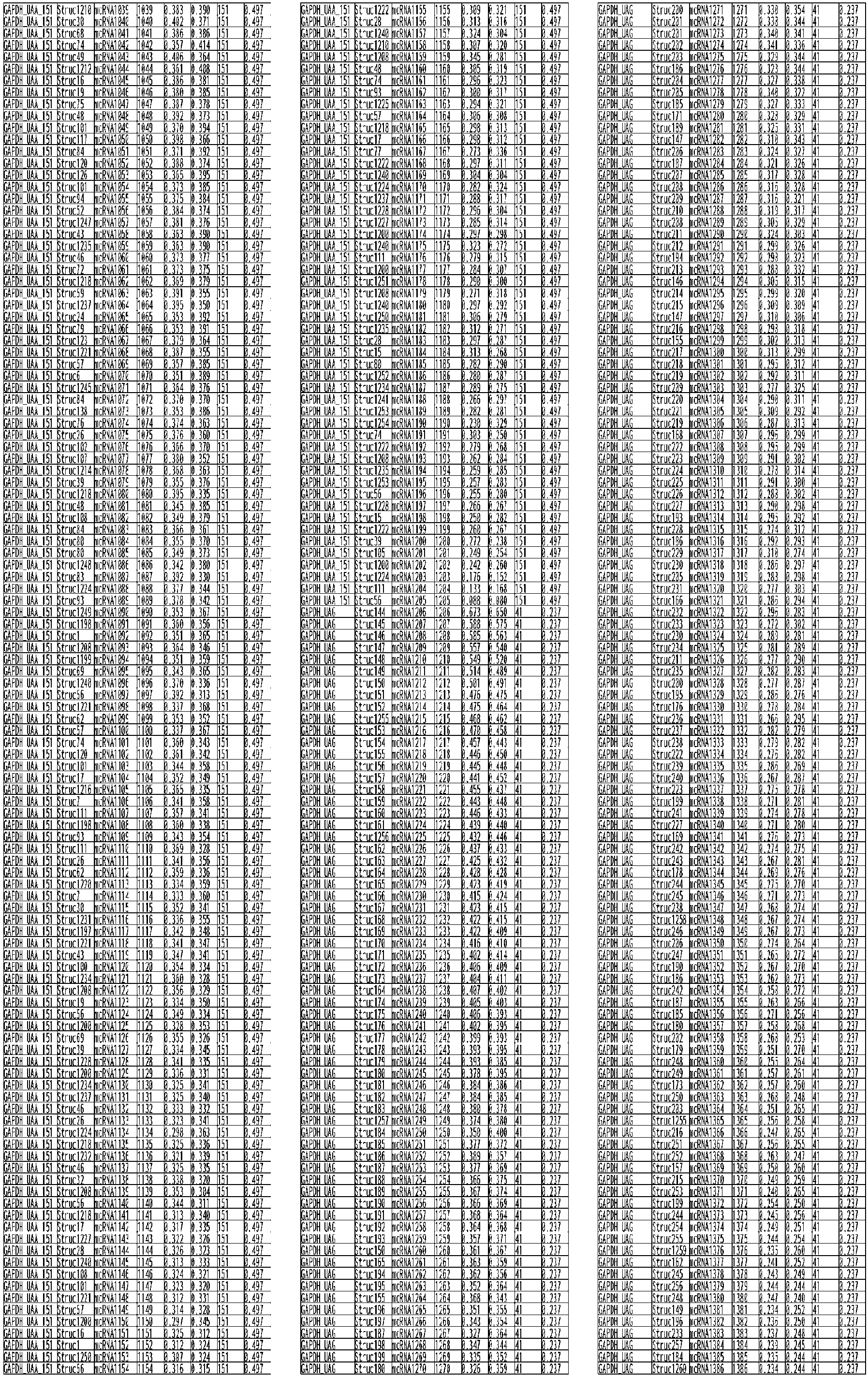

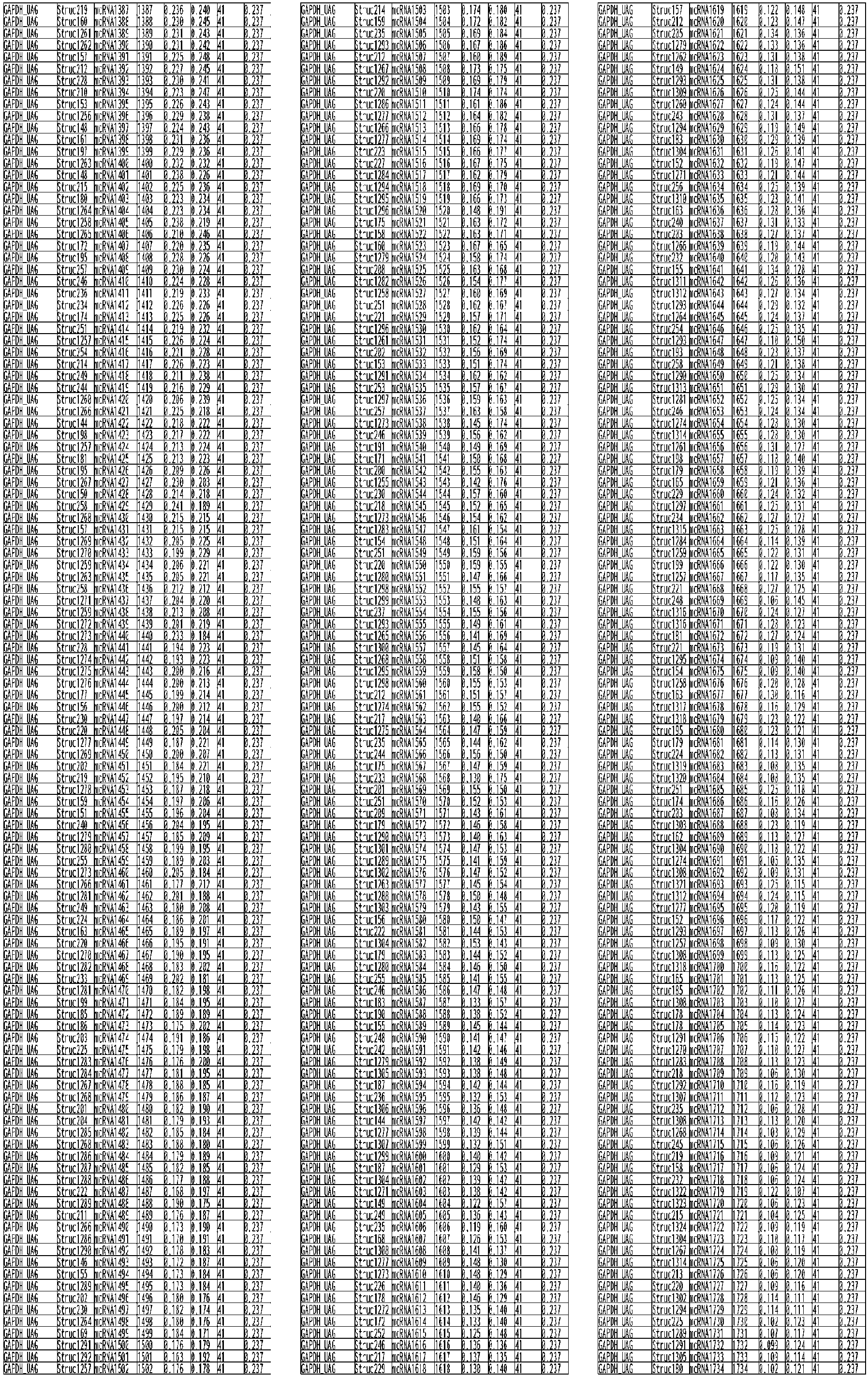

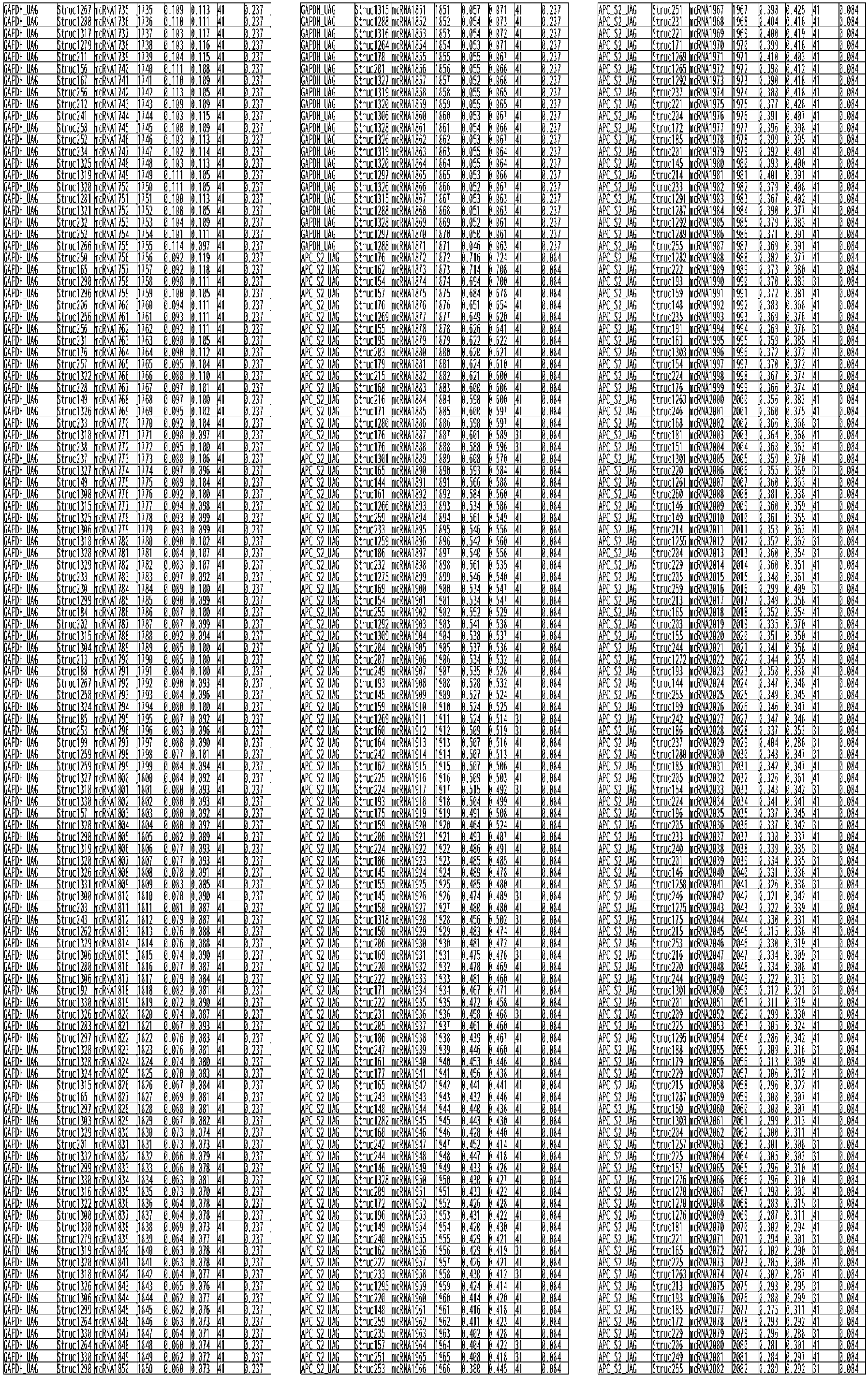

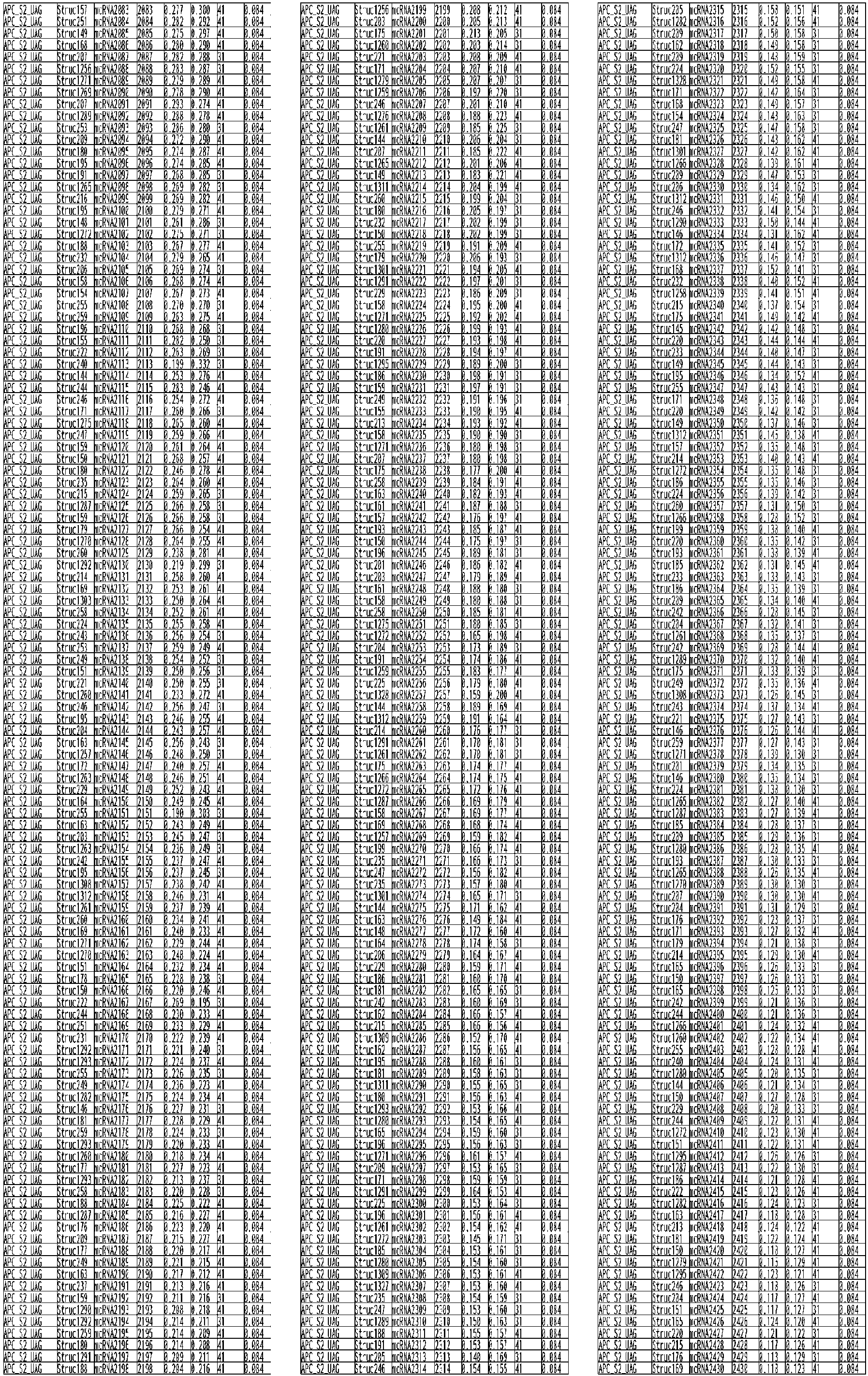

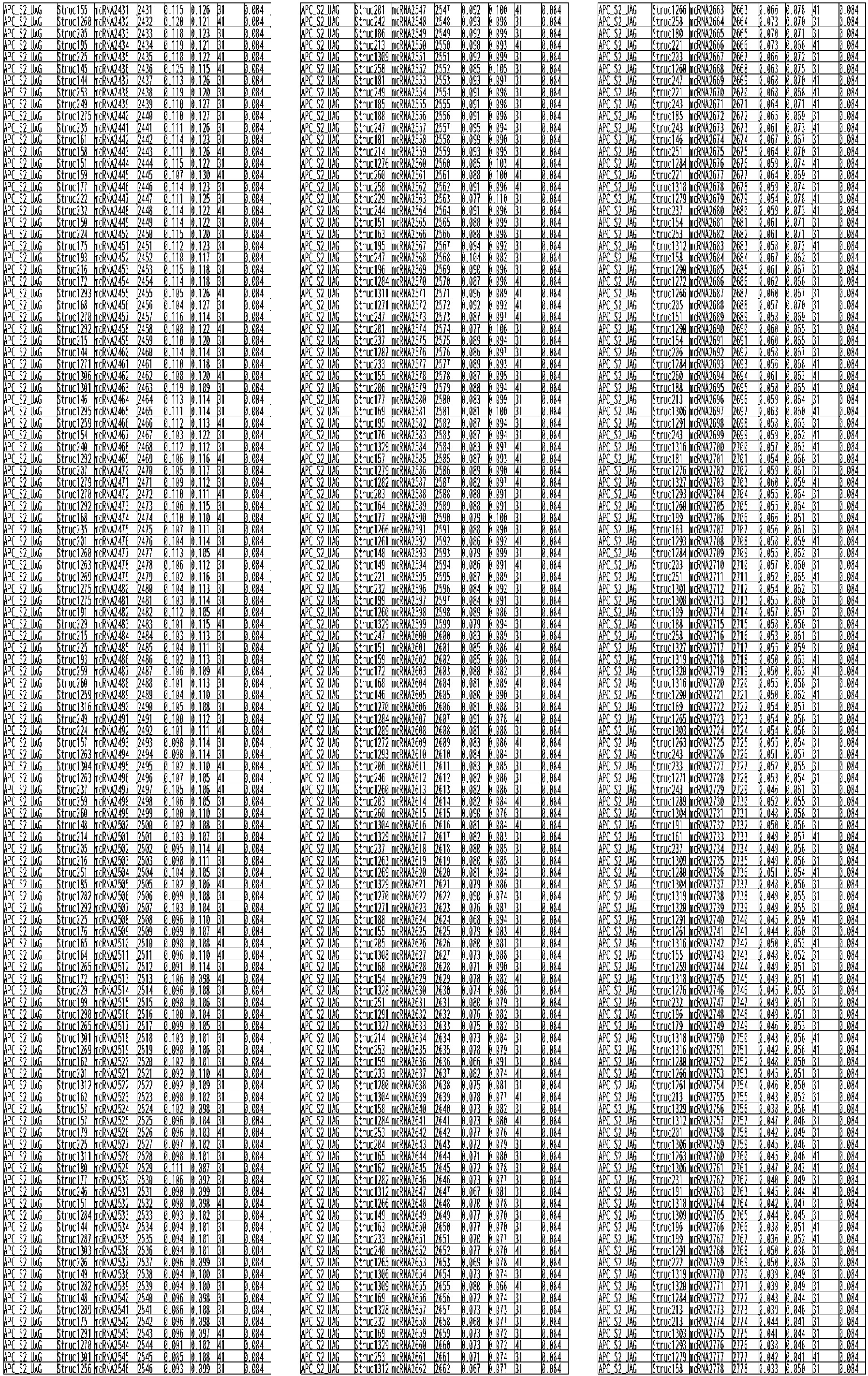

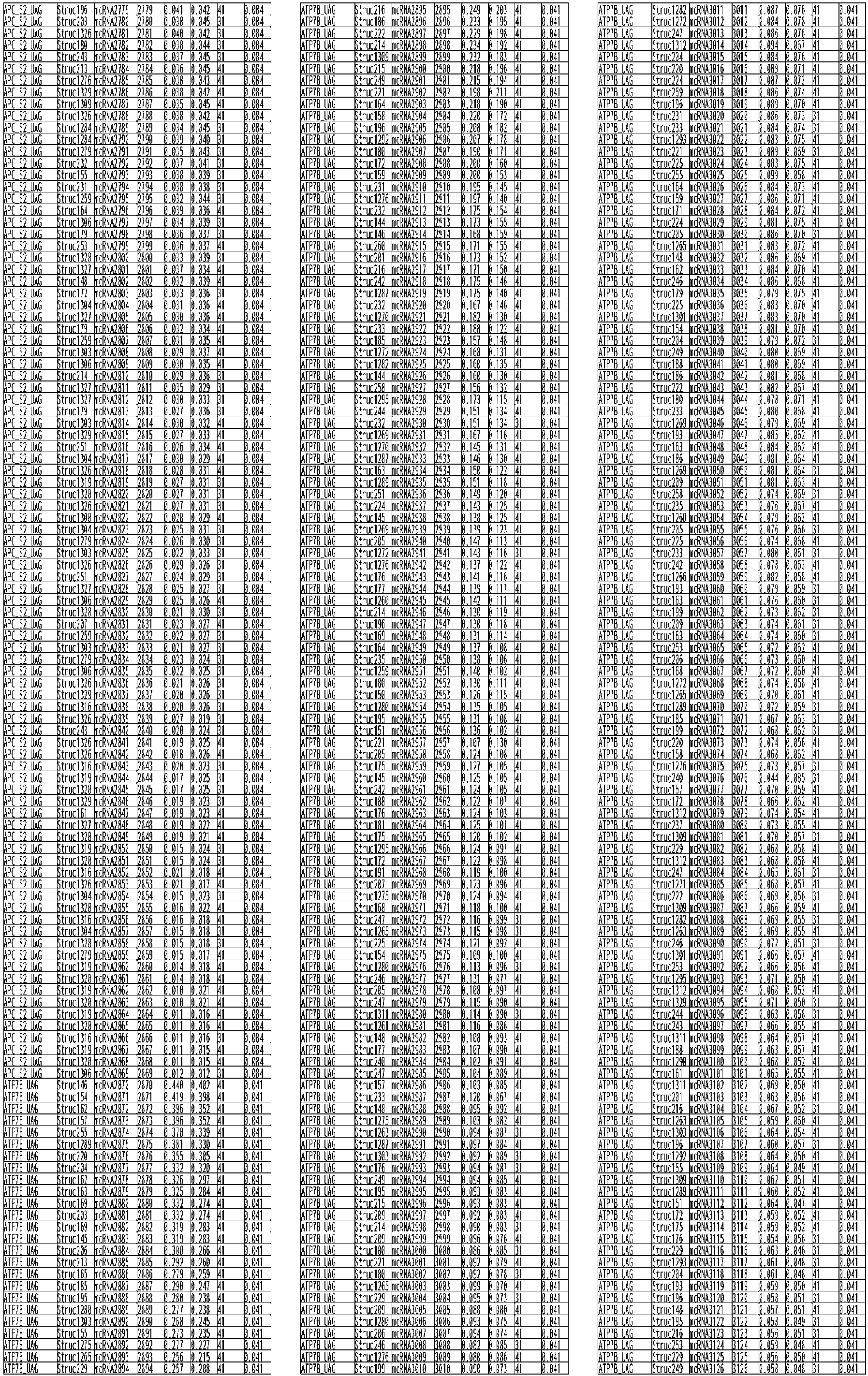

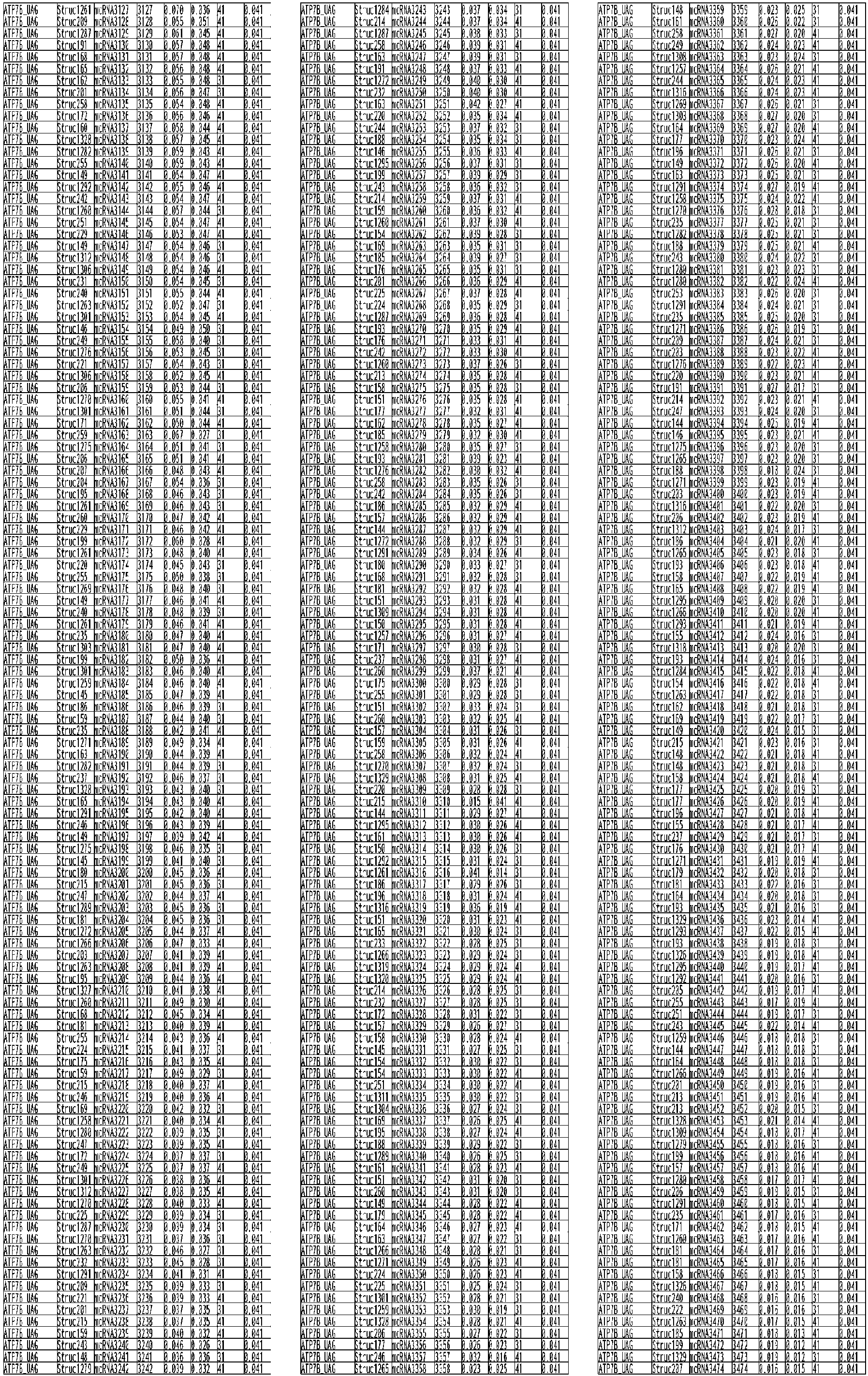

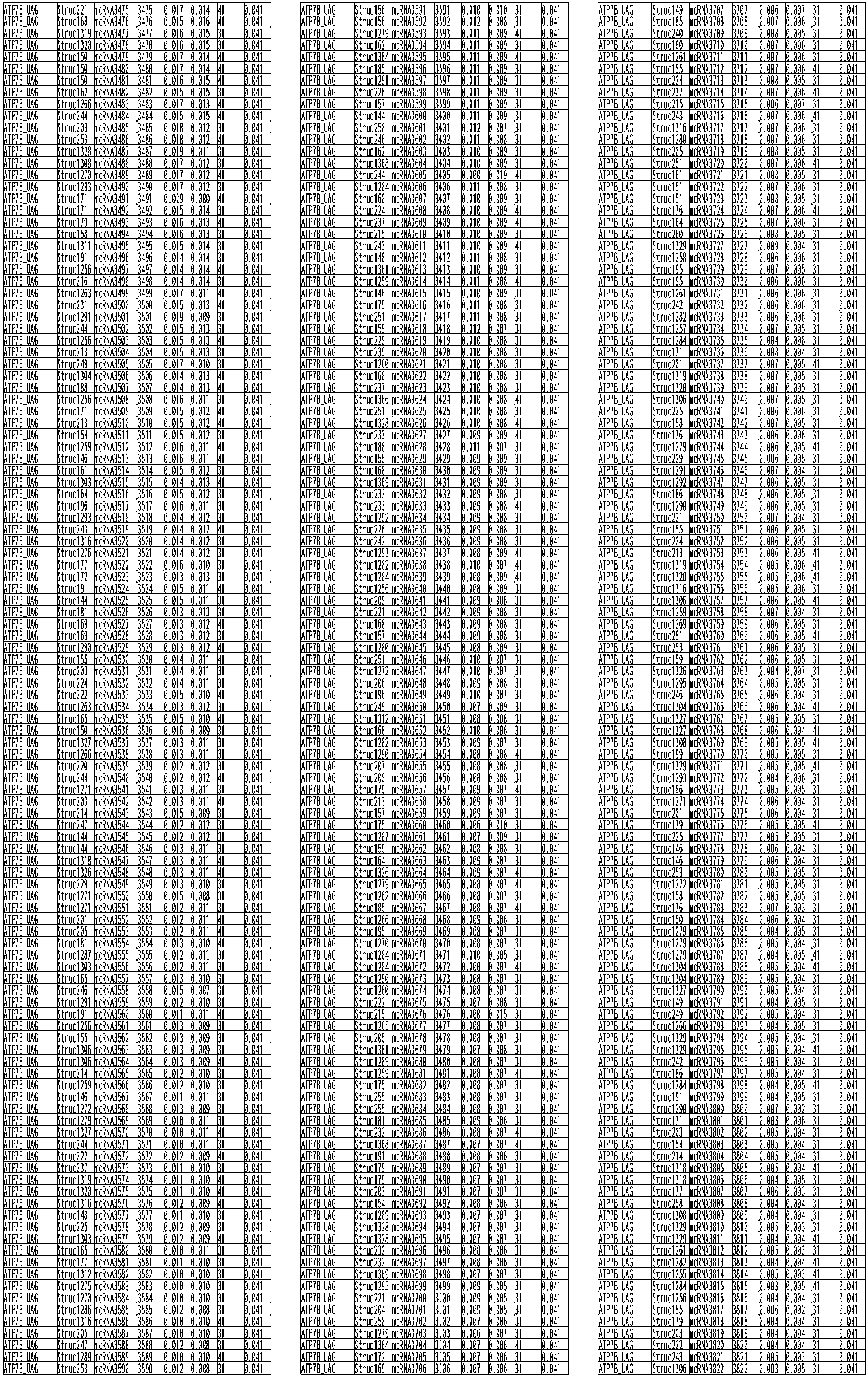

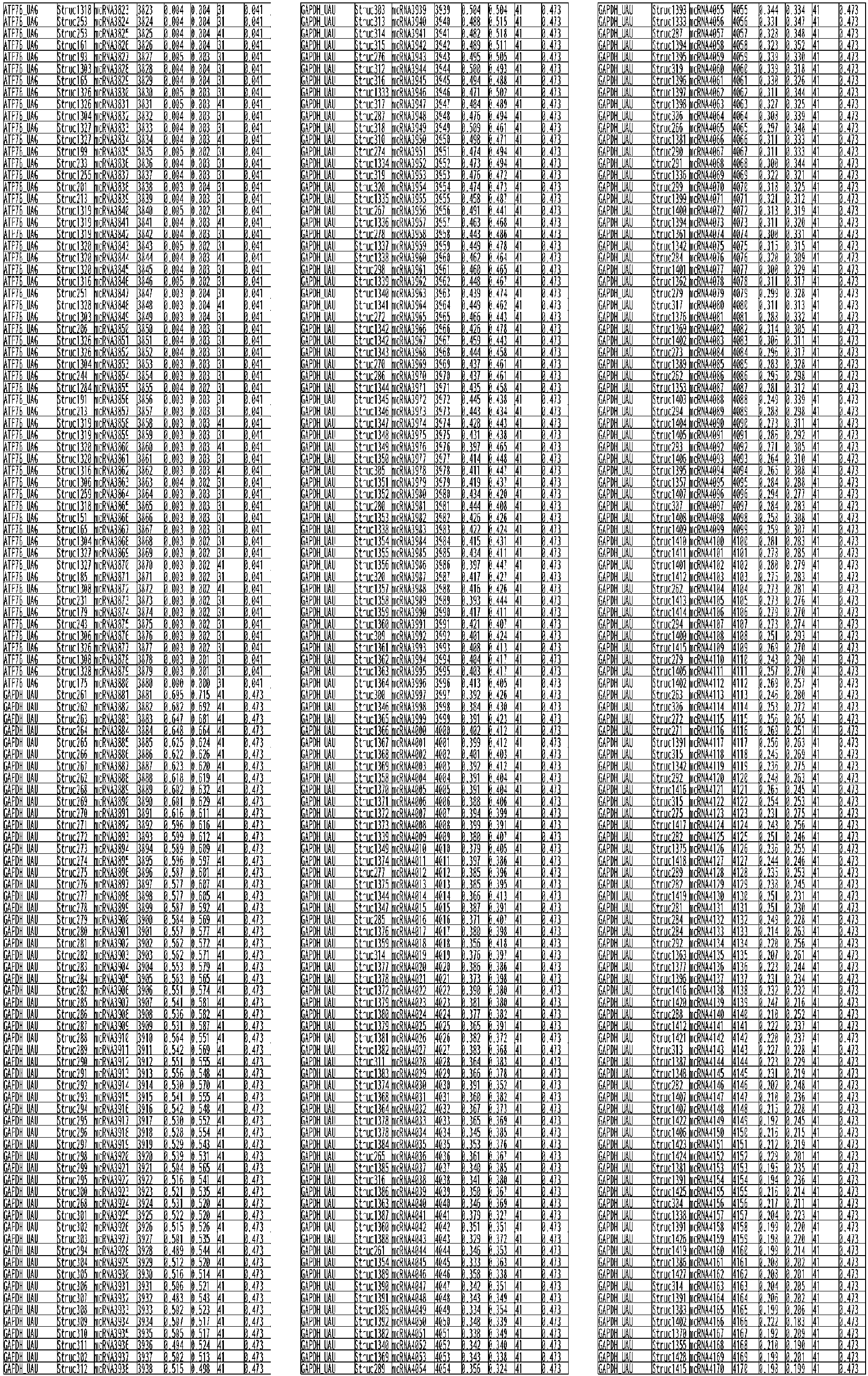

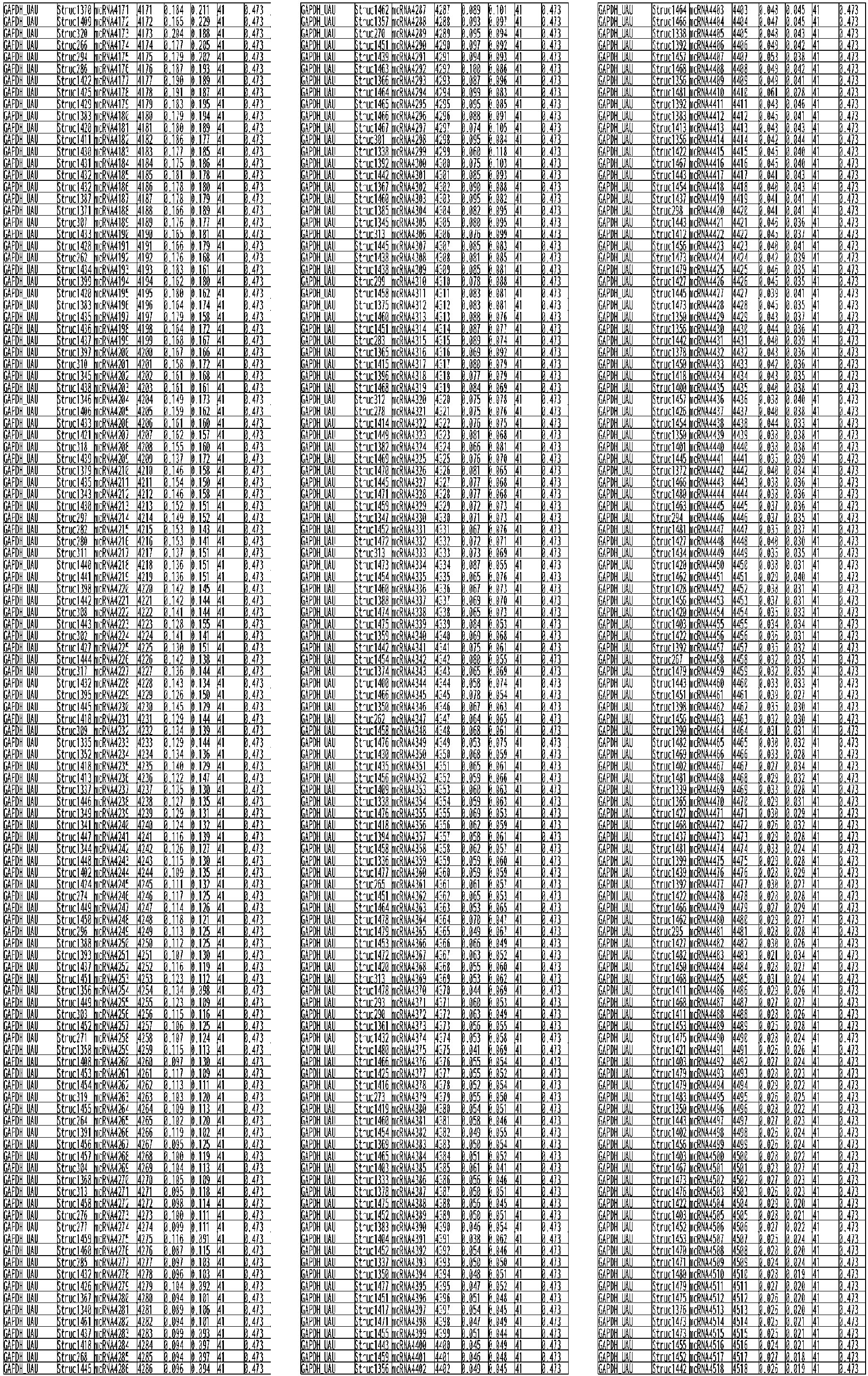

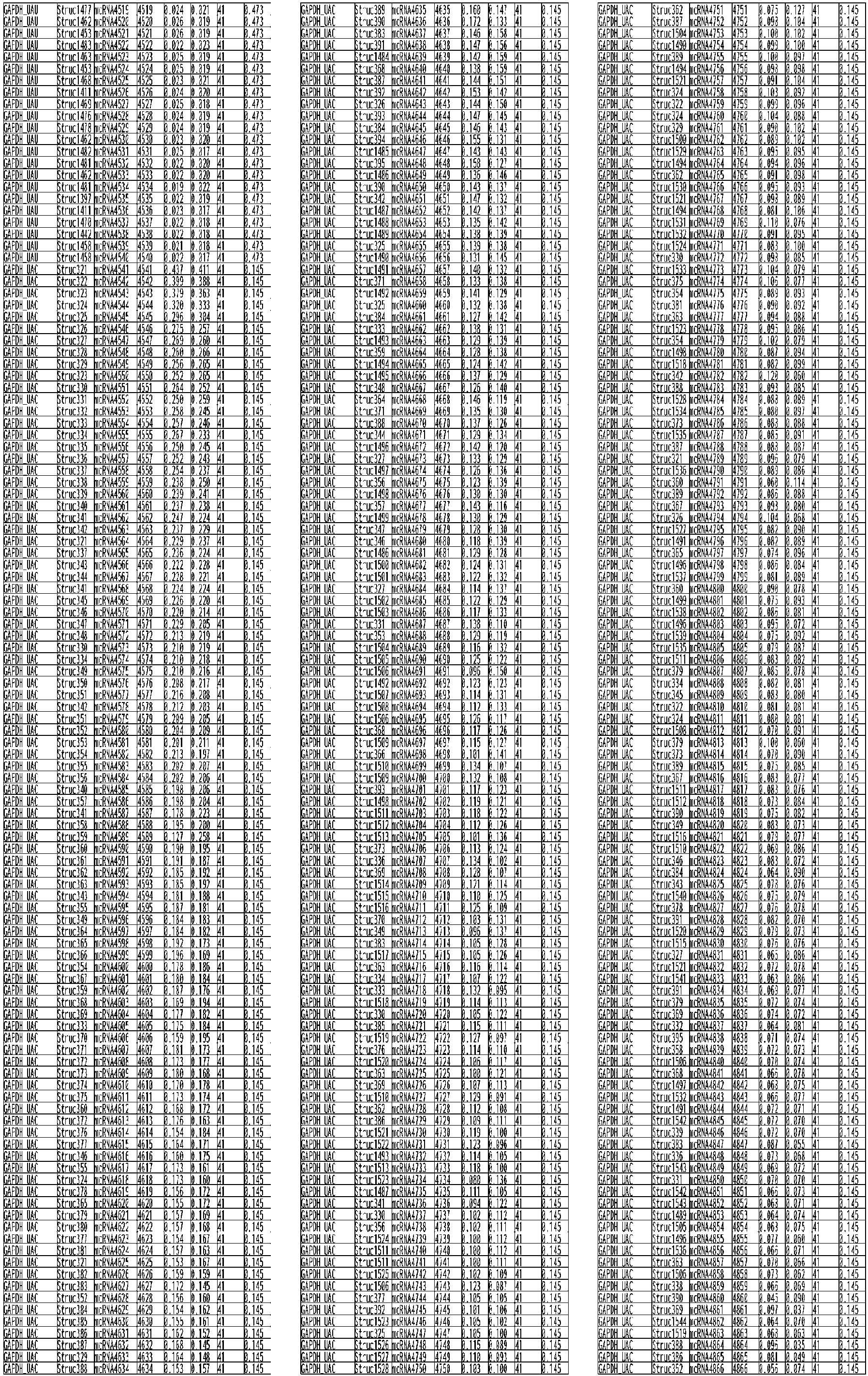

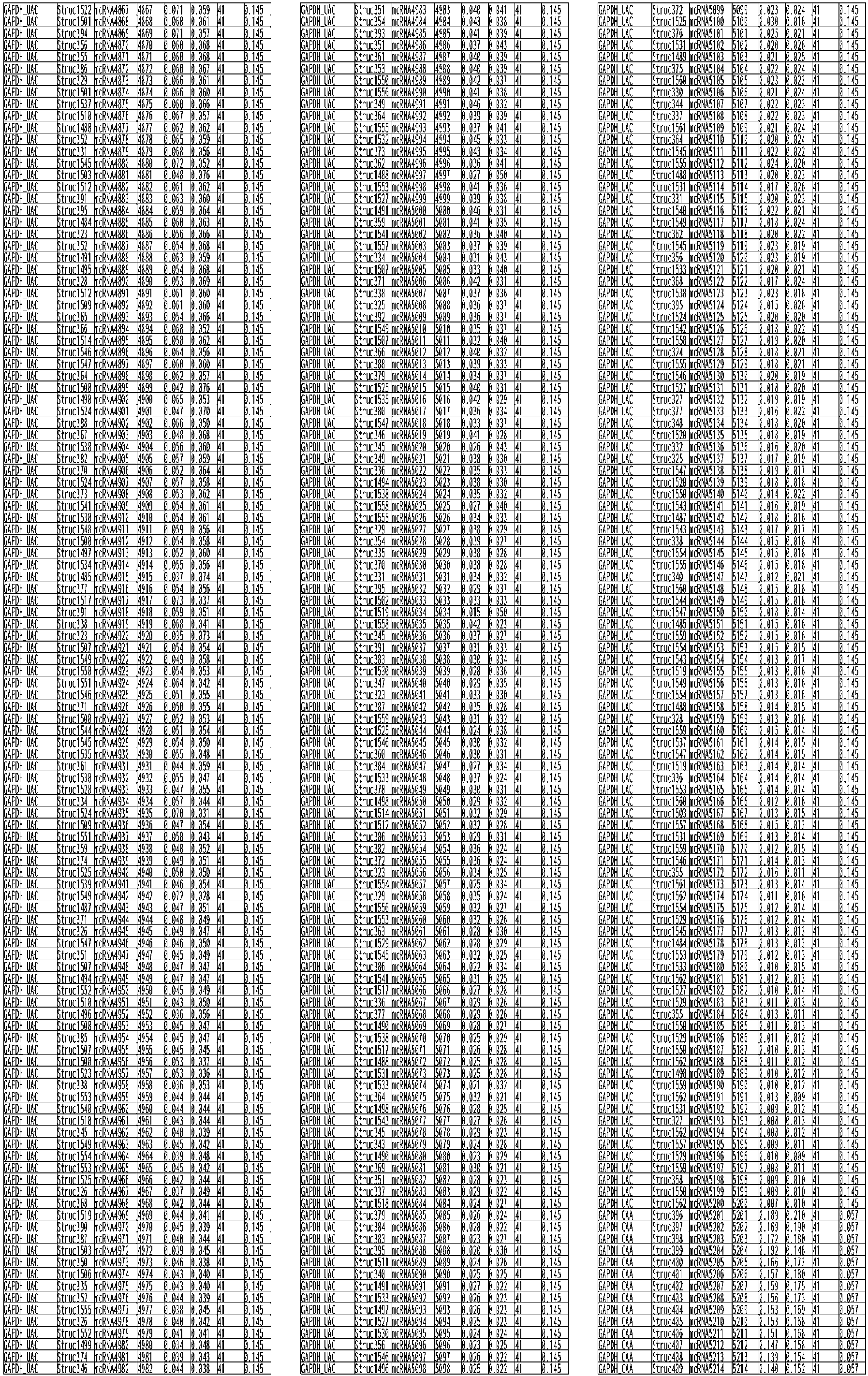

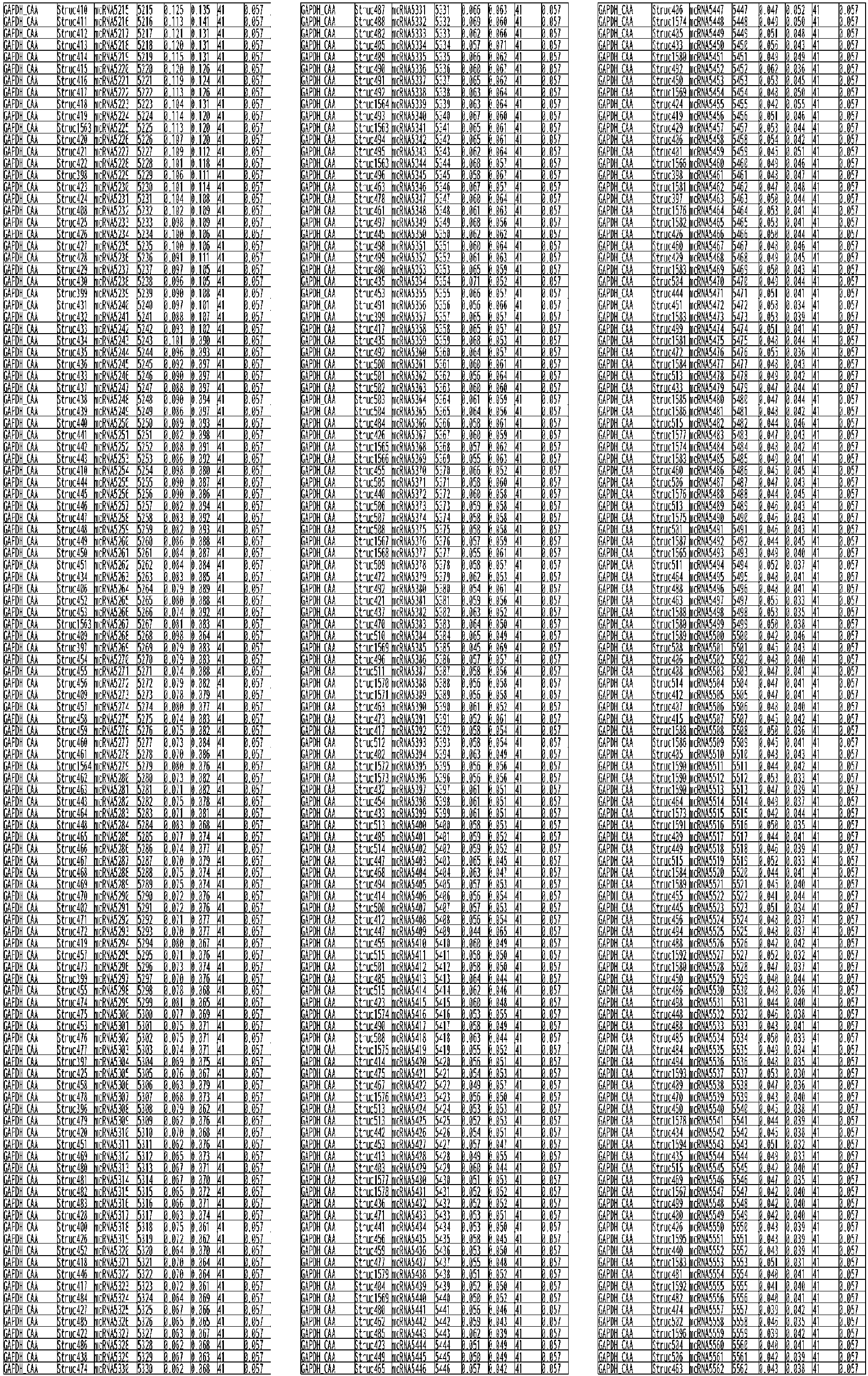

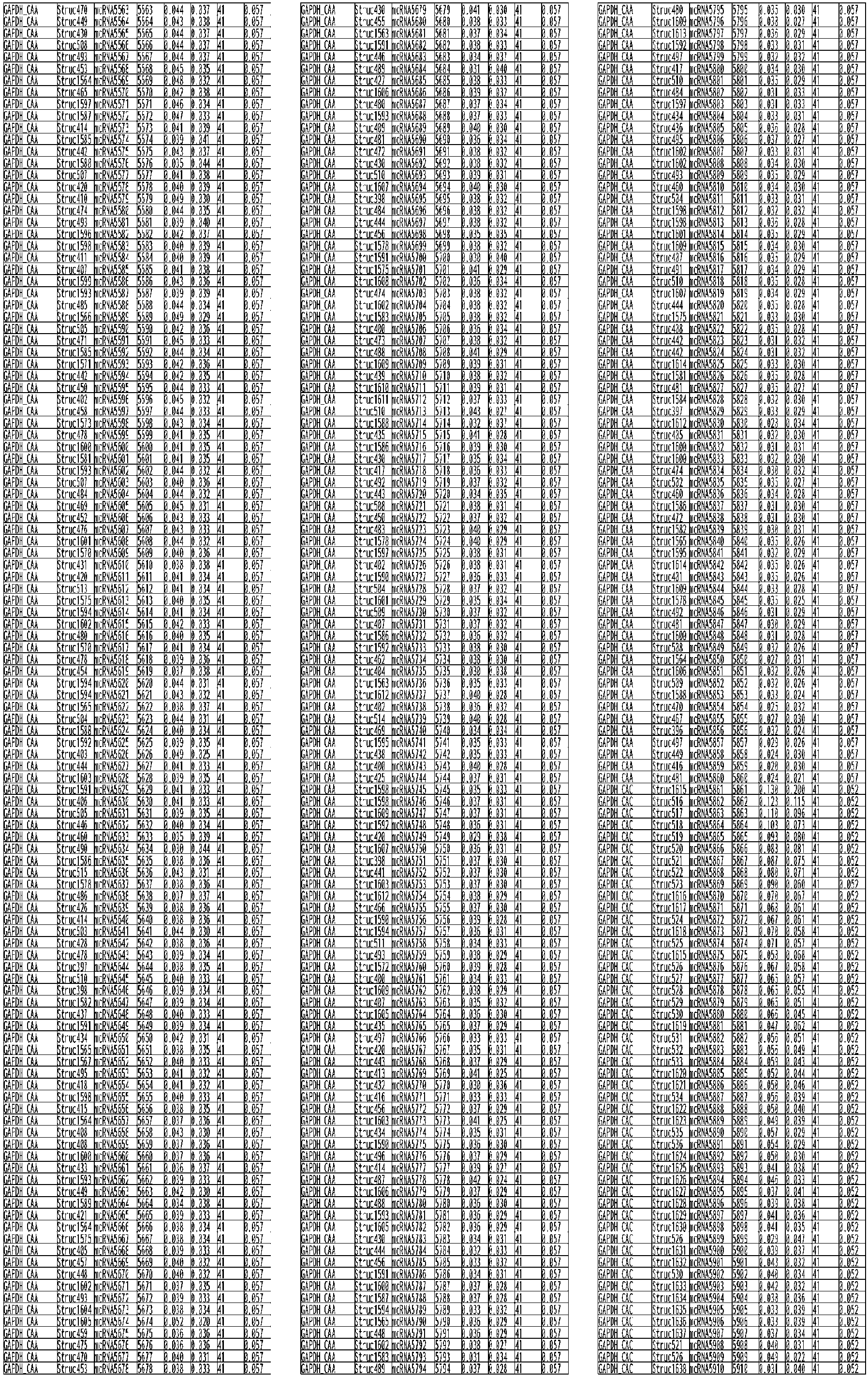

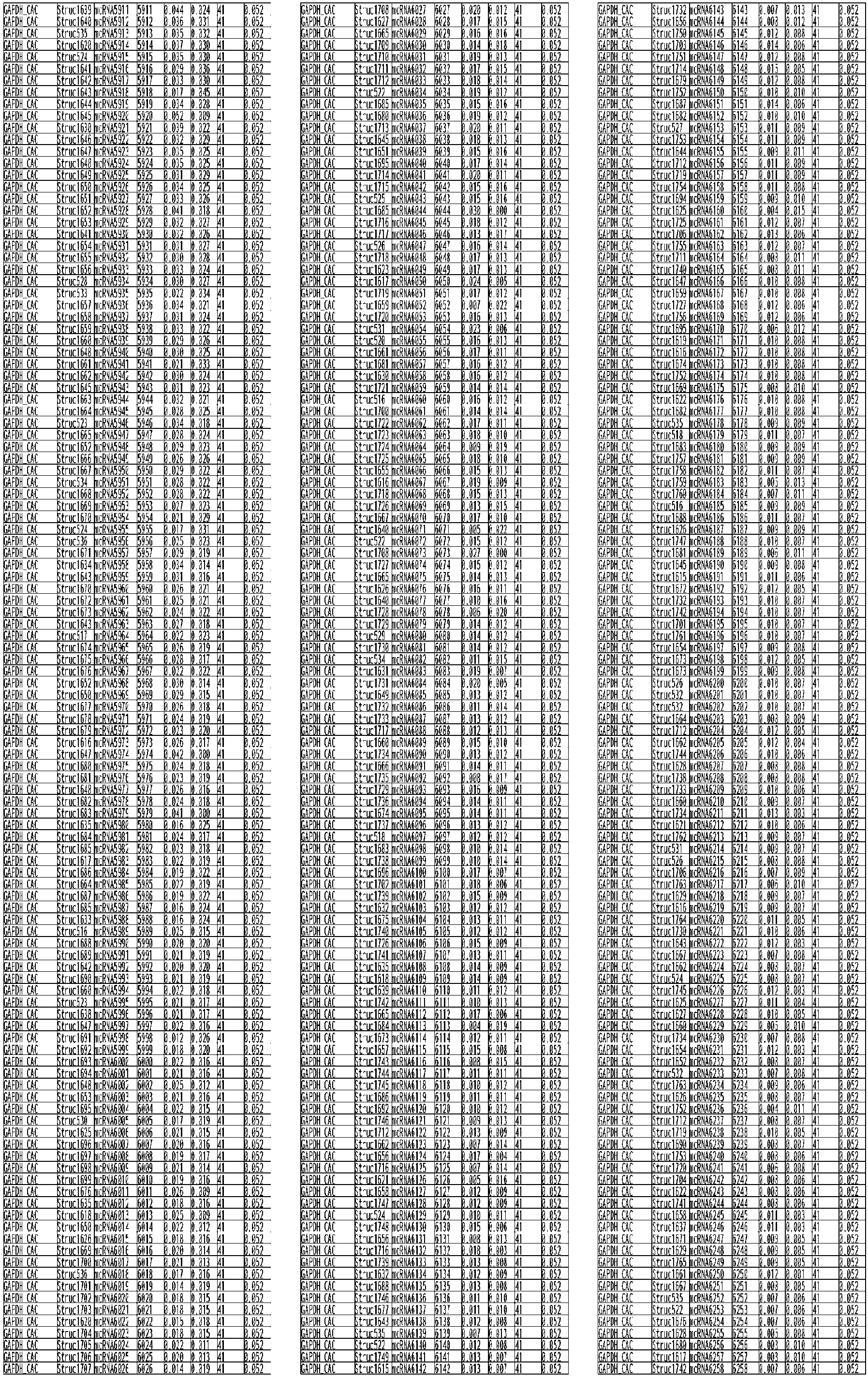

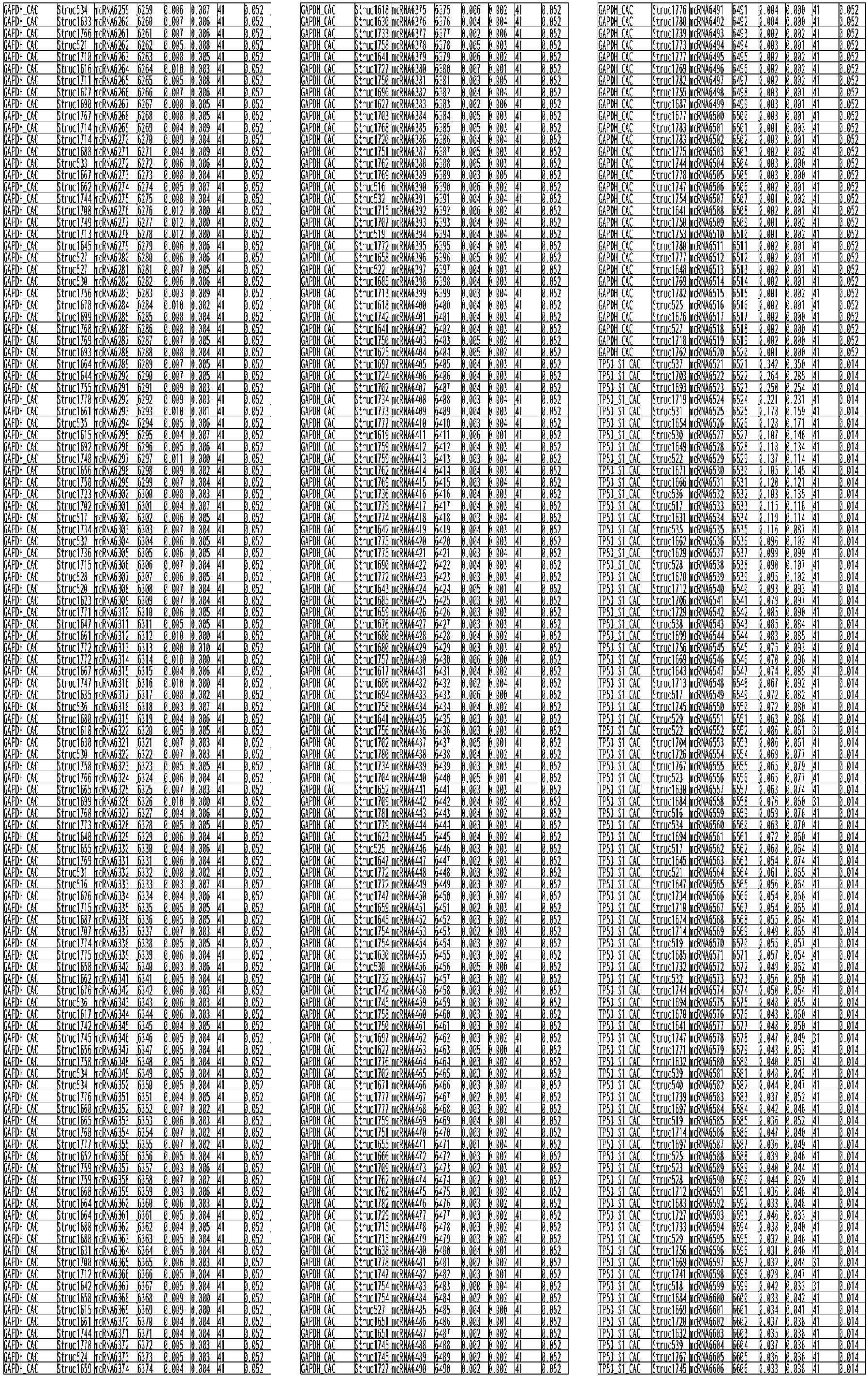

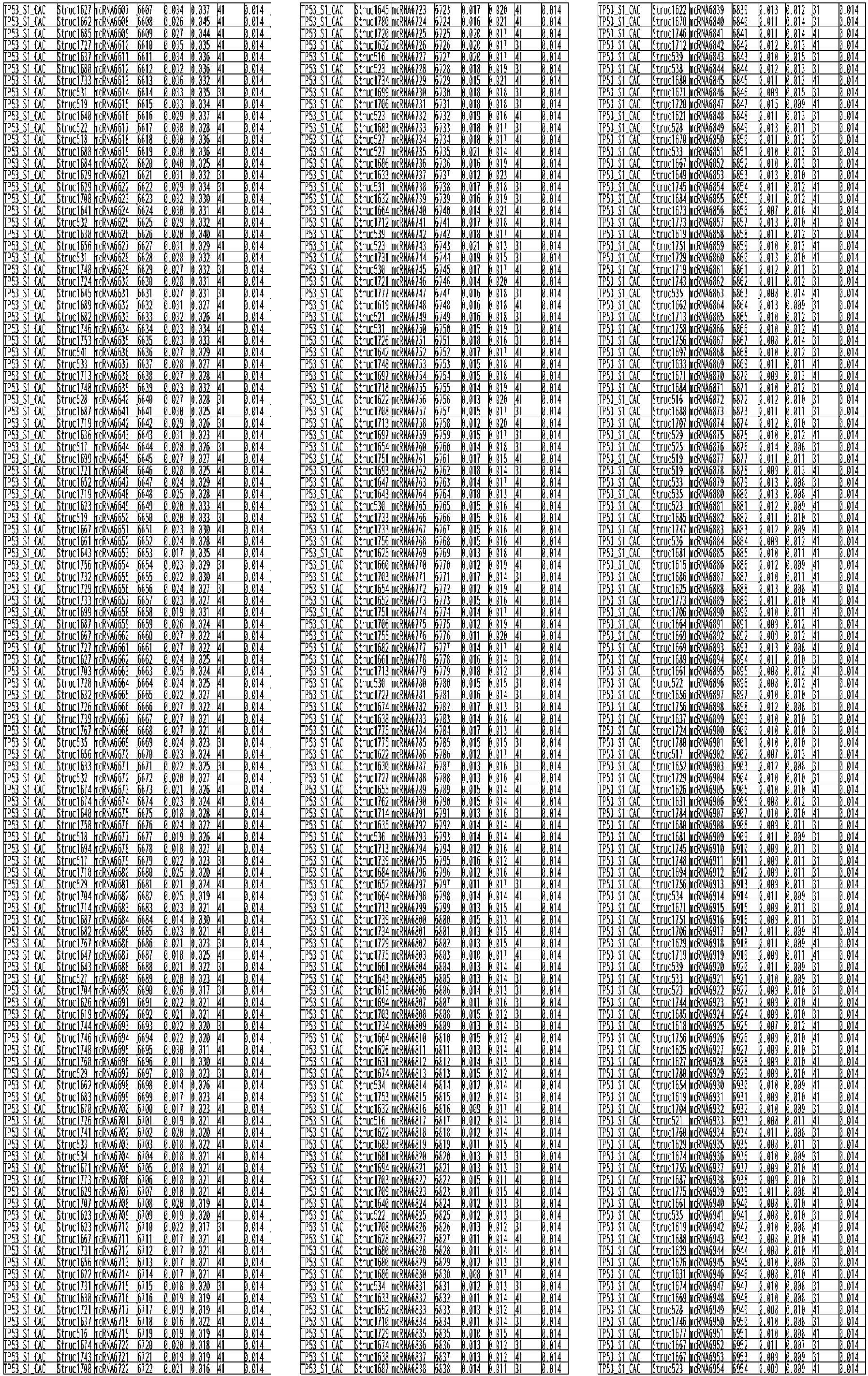

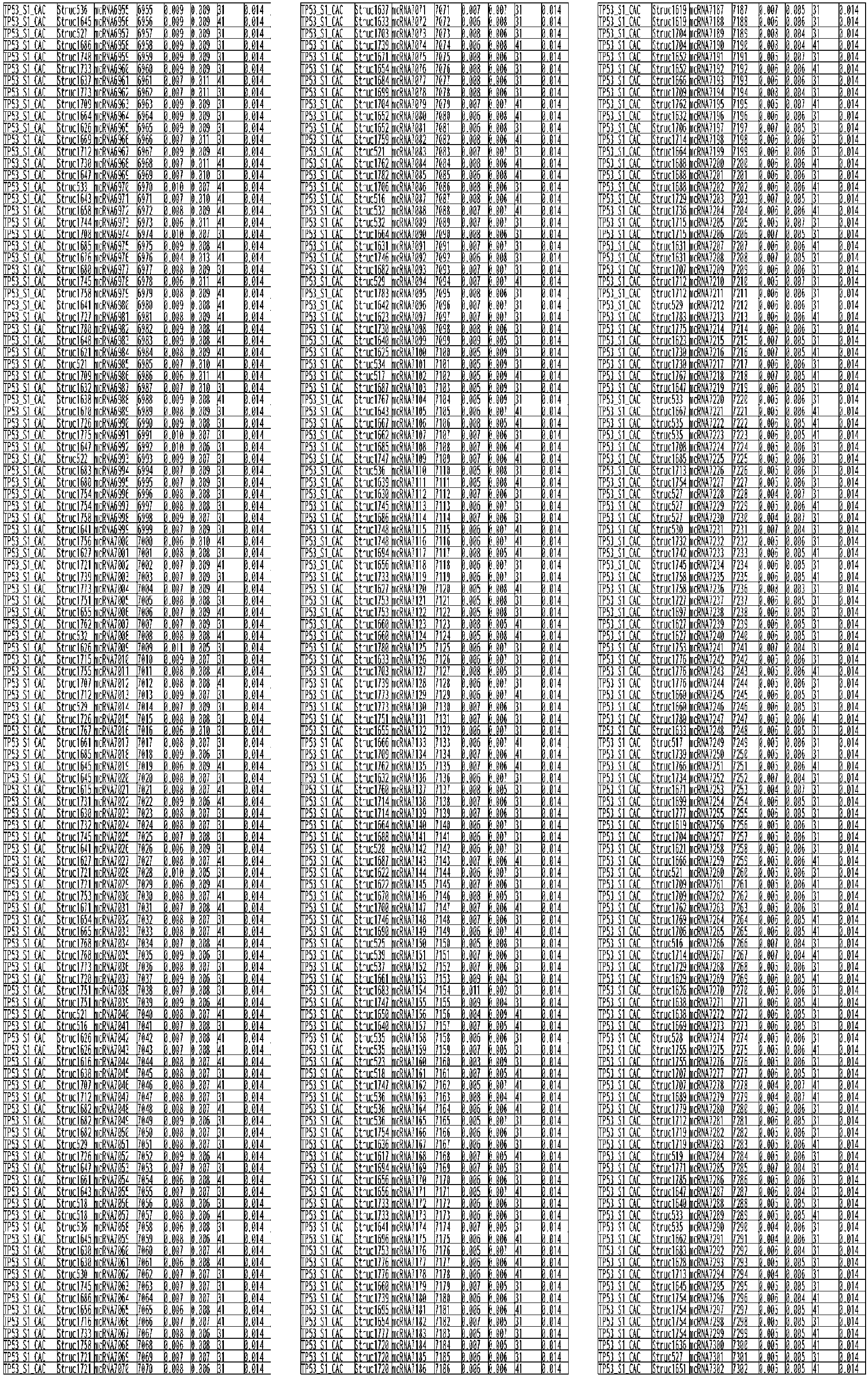

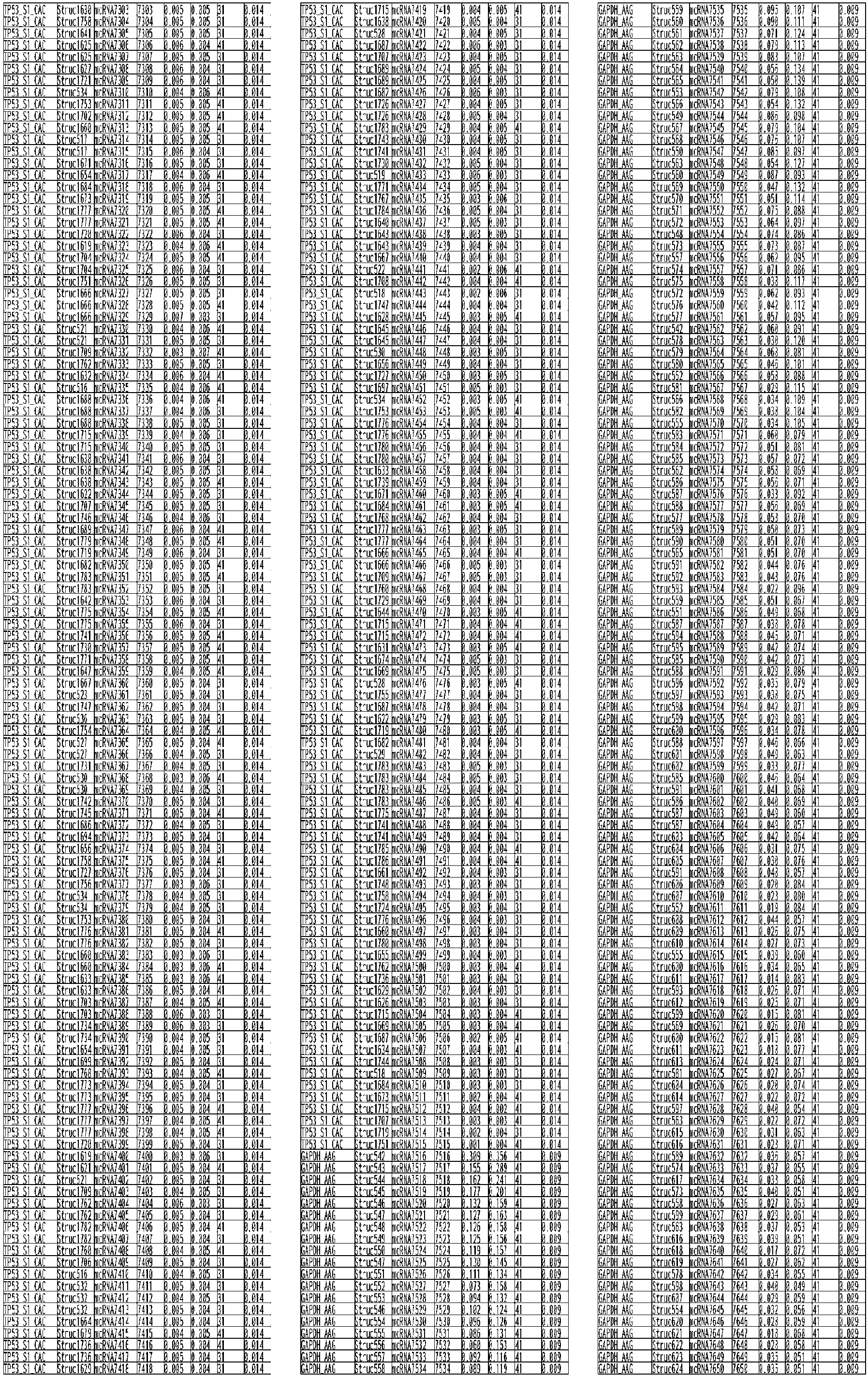

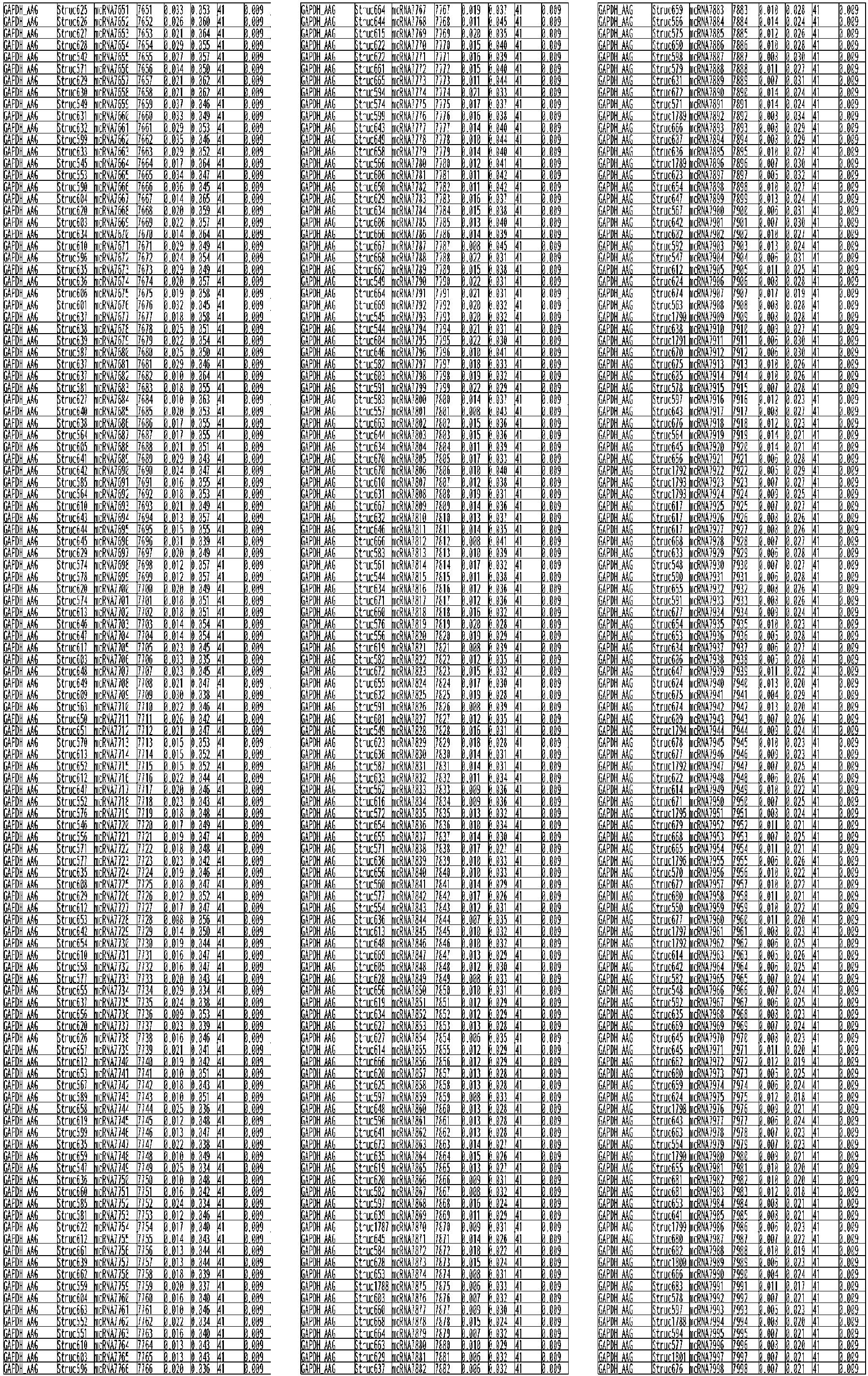

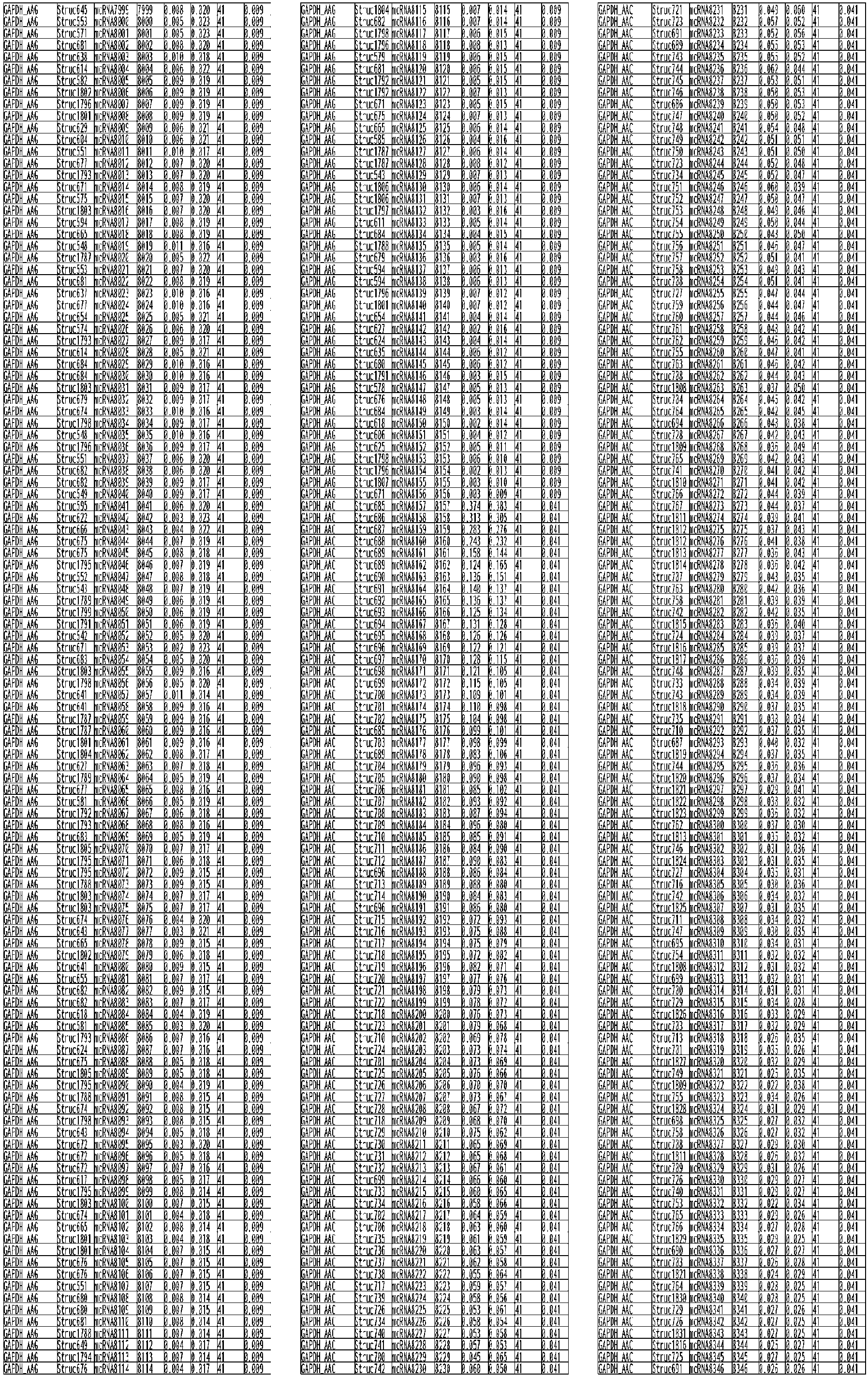

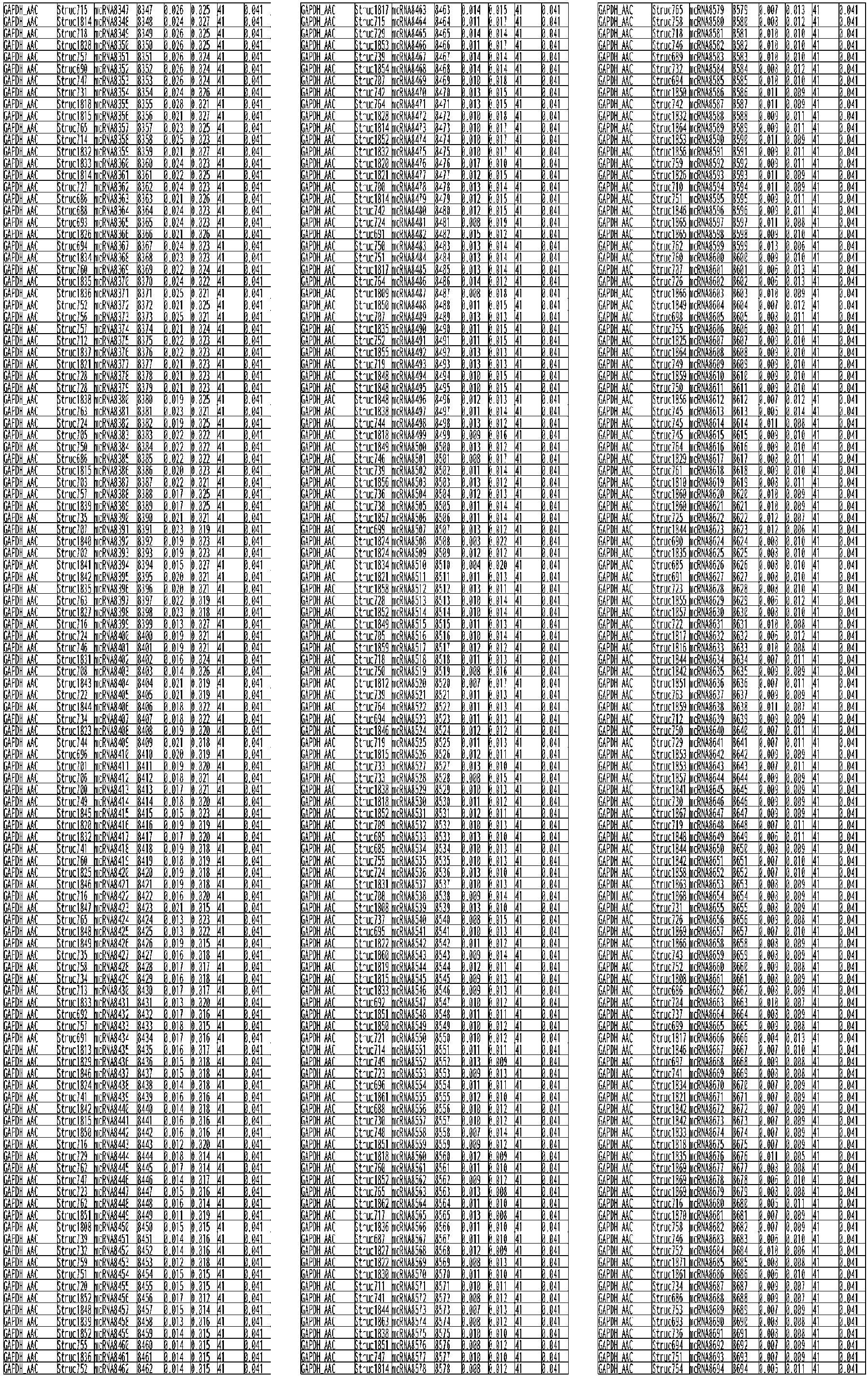

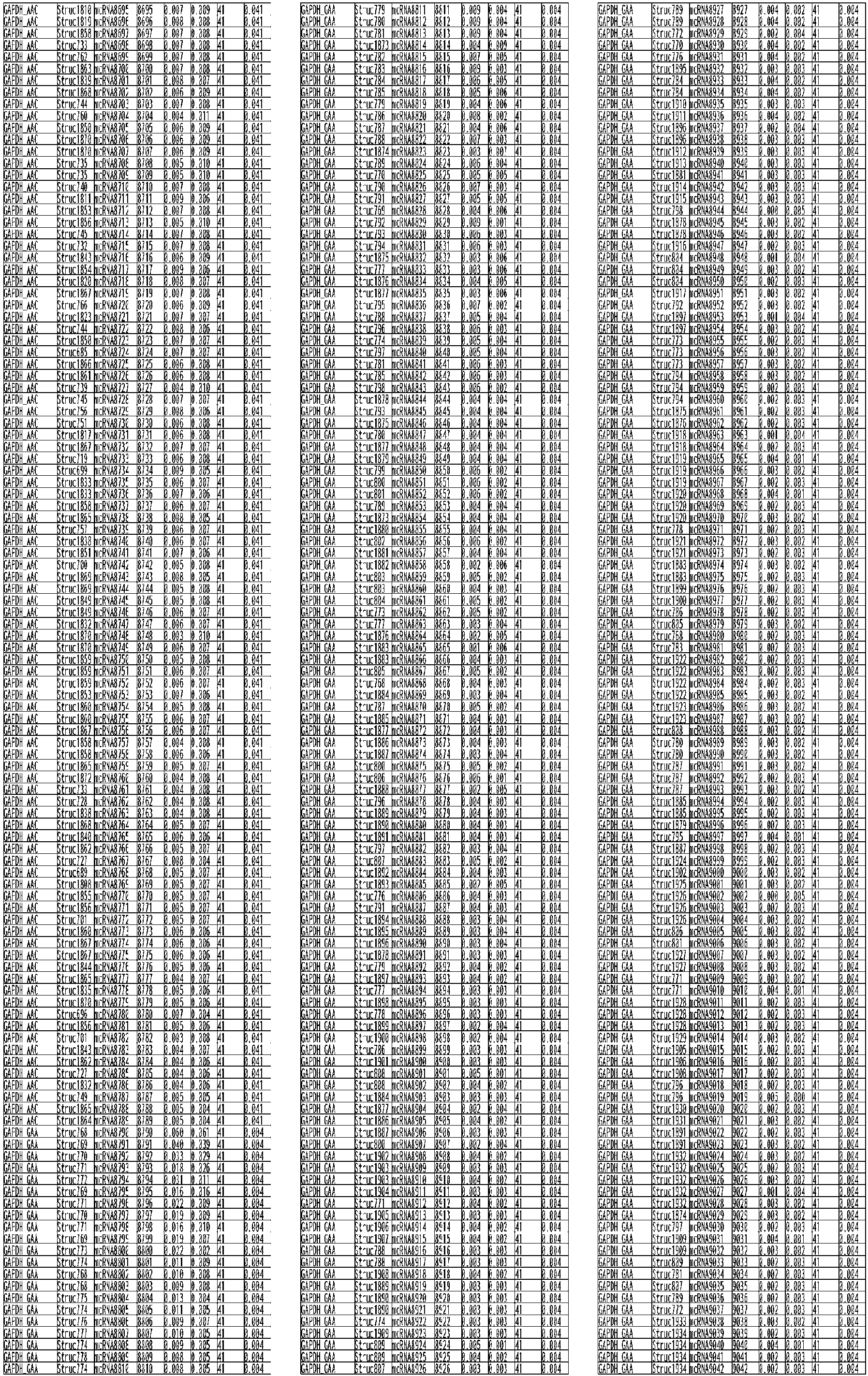

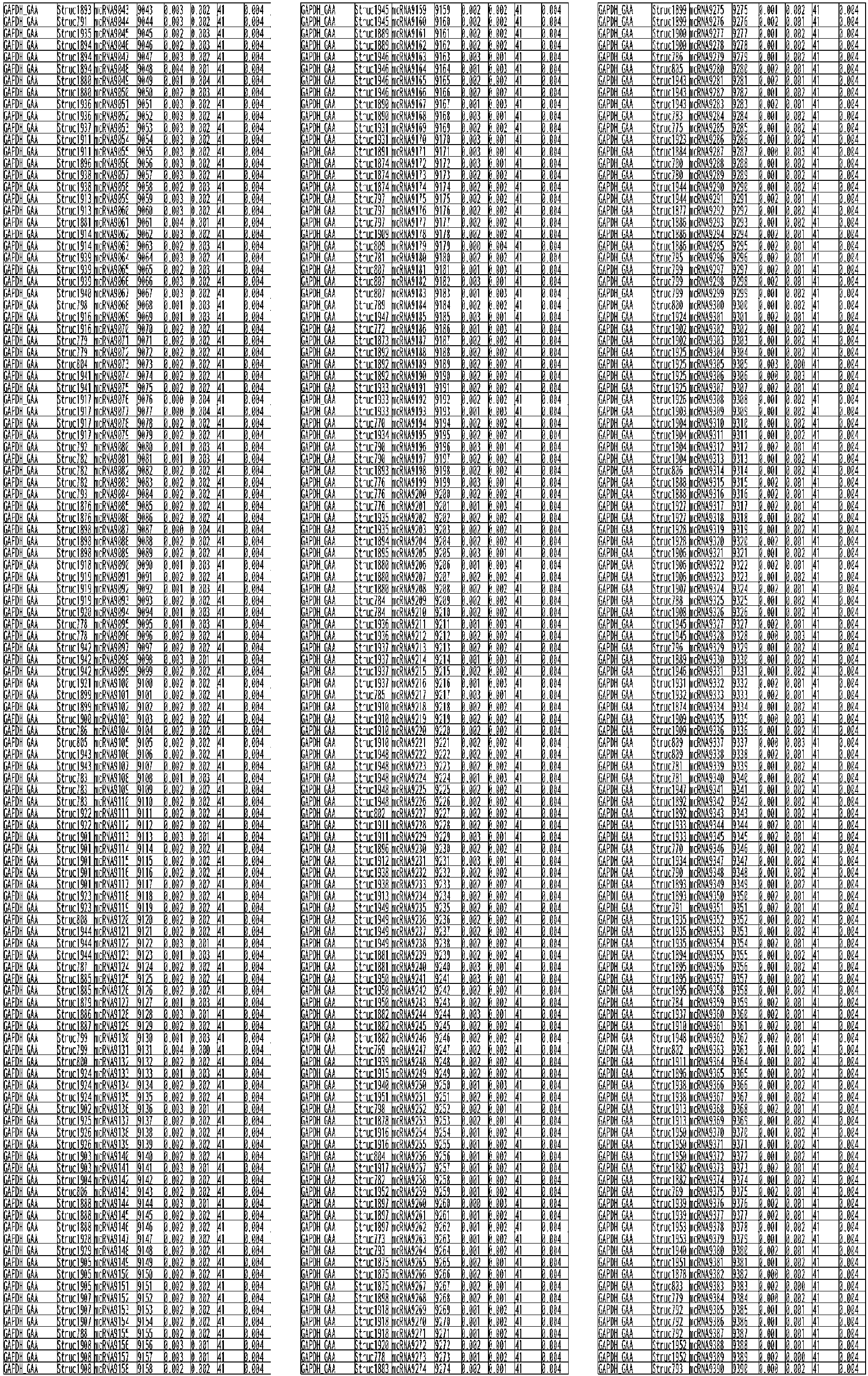

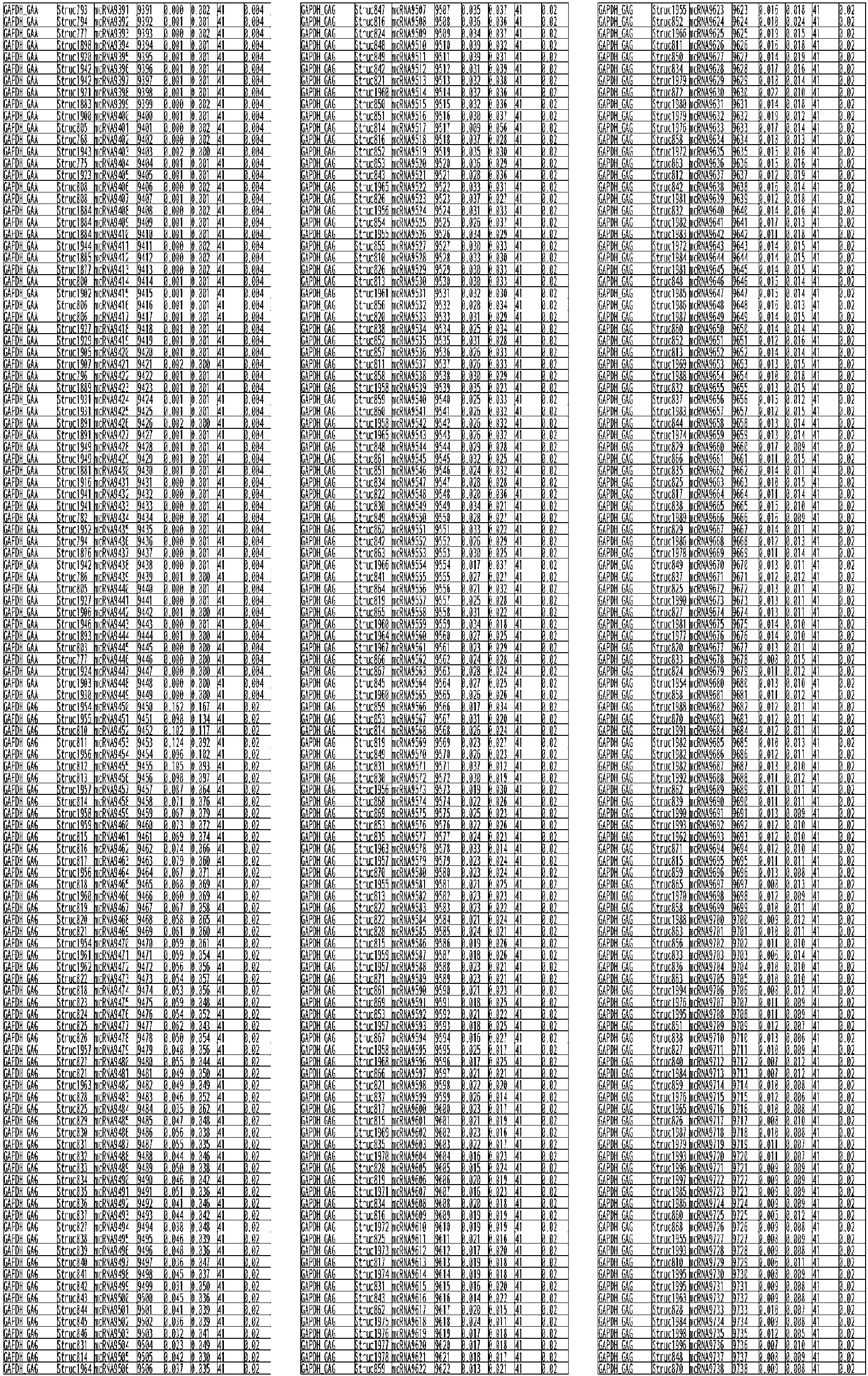

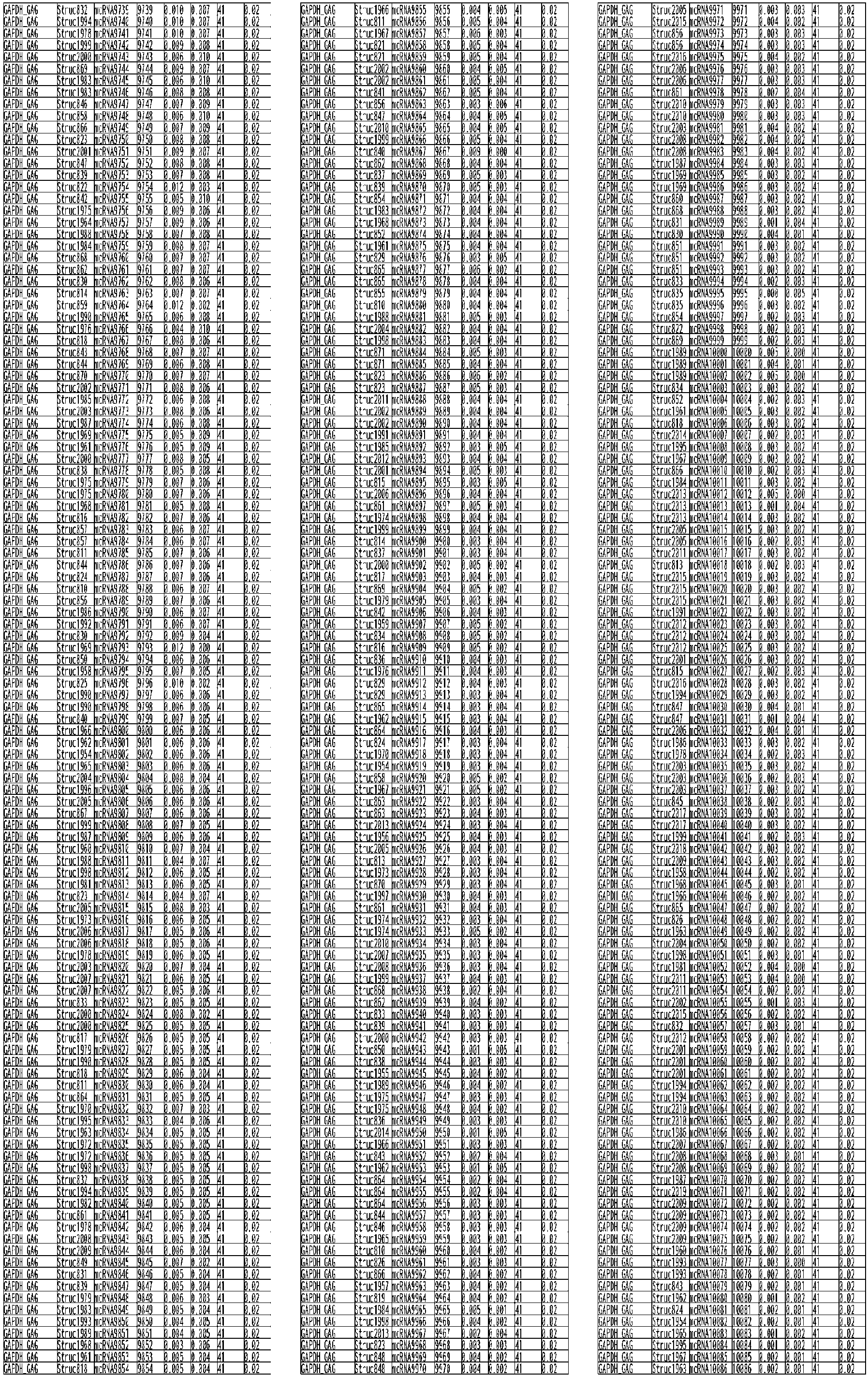

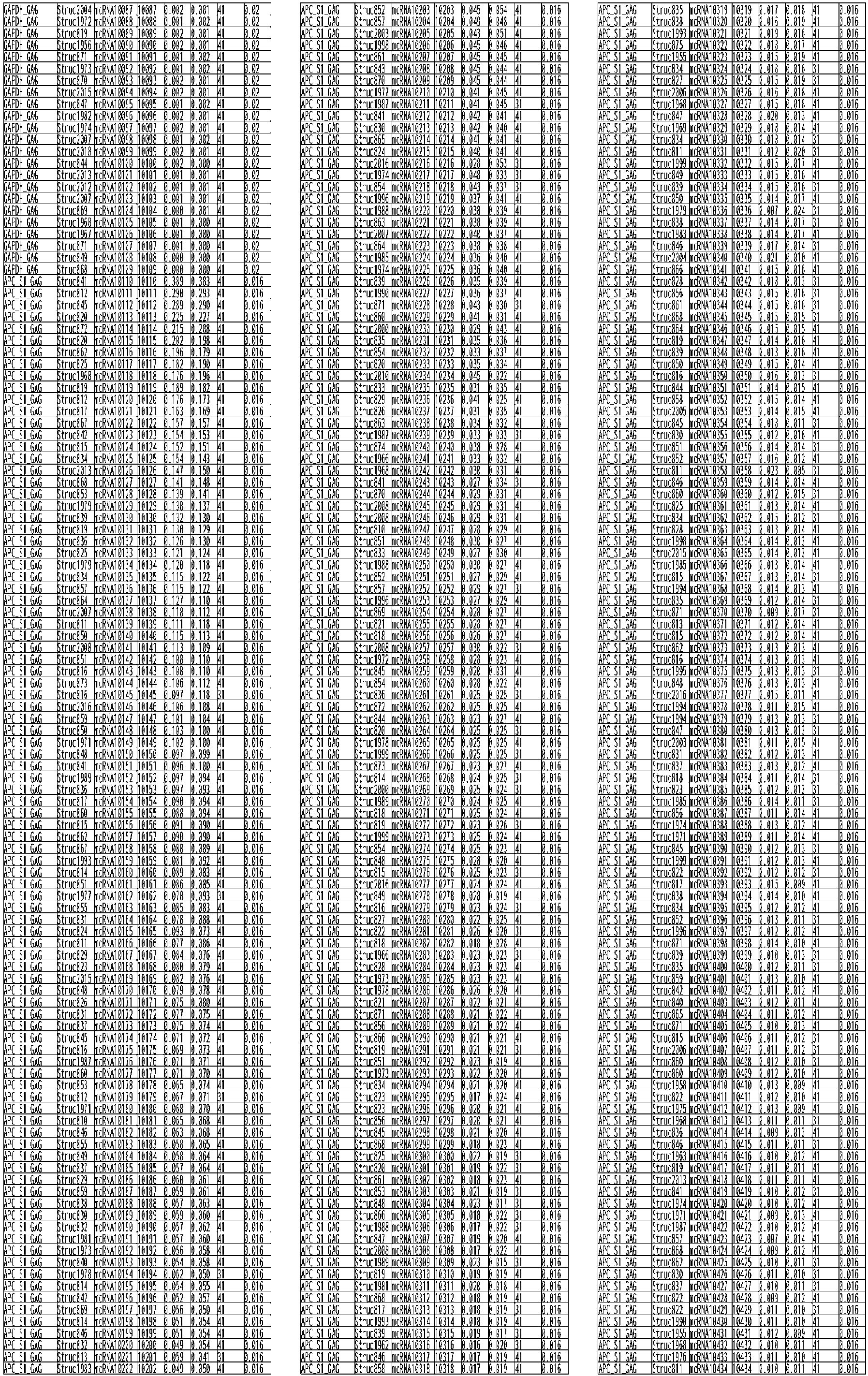

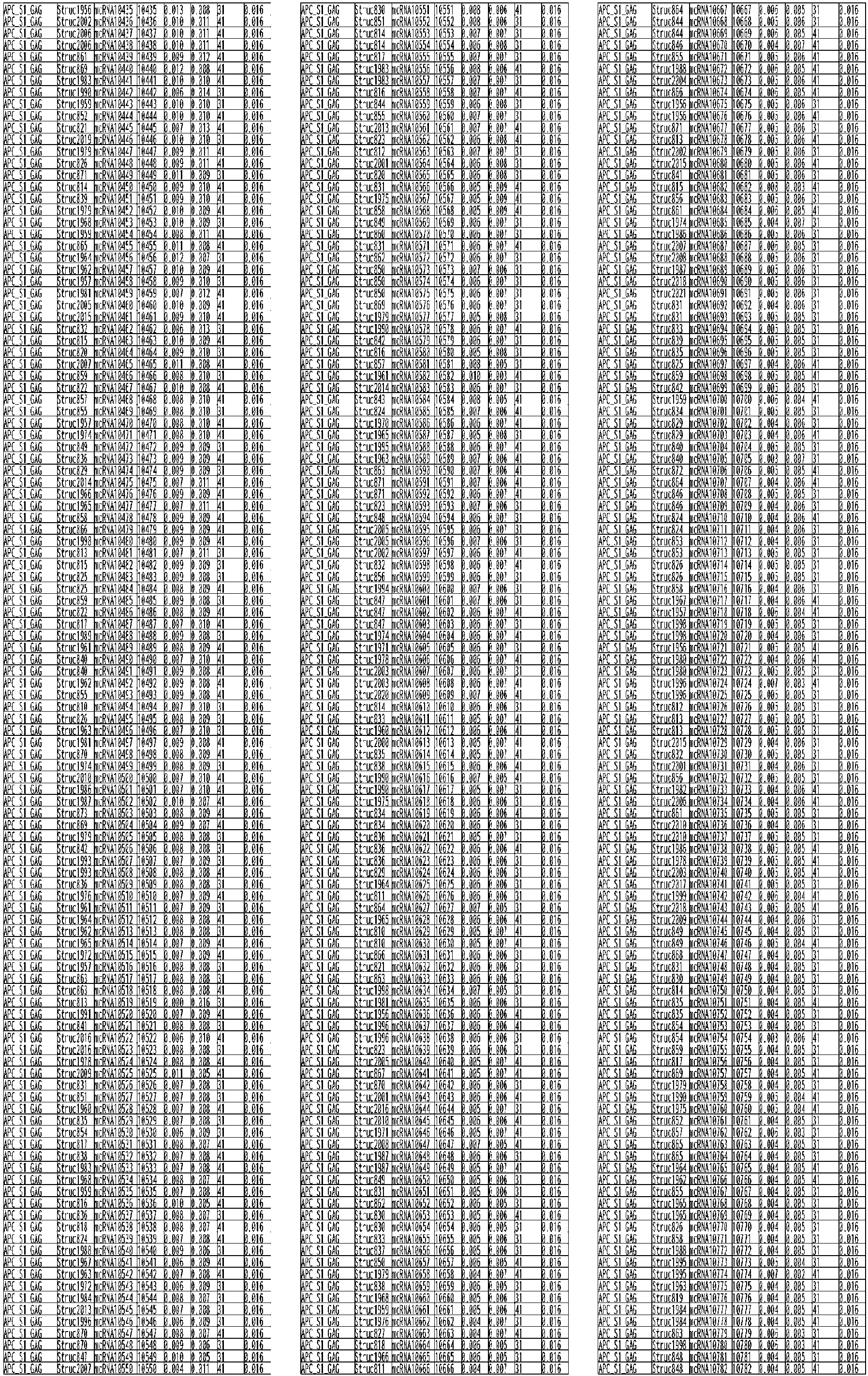

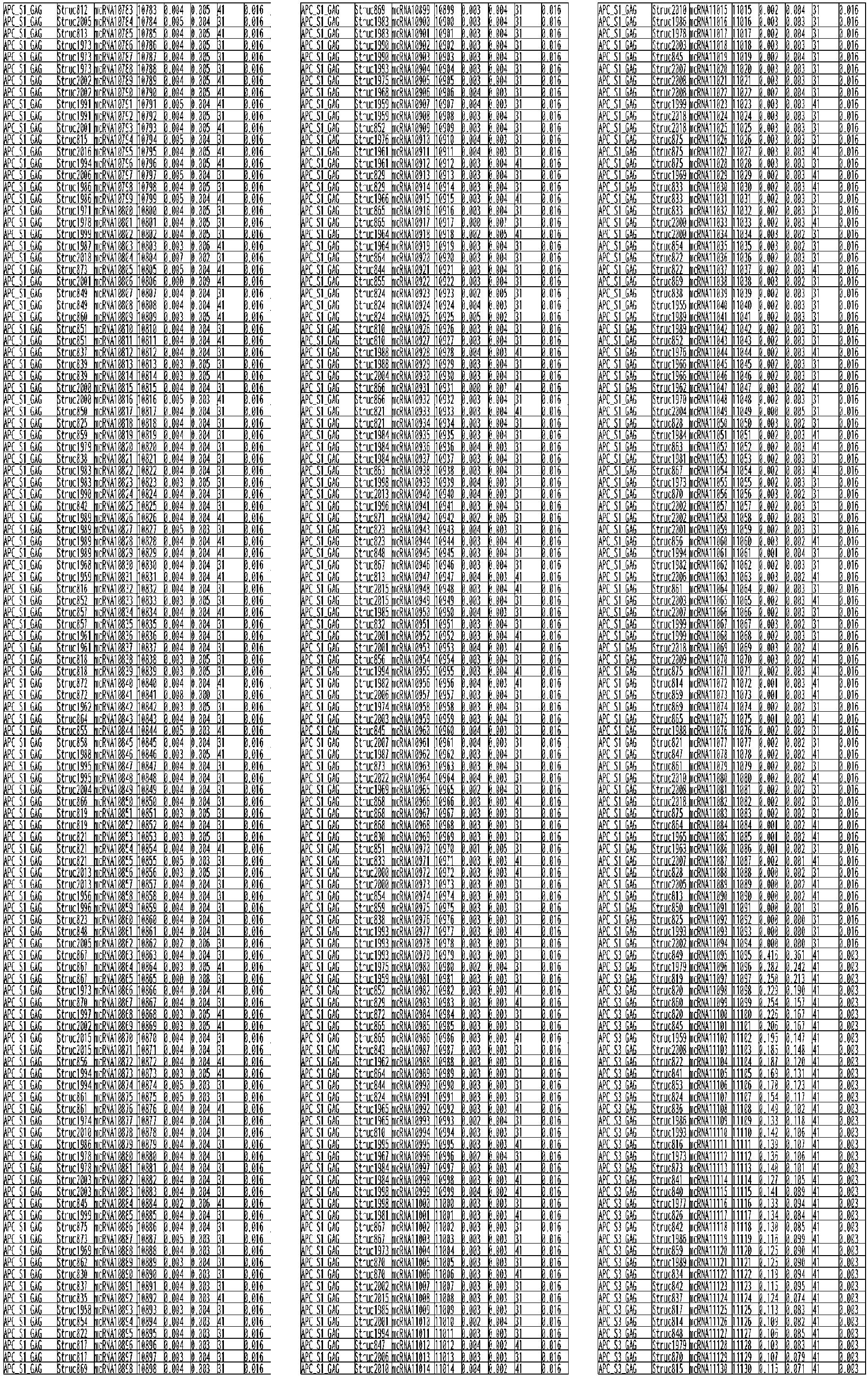

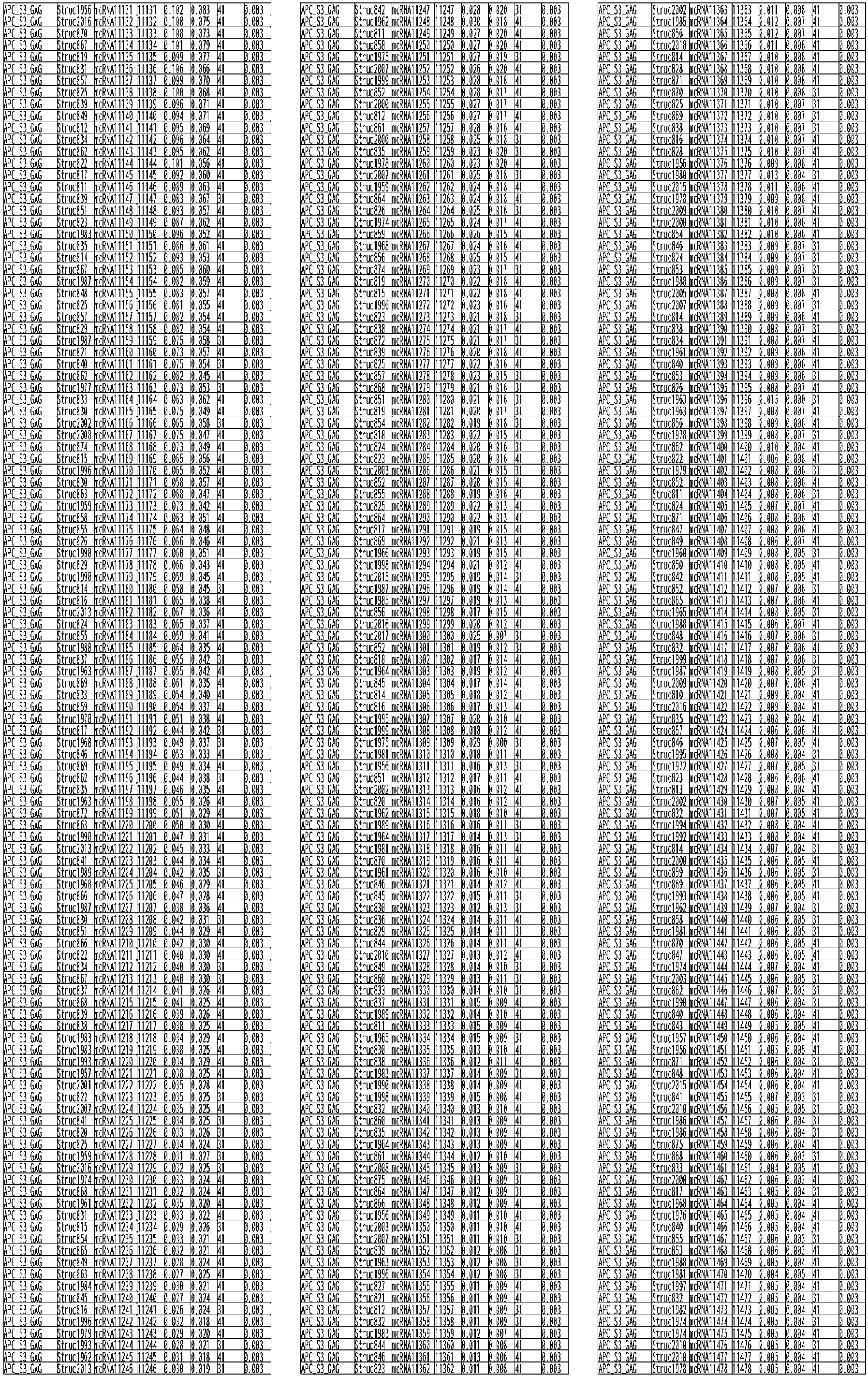

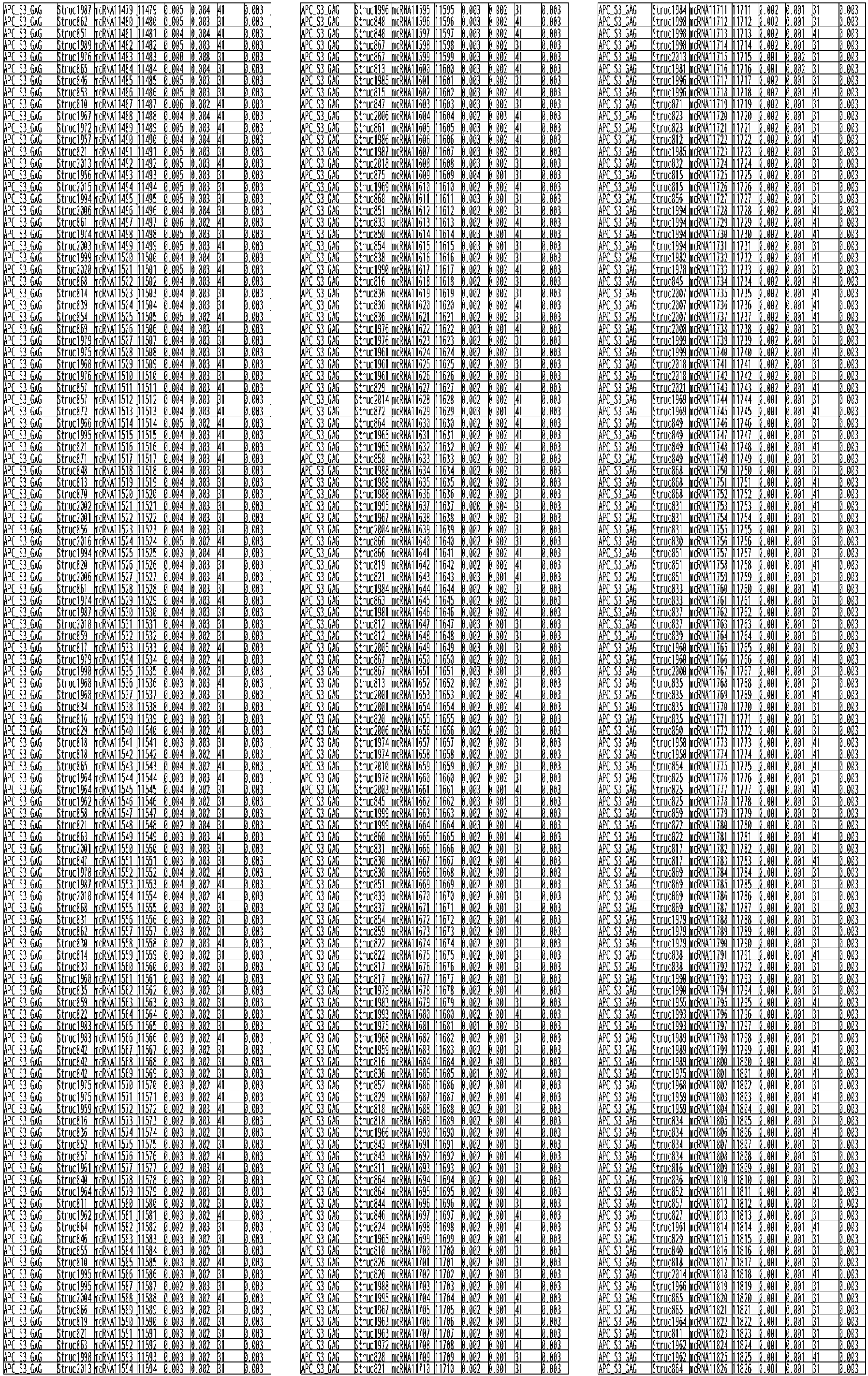

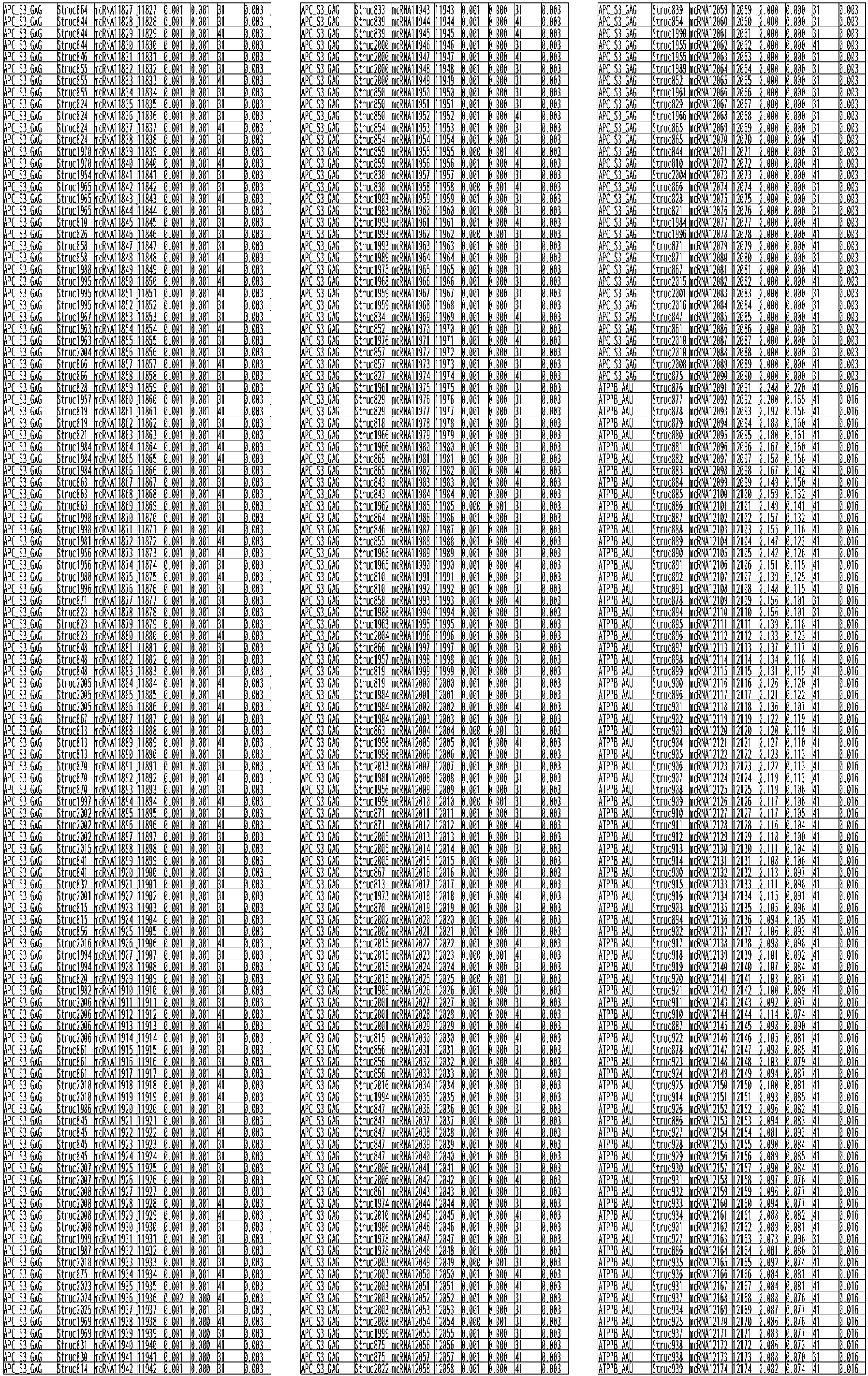

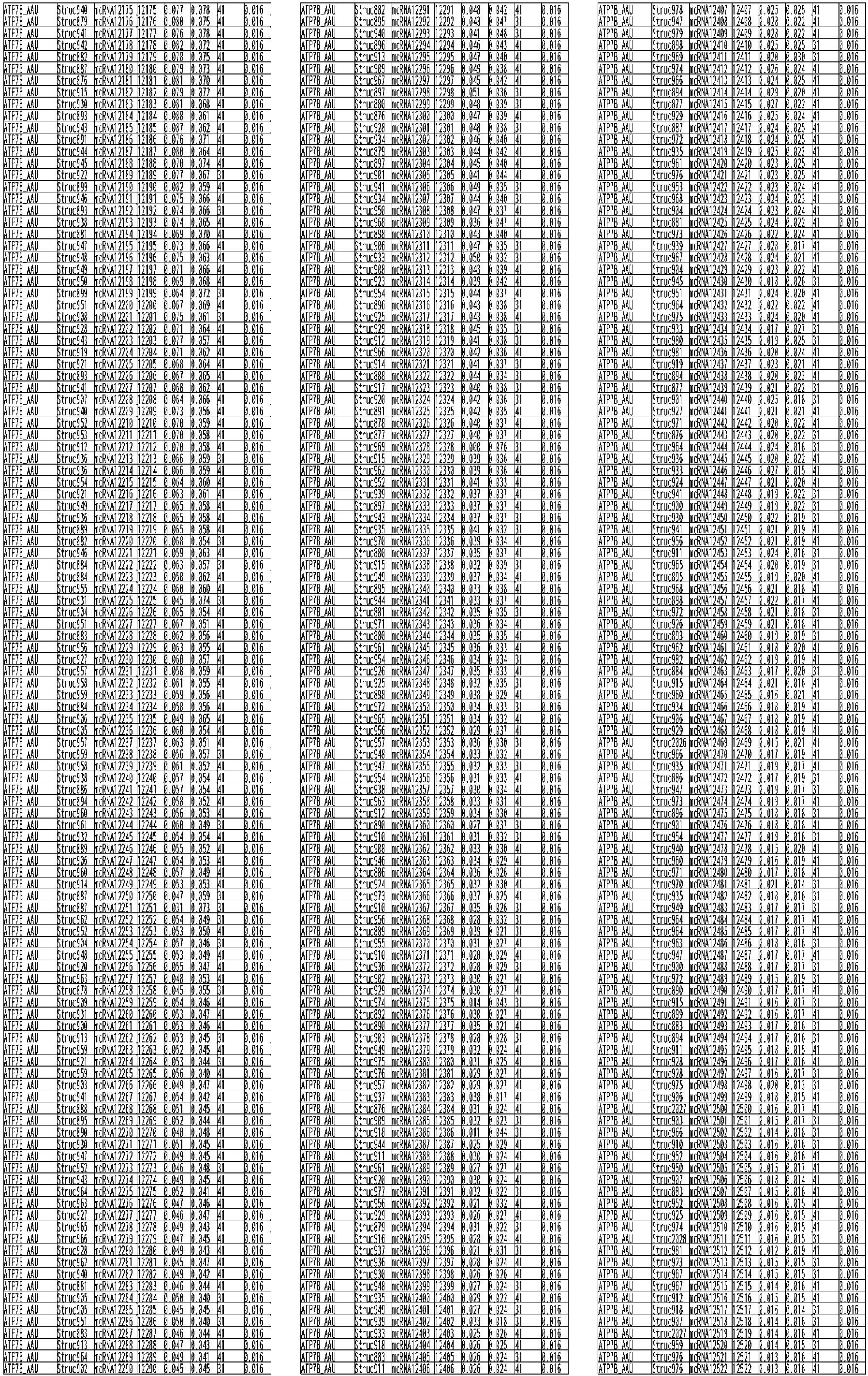

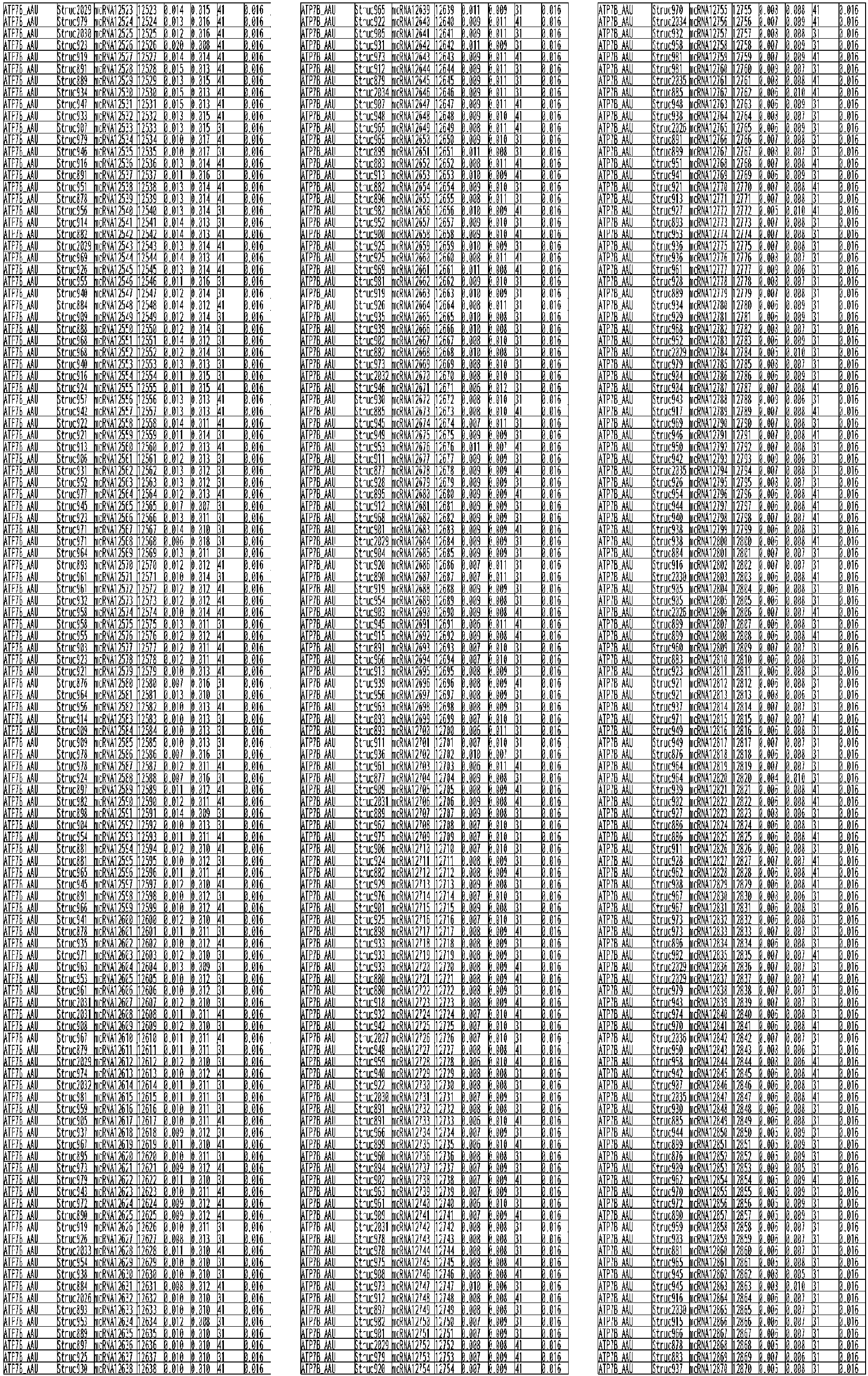

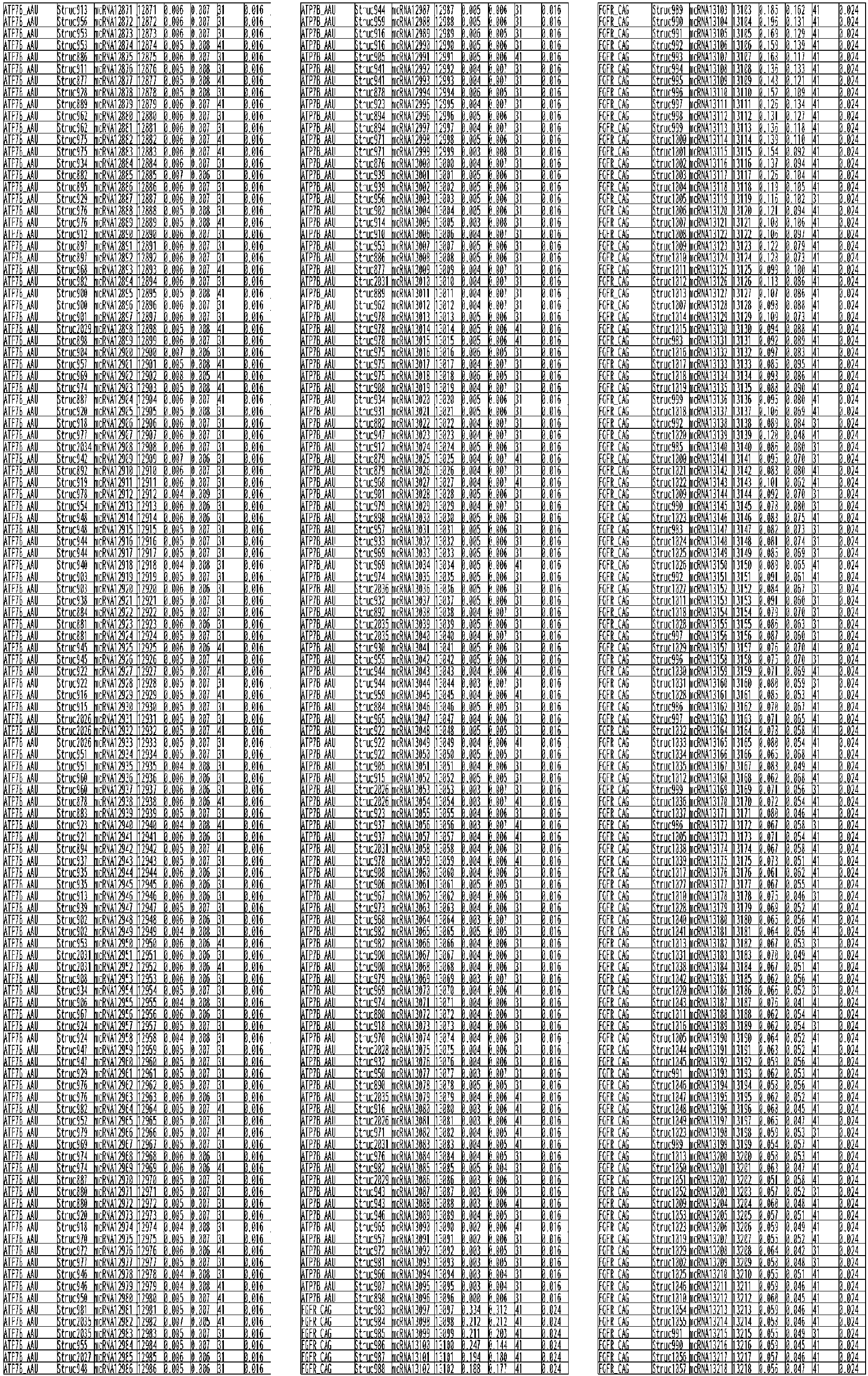

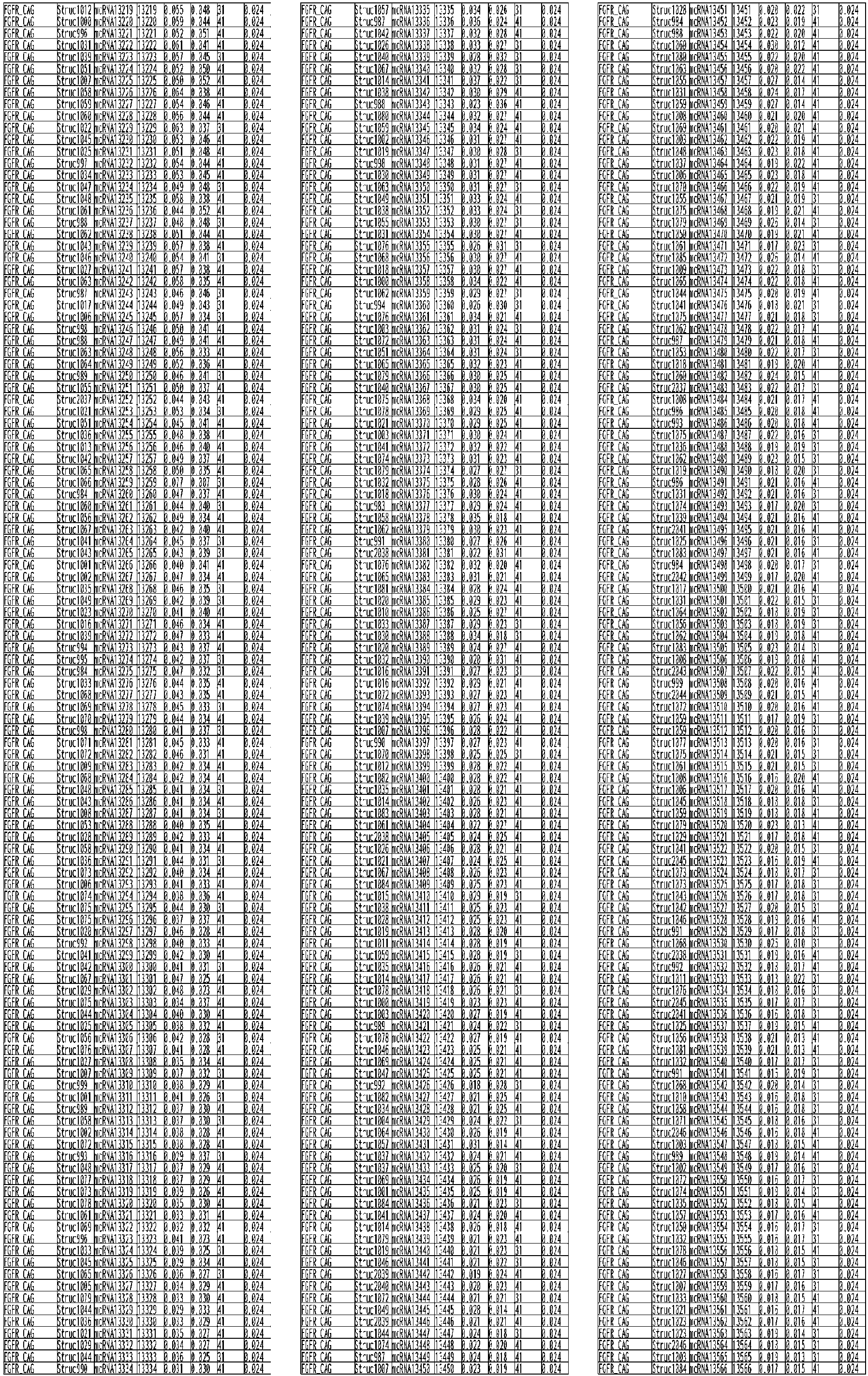

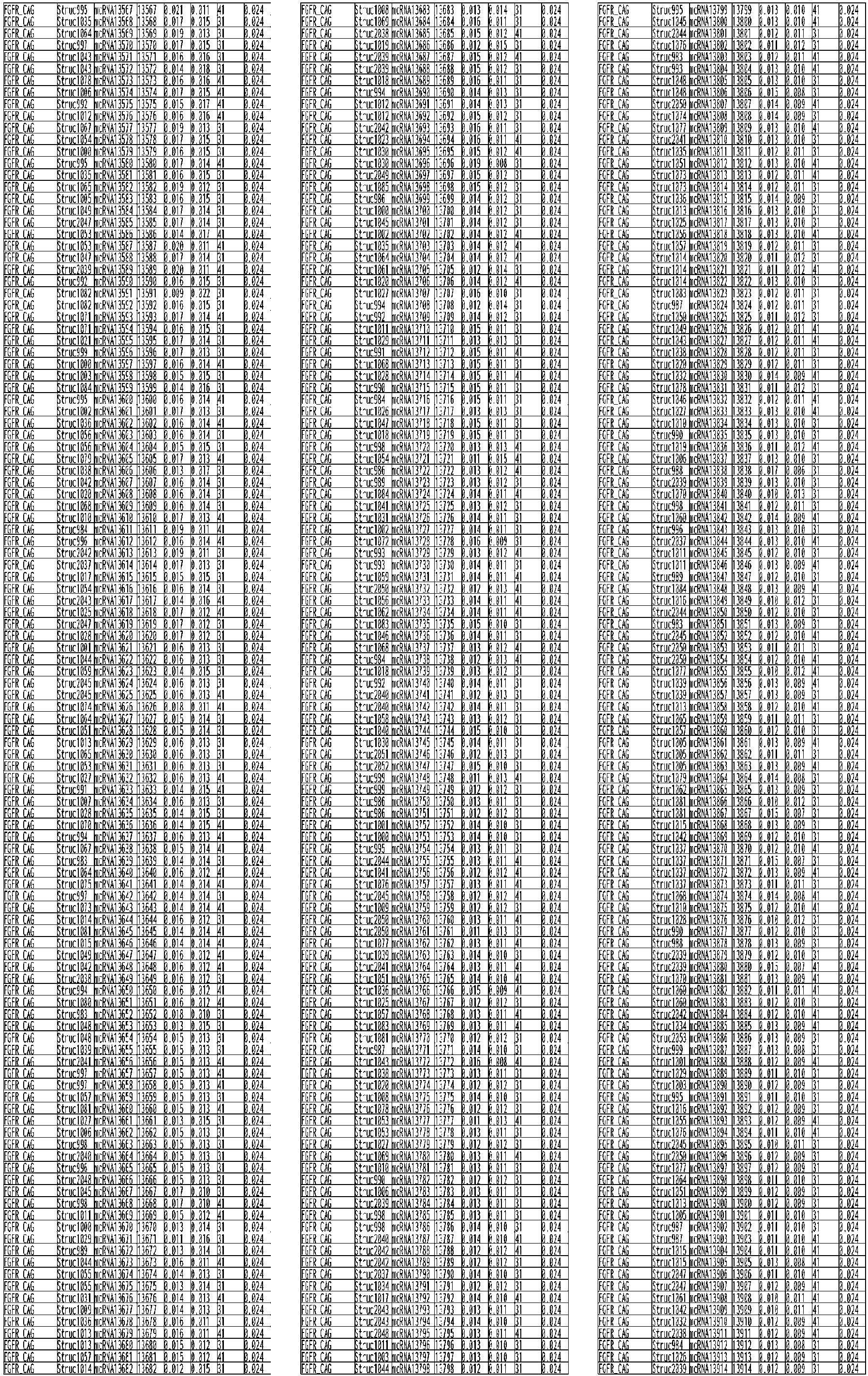

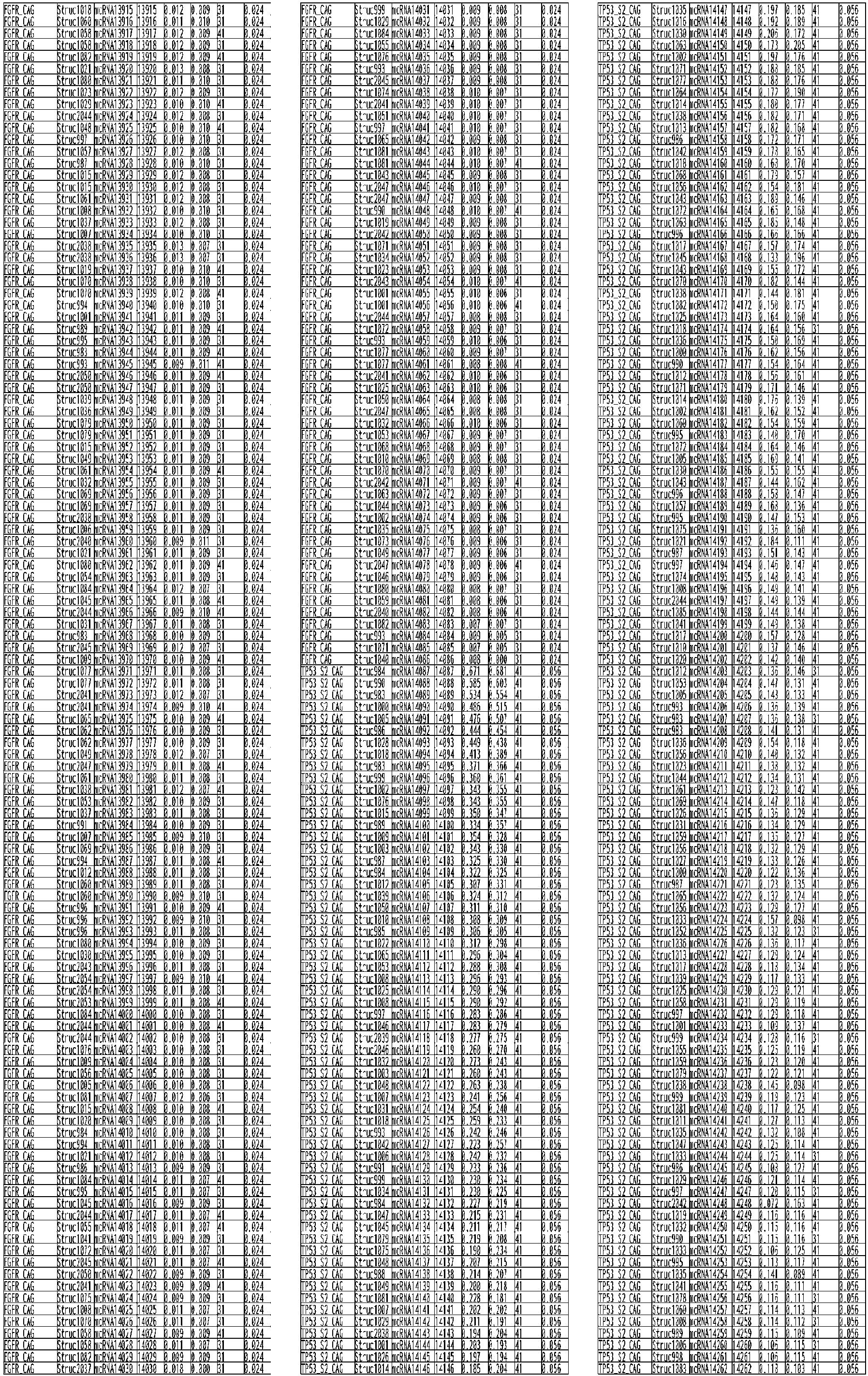

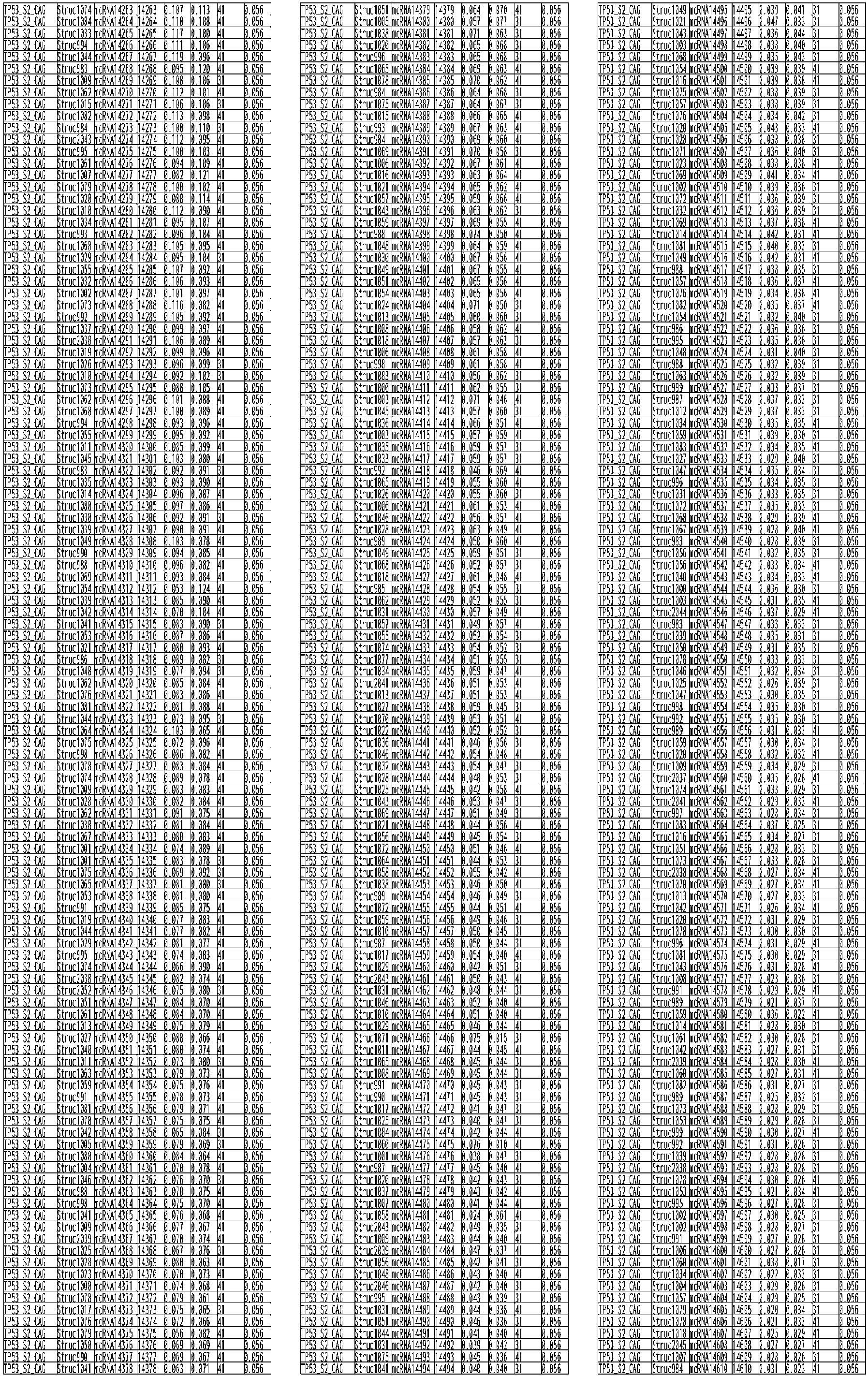

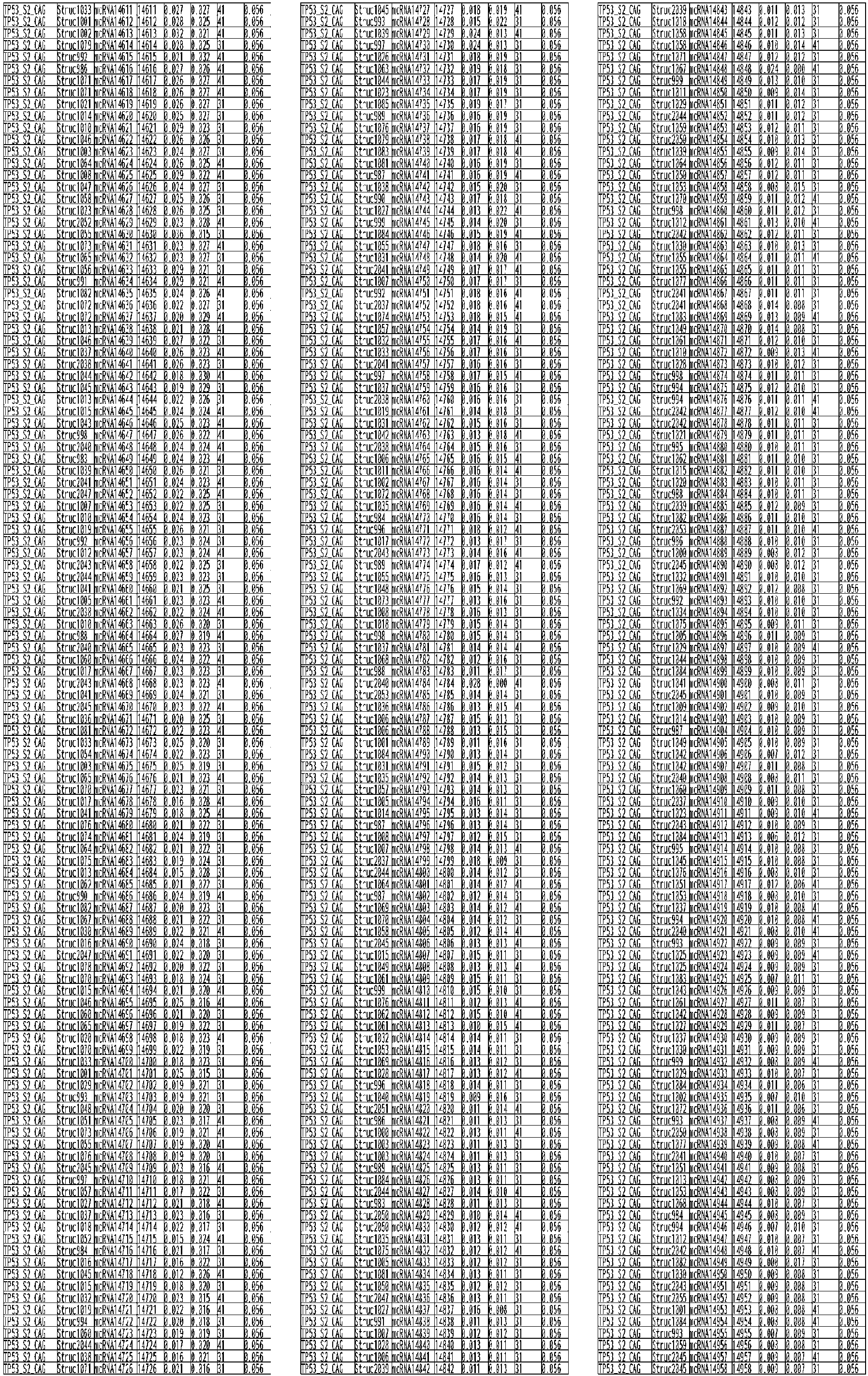

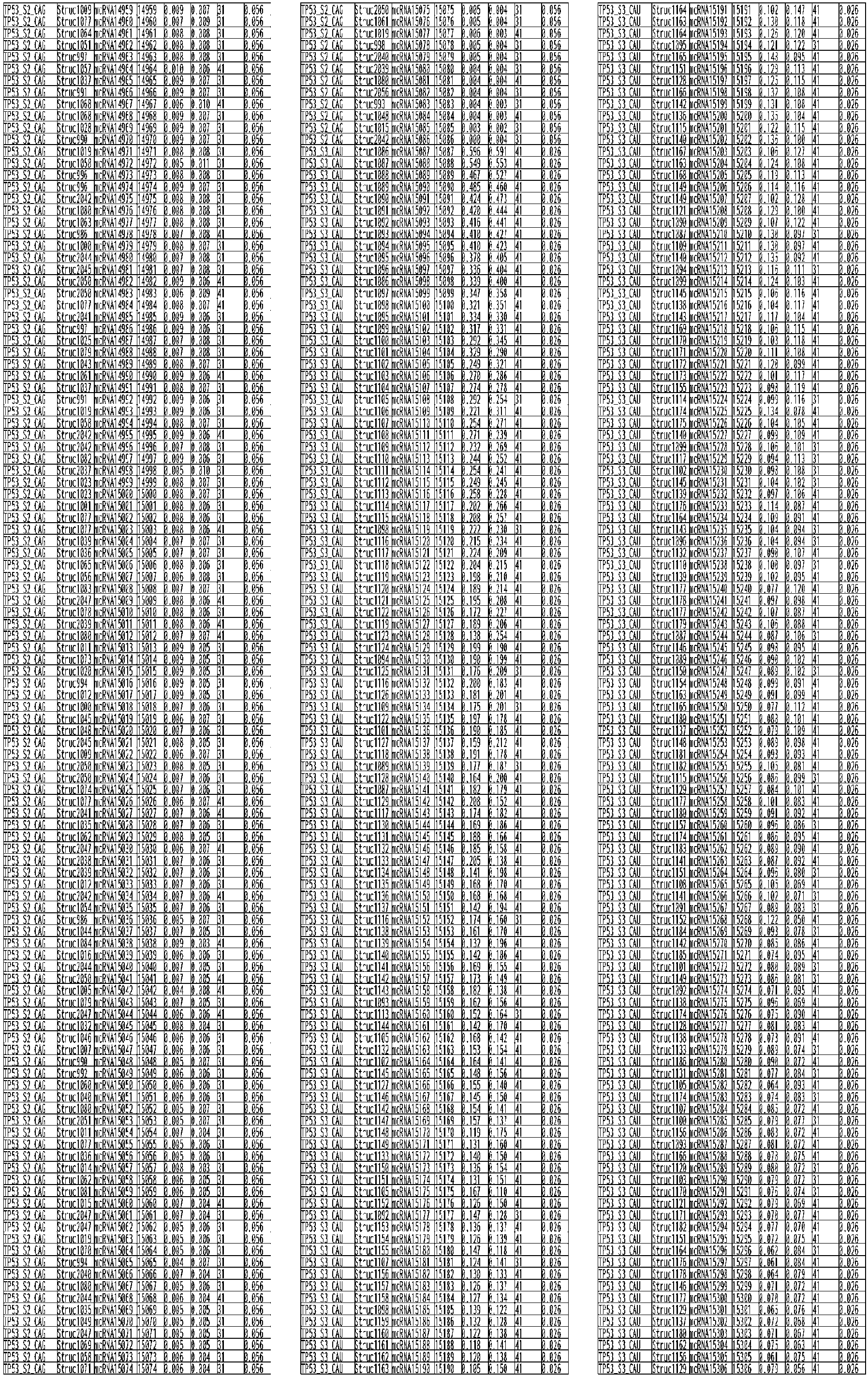

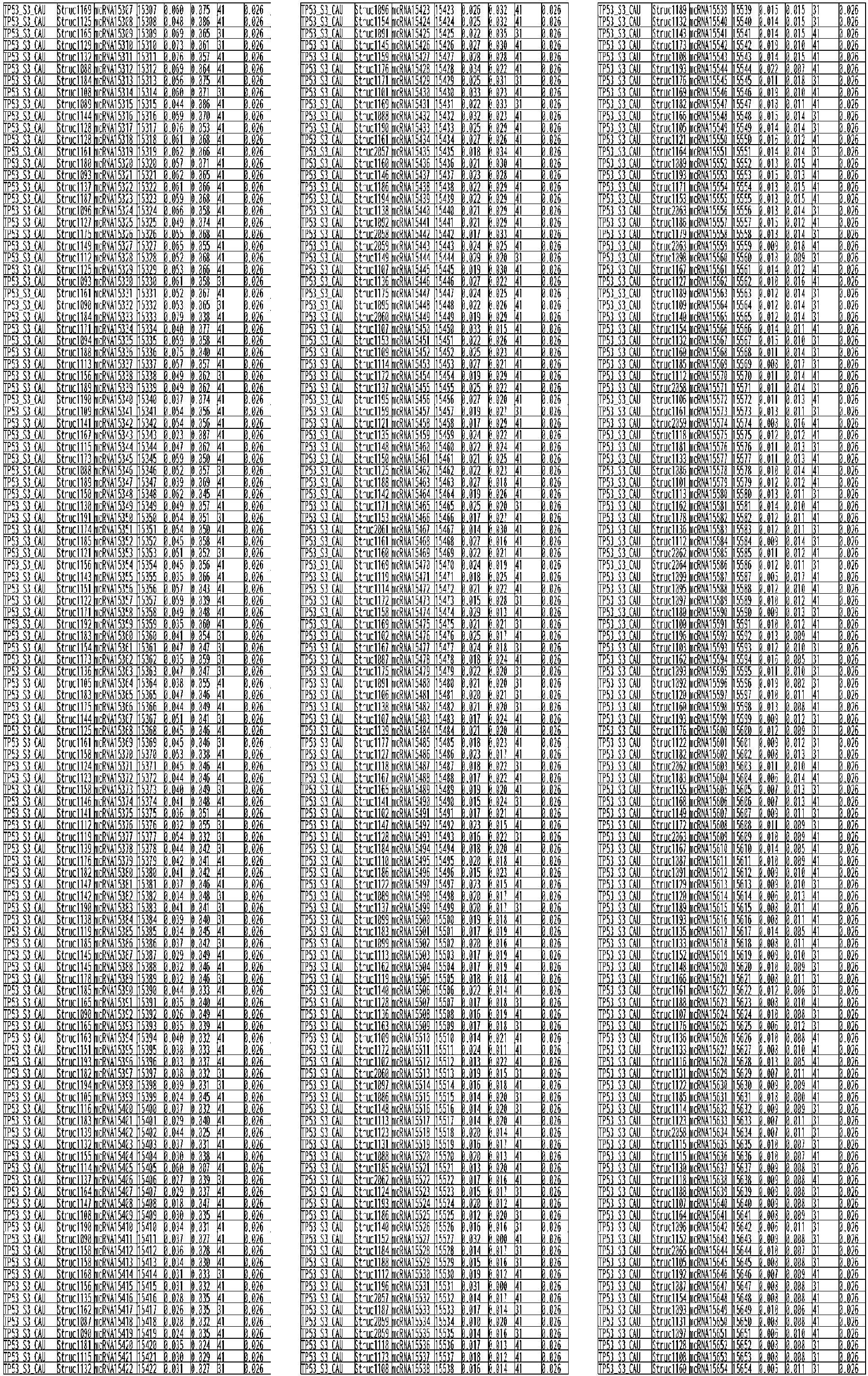

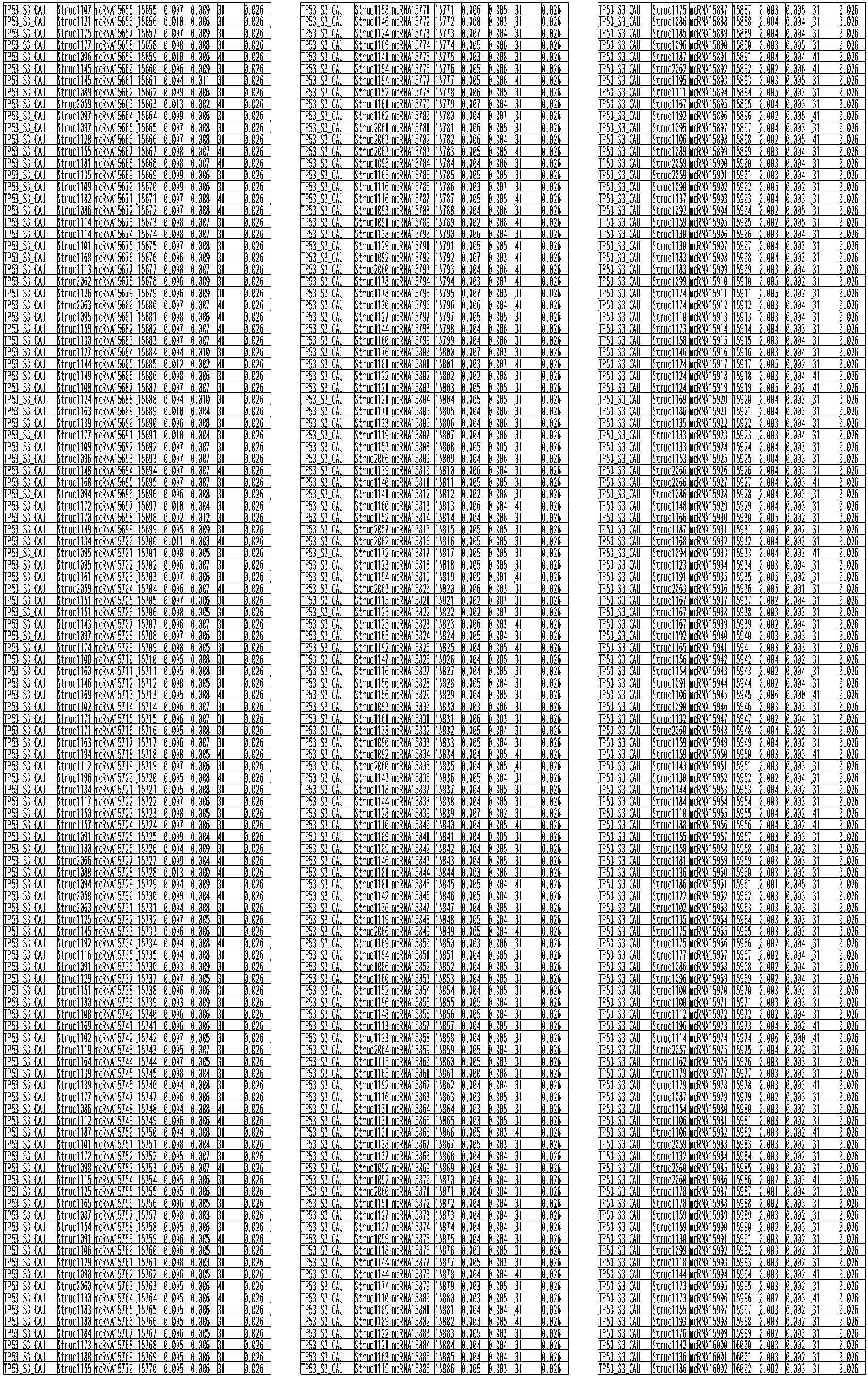

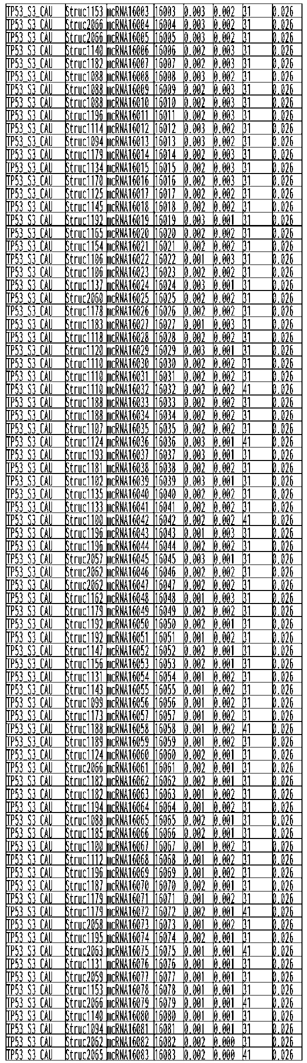

## Claims

1. A sequence for forming an editing substrate together with a target RNA editing site, wherein the sequence, in the form of a complementary strand, forms a double-stranded secondary structure with upstream and downstream portions of the target RNA editing site, wherein a structure of the complementary strand comprises at least one of sets of features indicated by i to x:
i. 0 to 4 mismatches, 0 to 7 wobble base pairs, 0 to 3 internal loops, 0 to 5 bulges, and 0 to 12 deletions from -100 to -81 of the editing site;
ii. 0 to 5 mismatches, 0 to 7 wobble base pairs, 0 to 5 internal loops, 0 to 5 bulges, and 0 to 15 deletions from -80 to -61 of the editing site;
iii. 0 to 5 mismatches, 0 to 7 wobble base pairs, 0 to 3 internal loops, 0 to 5 bulges, and 0 to 18 deletions from -60 to -41 of the editing site;
iv. 0 to 4 mismatches, 0 to 7 wobble base pairs, 0 to 3 internal loops, 0 to 8 bulges, and 0 to 11 deletions from -40 to -21 of the editing site;
v. 0 to 5 mismatches, 0 to 6 wobble base pairs, 0 to 4 internal loops, 0 to 5 bulges, and 0 to 9 deletions from -20 to -1 of the editing site;
vi. 0 to 5 mismatches, 0 to 7 wobble base pairs, 0 to 3 internal loops, 0 to 3 bulges, and 0 to 5 deletions from 0 to 19 of the editing site;
vii. 0 to 4 mismatches, 0 to 7 wobble base pairs, 0 to 3 internal loops, 0 to 6 bulges, and 0 to 9 deletions from 20 to 39 of the editing site;
viii. 0 to 5 mismatches, 0 to 6 wobble base pairs, 0 to 3 internal loops, 0 to 5 bulges, and 0 to 17 deletions from 40 to 59 of the editing site;
ix. 0 to 5 mismatches, 0 to 8 wobble base pairs, 0 to 4 internal loops, 0 to 5 bulges, and 0 to 19 deletions from 60 to 79 of the editing site; and
x. 0 to 4 mismatches, 0 to 6 wobble base pairs, 0 to 3 internal loops, 0 to 5 bulges, and 0 to 19 deletions from 80 to 100 of the editing site.

2. The sequence according to claim 1, wherein a mismatch type on the complementary strand is one of A-A, A-G, A-C, U-U, U-C, G-G, G-A, C-A, C-C, and C-U;
preferably, the wobble base pair on the complementary strand is a G-U pair;
preferably, the bulge on the complementary strand refers to that one base upstream of and one base downstream of a bulge region are both complementarily paired with a target editing strand and corresponding two bases of the target editing strand are consecutive;
preferably, the deletion on the complementary strand is that bases upstream and downstream of a deletion region are consecutive, and the target editing strand in the deletion region has a corresponding number of bases; and
preferably, the internal loop on the complementary strand refers to that the target editing strand is complementarily paired with bases of the complementary strand, one upstream of and one downstream of the internal loop, and the internal loop has a corresponding number of bases mismatched.

3. The sequence according to claim 1, wherein the double-stranded secondary structure is one selected from structures Struc1 to Struc1196;
preferably, structural features of the double-stranded secondary structure are as shown in Table 1;
preferably, the structural features of the double-stranded secondary structure comprise one or more of:
a mismatch formed between bases, denoted by M;
wobble base pairing, denoted by W;
an internal loop, denoted by Li;
a deletion, denoted by D;
a bulge, denoted by Ii; and
Watson-Crick pairing, denoted by P;
preferably, the double-stranded secondary structure has a feature denoted by X, X = [n1, n2, n3 ...], with n1, n2, ... indicating positions of a structure X, wherein the editing site is at 0, -n indicates that the position is an n-th base upstream of the editing site, and n indicates that the position is an n-th base downstream of the editing site;
preferably, when the double-stranded secondary structure X is M, M = [n1, n2, n3 ...] indicates that mismatches are present at positions of n1, n2, n3 ... from the editing site;
when the double-stranded secondary structure X is W, W = [n1, n2, n3 ...] indicates that wobble base pairs are present at positions of n1, n2, n3 ... from the editing site;
when the double-stranded secondary structure X is D, D = [n1, n2, n3...] indicates that deletions are present at positions of n1, n2, n3 ... from the editing site;
when X is Ii, Ii = [n1, n2], with i being any positive integer indicating that i bases are inserted between n1 and n2 from the editing site;
when the double-stranded secondary structure X is Li, Li = [n1 to n2, n3 to n4 ...], with i being any positive integer indicating that internal loops having a size of i nt are formed in position ranges of n1 to n2, n3 to n4 ... from the editing site; and
in any double-stranded secondary structure, all positions are P except where X is one or more of M, W, D, Ii, and Li.

4. The sequence according to claim 1, wherein a motif of the target RNA editing site, and one base upstream of and one base downstream of the RNA editing site is NAN;
preferably, the NAN is UAN, AAN, CAN, or GAN;
preferably, the UAN is UAG, UAU, UAC, or UAA;
preferably, the AAN is AAU, AAC, AAG, or AAA;
preferably, the CAN is CAU, CAG, CAC, or CAA; and
preferably, the GAN is GAU, GAC, GAA, or GAG.

5. The sequence according to claim 1, wherein when the motif of the target RNA editing site, and one base upstream of and one base downstream of the RNA editing site is NAN, the double-stranded secondary structure is selected from Struc1 to Struc1196;
preferably, when the motif of the target RNA editing site, and one base upstream of and one base downstream of the RNA editing site is UAA, the double-stranded secondary structure is selected from Struc1 to Struc143;
preferably, when the motif of the target RNA editing site, and one base upstream of and one base downstream of the RNA editing site is UAG, the double-stranded secondary structure is selected from Struc144 to Struc260;
preferably, when the motif of the target RNA editing site, and one base upstream of and one base downstream of the RNA editing site is UAU, the double-stranded secondary structure is selected from Struc261 to Struc320;
preferably, when the motif of the target RNA editing site, and one base upstream of and one base downstream of the RNA editing site is UAC, the double-stranded secondary structure is selected from Struc321 to Struc395;
preferably, when the motif of the target RNA editing site, and one base upstream of and one base downstream of the RNA editing site is CAA, the double-stranded secondary structure is selected from Struc396 to Struc515;
preferably, when the motif of the target RNA editing site, and one base upstream of and one base downstream of the RNA editing site is CAC, the double-stranded secondary structure is selected from Struc516 to Struc541;
preferably, when the motif of the target RNA editing site, and one base upstream of and one base downstream of the RNA editing site is AAG, the double-stranded secondary structure is selected from Struc542 to Struc684;
preferably, when the motif of the target RNA editing site, and one base upstream of and one base downstream of the RNA editing site is AAC, the double-stranded secondary structure is selected from Struc685 to Struc767;
preferably, when the motif of the target RNA editing site, and one base upstream of and one base downstream of the RNA editing site is GAA, the double-stranded secondary structure is selected from Struc768 to Struc809;
preferably, when the motif of the target RNA editing site, and one base upstream of and one base downstream of the RNA editing site is GAG, the double-stranded secondary structure is selected from Struc810 to Struc875;
preferably, when the motif of the target RNA editing site, and one base upstream of and one base downstream of the RNA editing site is AAU, the double-stranded secondary structure is selected from Struc876 to Struc982;
preferably, when the motif of the target RNA editing site, and one base upstream of and one base downstream of the RNA editing site is CAG, the double-stranded secondary structure is selected from Struc983 to Struc1085; and
preferably, when the motif of the target RNA editing site, and one base upstream of and one base downstream of the RNA editing site is CAU, the double-stranded secondary structure is selected from Struc1086 to Struc1196.

6. The sequence according to claim 1, wherein the complementary strand is complementarily base-paired at a non-mismatch or non-deletion or non-bulge or non-internal loop or non-wobble-base-pairing site.

7. The sequence according to claim 1, wherein the sequence forming an editing substrate together with a target RNA editing site is 25 bp to 300 bp in length; and
preferably, the sequence is 25 bp to 151 bp in length.

8. The sequence according to claim 1, wherein the RNA is an RNA forming a specific secondary structure together with a target RNA to be edited;
preferably, the target RNA to be edited is a messenger RNA, a ribosomal RNA, a transfer RNA, a long non-coding RNA, or a small RNA; and
preferably, the small RNA is miRNA, pri-miRNA, pre-miRNA, piRNA, siRNA, snoRNA, snRNA, exRNA, or scaRNA.

9. An mcRNA, comprising a mimic domain, wherein a sequence of the mimic domain is the sequence according to any one of claims 1 to 8, and forms the double-stranded secondary structure together with a target RNA to be edited;
preferably, the mcRNA further comprises binding domains located at both ends of the mimic domain and complementarily paired with regions other than the double-stranded secondary structure of the target RNA to be edited;
preferably, the binding domain is 0 to 55 bp in length on a single side; and
preferably, a sequence of the mcRNA is any one selected from sequences of SEQ ID NOs.: 1 to 16083.

10. A design method for the mcRNA according to claim 9, comprising the following steps:
S1, obtaining a reference sequence: using a strand of a reference secondary structure where an editing site is located is an editing strand of the reference secondary structure and the other strand as a complementary strand of the reference secondary structure, and obtaining nucleotide sequences of both strands, respectively;
S2, obtaining a prepared mcRNA sequence of a target editing site: extending a preset length upstream and downstream from a site to be edited as a starting point to obtain a template sequence of a mimic domain, namely a target editing strand; and designing a sequence complementarily paired with the template sequence as the prepared mcRNA sequence;
S3, designing an mcRNA mimic domain: aligning the sequence of the target editing strand to a sequence of the editing strand of the reference secondary structure by extending from the editing site to both ends; aligning the prepared mcRNA sequence to a sequence of the complementary strand of the reference secondary structure; when an alignment result of the sequence of the target editing strand and the sequence of the editing strand of the reference secondary structure indicates consistency, setting a corresponding position of the mcRNA mimic domain to the corresponding sequence of the complementary strand of the reference secondary structure; and when the alignment result of the sequence of the target editing strand and the sequence of the editing strand of the reference secondary structure indicates inconsistency, setting and designing the corresponding position of the mcRNA mimic domain to a sequence forming a same structure as the corresponding reference secondary structure together with the target editing site; and
S4, designing mcRNA binding domains: on the basis of the mcRNA mimic domain designed in step S3, extending complementarily paired sequences a preset length from both ends of the mimic domain to form the binding domains.

11. Use of the sequence or the mcRNA according to any one of claims 1 to 9 in preparation of an RNA editing reagent or composition.

12. An RNA editing reagent or composition, comprising the sequence or the mcRNA according to any one of claims 1 to 9.

13. A method of editing a target RNA in a host cell, wherein an oligonucleotide comprising the sequence according to any one of claims 1 to 8 or the mcRNA according to claim 9 is introduced into the host cell; and
preferably, the target RNA is ATP7B gene, FGFR gene, TP53 gene, APC gene, SERPINA1 gene, MECP2 gene, or SCN1A gene.

14. Use of the sequence or the mcRNA according to any one of claims 1 to 9 for in preparation of a medicament for treating a disease or disorder, wherein the disease or disorder is an inherited genetic disease, or a disease or disorder associated with one or more acquired gene mutations;
preferably, the disease or disorder is a monogenic disease or disorder;
preferably, the disease or disorder is a polygenic disease or disorder;
preferably, the gene is ATP7B, FGFR, TP53, APC, SERPINA1, MECP2, or SCN1A; and
preferably, the disease or disorder is selected from the group consisting of hepatolenticular degeneration, rett syndrome, Dravet syndrome, cystic fibrosis, Hurler syndrome, alpha-1-antitrypsin (A1AT) deficiency, Parkinson's disease, Alzheimer's disease, albinism, amyotrophic lateral sclerosis, asthma, beta-thalassemia, Cadasil syndrome, peroneal muscular atrophy, chronic obstructive pulmonary disease (COPD), distal spinal muscular atrophy (DSMA), Duchenne/Becker muscular dystrophy, dystrophic epidermolysis bullosa, epidermolysis bullosa, Fabry disease, Leiden factor V related disease, familial adenoma, polyposis, galactosemia, Gaucher's disease, glucose-6-phosphate dehydrogenase, hemophilia, hereditary hemochromatosis, Hunter syndrome, Huntington's disease, inflammatory bowel disease (IBD), hereditary polyagglutination syndrome, Leber congenital amaurosis, Lesch-Nyhan syndrome, Lynch syndrome, marfan syndrome, mucopolysaccharidosis, muscular dystrophy, myotonic dystrophy type 1 and type 2, neurofibroma, Niemann-Pick diseases type A, type B and type C, NY-esol-associated cancers, Peutz-Jeghers syndrome, phenylketonuria, Pompe's disease, primary eyelash disease, and prothrombin mutation-associated diseases such as prothrombin G20210A mutation, pulmonary hypertension, retinitis pigmentosa, Sandhoff's disease, severe complicated immunodeficiency syndrome (SCID), sickle cell anemia, spinal muscular atrophy, Steger's disease, Tay-Sahr's disease, Usher's syndrome, X-linked immunodeficiency, and cancers.
